# EUROPEAN PATENT SPECIFICATION

(11) **EP 4 308 723 B1**
(45) Date of publication and mention of the grant of the patent: **23.04.2025**
(21) Application number: 22715975.3
(22) Date of filing: 14.03.2022
(51) Int. Cl.: C12Q 1/6806, C12Q 1/6853

(54) **TARGETED NEXT-GENERATION SEQUENCING VIA ANCHORED PRIMER EXTENSION**
GEZIELTE SEQUENZIERUNG DER NÄCHSTEN GENERATION ÜBER VERANKERTE PRIMEREXTENSION
SÉQUENÇAGE CIBLÉ DE NOUVELLE GÉNÉRATION PAR L'INTERMÉDIAIRE D'UNE EXTENSION D'AMORCE ANCRÉE

(30) Priority: 15.03.2021 US 202163161062 P
(43) Date of publication of application: 24.01.2024
(73) Proprietor: F. Hoffmann-La Roche AG, 4070 Basel (CH); Roche Diagnostics GmbH, 68305 Mannheim (DE)
(72) Inventor: BURGESS, Daniel, Pleasanton, CA 94588 (US); JEFFERSON, Keynttisha, Pleasanton, CA 94588 (US)
(74) Representative: Simmons & Simmons LLP (Munich)
(86) International application number: PCT/EP2022/056511
(87) International publication number: WO 2022/194764

(56) References cited:
- EP-A1- 3 674 413
- WO-A1-2013/036668
- WO-A1-2013/036685
- WO-A1-2016/016452
- WO-A1-2018/108328
- WO-A1-2019/038372
- WO-A1-2020/180813
- WO-A1-2022/008578
- WO-A2-2015/026853
- SHI X ET AL: "5 ' -RACEing Across a Bridging Oligonucleotide", BIOTECHNIQUES, 1 March 2002 (2002-03-01), pages 480 - 482, XP055929499, Retrieved from the Internet <URL:https://www.future-science.com/doi/pdf/10.2144/02323bm01> [retrieved on 20220609]
- HUANG JUN-CHAO ET AL: "Simultaneous amplification of 5' and 3' cDNA ends based on template-switching effect and inverse PCR", BIOTECHNIQUES, INFORMA HEALTHCARE, US, vol. 40, no. 2, 1 February 2006 (2006-02-01), pages 187 - 189, XP009110156, ISSN: 0736-6205, DOI: 10.2144/000112051

## Description

### BACKGROUND OF THE DISCLOSURE

Several methods have been described for selectively amplifying regions of interest within a complex genome, or set of nucleic acids, in preparation for next generation sequencing (NGS) to identify sequence and/or structural variants. Each method has its own unique combination of strengths and weaknesses in terms of sensitivity, specificity, cost, scalability, turnaround time and other variables.

Unidirectional primer extension (UPE) methods are advantageous over bidirectional primer extension (BPE) methods (e.g. traditional PCR) because only one primer binding target must be known and present in the sample for the assay to succeed. This enables the targeted amplification and sequencing of important categories of structural variants (e.g. translocations, inversions, deletions) that alter the relative position of primer binding targets and therefore cannot be detected by BPE methods.

The central challenge in designing an effective UPE method is to incorporate a mechanism for amplifying the UPE products so that they can be analyzed further. Traditional PCR may be used for the amplification step, but this requires de novo creation of a second PCR primer binding target on the distal end of the initial single-stranded DNA primer extension product, where the sequence is unknown, for the "reverse" PCR primer to anneal. Methods that have been described for creating a "reverse" PCR primer binding target include blunt-ended ligation, single stranded DNA ligation and homopolymer tailing (e.g. using a template independent polymerase such as terminal transferase (TdT)). These approaches, however, are often inefficient. Another common method for amplifying DNA is rolling-circle amplification (RCA). This method involves only a single primer binding target, but requires that the template molecule exists as a covalently closed circle structure.

WO 2019/038372 relates to a next generation DNA sequencing method and use for accurate and massively parallel quantification of one or more nucleic acid targets.

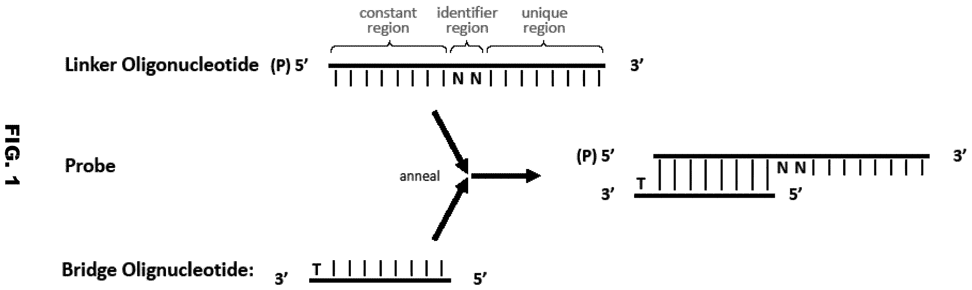

Shi et al., BioTechniques 32:480-482, 2002 describe a method to amplify cDNA5' ends, termed BO-5'RACE, for 5'-RACEing across a bridging oligonucleotide (BO). A partial sequence of the cDNA was used to design oligonucleotides for reverse transcription (RT), the BO, and PCR (F1, F2, R1, and R2) to test the BO-5'RACE protocol (Figure 1).

### BRIEF SUMMARY OF THE DISCLOSURE

The present disclosure addresses the challenge of creating a second PCR primer binding target on the distal end of the initial UPE DNA primer extension product by creating a covalently-closed circle structure so that "forward" and "reverse" PCR primer binding targets are both already present on the initial primer extension product (e.g. where the binding targets are included within a "constant region" as described herein). In this manner, a separate step does not need to be performed to incorporate a "reverse" PCR primer binding target into the primer extension product. The creation of the covalently closed circle structure is believed to facilitate the amplification of the UPE product either by "inverse PCR" (a variant of traditional PCR) or by RCA, an option which provides valuable workflow flexibility.

Applicants have discovered that the presently disclosed methods allow for a unidirectional primer extension based method of targeted sequencing that relies on inverse PCR or rolling circle amplification to amplify captured products, enabled by circularization and/or concatenation of the initial extension product(s). In some embodiments, the methods disclosed herein are compatible with barcoding technology, such as unique molecular identifiers. In some embodiments, the methods disclosed herein are compatible with product amplification via inverse PCR or rolling-circle amplification (RCA), or a combination of these. Applicants have surprisingly discovered that the disclosed methods utilize as little as 10ng of a sample (e.g. sample including one or more target nucleic acids) as a template, e.g. as little as 10ng of human gDNA as template. Applicants have further discovered that the disclosed methods may be utilized to identify sequence variants present at sub-Mendelian allele frequencies.

In accordance with the foregoing, a first aspect of the present invention is a method of enriching one or more target nucleic acids in a sample, comprising:
(a) annealing one or more probes to the one or more target nucleic acid molecules in the sample to form one or more probe-target nucleic acid molecule complexes, wherein the one or more probes each comprise a constant region and a unique region, wherein the unique region of each probe includes a nucleotide sequence which hybridizes to a complementary nucleic acid sequence within the one or more target nucleic acid molecules;
(b) extending the one or more probe-target nucleic acid molecule complexes to form one or more extended probe-target nucleic acid molecule complexes, and performing A-tailing;
(c) generating one or more ligation products from the one or more extended probe-target nucleic acid molecule complexes;
(d) introducing one or more primers to the generated one or more ligation products, wherein the one or more primers hybridize to one or more complementary portions of the constant region within each of the one or more ligation products; and
(e) amplifying the one or more ligation products in the presence of a polymerase to provide one or more amplification products,
wherein the one or more probes each include a bridge oligonucleotide comprising a 3' overhang and having Formula (II):

3' - [D] - 5' (II),

wherein [D] comprises between 5 and 50 nucleotides, and where at least a portion of the nucleotide sequence of [D] is complementary to the constant region, and the 3' overhang is a T overhang.

Also disclosed is a method of amplifying one or more target nucleic acids in a sample, comprising: annealing one or more probes to the one or more target nucleic acid molecules in the sample to form one or more probe-target nucleic acid molecule complexes, wherein the one or more probes each comprise a constant region and a unique region, wherein the unique region of each probe includes a nucleotide sequence which hybridizes to a complementary nucleic acid sequence within the one or more target nucleic acid molecules; extending the one or more probe-target nucleic acid molecule complexes to form one or more extended probe-target nucleic acid molecule complexes; generating one or more ligation products from the one or more extended probe-target nucleic acid molecule complexes; introducing one or more primers to the generated one or more ligation products, wherein the one or more primers hybridize to one or more complementary portions of the constant region within each of the one or more ligation products; and amplifying the one or more ligation products in the presence of a polymerase to provide one or more amplification products.

In some embodiments, the method further comprises sequencing the one or more amplification products. In some embodiments, the sequencing is a next-generation sequencing method. In some embodiments, the next generation sequencing method utilizes a nanopore.

In some embodiments, the one or more ligation products include intramolecular circular ligation products, intermolecular circular ligation products, intermolecular linear ligation products, or any mixture thereof. In some environments, the one or more ligation products include at least two of intramolecular circular ligation products, intermolecular circular ligation products, and intermolecular linear ligation products.

In some embodiments, the one or more ligation products include intramolecular ligation products derived from a single extended probe-target nucleic acid molecule complex. In some embodiments, the one or more ligation products include intermolecular ligation products formed from two or more extended probe-target nucleic acid molecule complexes. In some embodiments, the intermolecular ligation products are intermolecular circular ligation products. In some embodiments, the intermolecular ligation products are intermolecular linear ligation products.

In some embodiments, the method further comprises introducing one or more adapter oligonucleotides to a mixture including the one or more extended probe-target nucleic acid molecule complexes. In some embodiments, the introduction of the one or more adapter oligonucleotides facilitates the formation of one or more intermolecular linear ligation products.

In some embodiments, the one or more primers are forward and reverse inverse PCR primers. In some embodiments, the forward inverse PCR primer hybridizes to a first portion of the constant region within each of the one or more ligation products. In some embodiments, the reverse inverse PCR primer hybridizes to a second portion of the constant region within each of the one or more ligation products. In some embodiments, the one or more primers are universal primers. In some embodiments, the one or more primers are target-specific primers. In some embodiments, the amplification is by inverse polymerase chain reaction. In some embodiments, the amplification is by rolling circle amplification.

In some embodiments, the one or more probes further comprises a barcode. In some embodiments, each of the one or more probes are derived from: (i) a linker oligonucleotide comprising a 5' phosphate group and having Formula (IA):

5' - [A] - [B]ₓ - [C]_{y} - 3' (IA),

wherein [A] is the "constant region" and includes between about 5 and about 60 nucleotides; [B] is an "identifier region" including an identifier nucleic acid sequence; [C] is the "unique region" and includes between about 5 and about 100 nucleotides and, where [C] of Formula (IA) hybridizes to the complementary portion of each of the one or more target nucleic acid molecules in the sample; x is 0 or 1; and y is 0 or 1; and

3' - [D] - 5' (II),

wherein includes [D] comprises between about 5 and about 50 nucleotides, and wherein at least a portion of the nucleotide sequence of [D] is complementary to the "constant region" [A] of Formula (IA).

In some embodiments, [A] comprises between about 15 and about 35 nucleotides. In some embodiments, [A] comprises between about 20 and about 35 nucleotides. In some embodiments, [A] comprises between about 25 and about 35 nucleotides. In some embodiments, the linker oligonucleotide comprises any one of SEQ ID NOS: 1 - 3.

A second aspect of the present invention is a kit comprising: (a) a first linker oligonucleotide comprising a 5' phosphate group and having Formula (IA):

5' - [A] - [B]x - [C]y - 3' (IA),

wherein [A] is a "constant region" including between about 5 and about 60 nucleotides; [B] is an "identifier region" including an identifier nucleic acid sequence; [C] is a "unique region" including between about 5 and about 100 nucleotides; x is 0 or 1; and y is 0 or 1; and
(b) a bridge oligonucleotide comprising a 3' overhang and having Formula (II):

   3' - [D] - 5' (II),
wherein [D] comprises between 5 and 50 nucleotides, and where at least a portion of the nucleotide sequence of [D] of Formula (II) is complementary to the "constant region" [A] of Formula (IA) and the 3' overhang is a T overhang.

In some embodiments, [A] comprises between about 15 and about 35 nucleotides. In some embodiments, [A] comprises between about 20 and about 35 nucleotides. In some embodiments, [A] comprises between about 25 and about 35 nucleotides.

In some embodiments, the kit further comprises a polymerase. In some embodiments, the polymerase is a DNA polymerase.

In some embodiments, the kit further comprises a ligase. In some embodiments, the ligase is a T4 ligase.

In some embodiments, the kit further comprises one or more primers. In some embodiments, the one or more primers are each at least partially complementary to a portion of the "constant region" [A] of the first linker oligonucleotide having Formula (IA).

In some embodiments, the kit further comprises a second linker oligonucleotide comprising a 5' phosphate group and having Formula (IC):

5' - [A] - 3' (IC).

In some embodiments, [A] of Formula (IC) comprises between about 15 and about 35 nucleotides. In some embodiments, [A] of Formulas (IA) and (IC) are the same.

In some embodiments, the first linker oligonucleotide has Formula (IB):

5' - [A] - [B]x - [C] - 3' (IB).

In some embodiments, [A] of Formula (IB) comprises between about 15 and about 35 nucleotides. In some embodiments, [A] of Formula (IB) comprises between about 20 and about 35 nucleotides. In some embodiments, [A] of Formula (IB) comprises between about 25 and about 35 nucleotides. In some embodiments, [A] of Formula (IB) comprises any one of SEQ ID NOS: 1 - 3.

In some embodiments, the kit further comprises one or more primers. In some embodiments, the one or more primers are forward and reverse inverse PCR primers. In some embodiments, the forward inverse PCR primer hybridizes to a first portion of the constant region within each of the one or more ligation products. In some embodiments, the reverse inverse PCR primer hybridizes to a second portion of the constant region within each of the one or more ligation products. In some embodiments, the one or more primers are universal primers. In some embodiments, the one or more primers are target-specific primers.

A third aspect of the present inevention is a master mix comprising: (a) one or more ligation products, wherein each ligation product of the one or more ligation products includes a probe derived from:
(a) a linker oligonucleotide comprising a 5' phosphate group and having Formula (IA):

   5' - [A] - [B]x - [C]y - 3' (IA),

   wherein [A] is a "constant region" comprising between about 5 and about 60 nucleotides; [B] is an "identifier region" including an identifier nucleic acid sequence; [C] is a "unique region" including between about 5 and about 100 nucleotides; x is 0 or 1; and y is 0 or 1; and
   a bridge oligonucleotide comprising a 3' overhang and having Formula (II):

      3' - [D] - 5' (II),
   wherein [D] comprises between 5 and 50 nucleotides, and wherein at least a portion of the nucleotide sequence of [D] is complementary to the "constant region" [A] of Formula (IA) and the 3' overhang is a T overhang; and
(b) one or more primers each having a nucleotide sequence which is at least partially complementary to a portion of the "constant region" [A] included within the one or more ligation products. In some embodiments, the master mix further comprises a polymerase.

In some embodiments, the one or more ligation products include intramolecular circular ligation products, intermolecular circular ligation products, intermolecular linear ligation products, or any mixture thereof. In some embodiments, constant region [A] comprises between about 15 and about 35 nucleotides. In some embodiments, constant region [A] comprises between about 20 and about 35 nucleotides. In some embodiments, constant region [A] comprises between about 25 and about 35 nucleotides. In some embodiments, the one or more primers are forward and reverse inverse PCR primers. In some embodiments, the forward inverse PCR primer hybridizes to a first portion of the constant region [A] within each of the one or more ligation products. In some embodiments, the reverse inverse PCR primer hybridizes to a second portion of the constant region [A] within each of the one or more ligation products. In some embodiments, the one or more primers are universal primers. In some embodiments, the one or more primers are target-specific primers.

A fourth aspect of the present invention is a master mix comprising: (a) one or more ligation products, wherein each ligation product of the one or more ligation products includes a probe derived from:
(a) a linker oligonucleotide comprising a 5' phosphate group and having Formula (IA):

   5' - [A] - [B]x - [C] - 3' (IB),

   wherein [A] is a "constant region" comprising between 5 and 60 nucleotides; [B] is an "identifier region" including an identifier; [C] is a "unique region" comprising between 5 and 100 nucleotides; and x is 0 or 1;
   a bridge oligonucleotide comprising a 3' overhang and having Formula (II):

      3' - [D] - 5' (II),
   wherein [D] comprises between 5 and 50 nucleotides, and wherein at least a portion of the nucleotide sequence of [D] is complementary to the "constant region" [A] of Formula (IB) and the 3' overhang is a T overhang; and
(b) one or more primers having a nucleotide sequence which is at least partially complementary to a portion of the "constant region" [A] included within the one or more ligation products. In some embodiments, the master mix further comprises a polymerase.

In some embodiments, the one or more ligation products include intramolecular circular ligation products, intermolecular circular ligation products, intermolecular linear ligation products, or any mixture thereof. In some embodiments, the one or more primers are forward and reverse inverse PCR primers. In some embodiments, the forward inverse PCR primer hybridizes to a first portion of the constant region [A] within each of the one or more ligation products. In some embodiments, the reverse inverse PCR primer hybridizes to a second portion of the constant region [A] within each of the one or more ligation products. In some embodiments, the one or more primers are universal primers. In some embodiments, the one or more primers are target-specific primers.

A fifth aspect of the present invention is a composition comprising: (a) a probe; and (b) an adapter oligonucleotide,
where the probe is derived from:
a first linker oligonucleotide comprising a 5' phosphate group and having Formula (IB):

   5' - [A] - [B]x - [C] - 3' (IB),
wherein [A] is a "constant region" comprising between about 5 and about 60 nucleotides; [B] is an "identifier region" including an identifier; [C] is a "unique region" comprising between 5 and 100 nucleotides; and x is 0 or 1;
a first bridge oligonucleotide comprising a 3' overhang and having Formula (II):

   3' - [D] - 5' (II),
wherein [D] comprises between about 5 and about 50 nucleotides, and wherein at least a portion of the nucleotide sequence of [D] of the first bridge oligonucleotide is complementary to the "constant region" [A] of the first linker oligonucleotide and the 3' overhang is a T overhang; and
where the adapter oligonucleotide is derived from:
   a second linker comprising a 5' phosphate group and having Formula (IC):

      5' - [A] - 3' (IC);

      and
   a second bridge oligonucleotide comprising a 3' overhang and having Formula (II):

      3' - [D] - 5' (II),
   wherein at least a portion of the nucleotide sequence of [D] of the second bridge oligonucleotide is complementary to the "constant region" [A] of the second linker oligonucleotide, and the 3' overhang is a T overhang. In some embodiments, [A] of Formulas (IB) and (IC) are the same.

In some embodiments, the composition further comprises at least one target nucleic acid, wherein [C] of Formula (IB) is complementary to at least a portion of the at least one target nucleic acid. In some embodiments, the composition further comprises a polymerase. In some embodiments, the composition further comprises at least one ligation product, wherein the at least one ligation product is derived from the at least one target nucleic acid and the probe. In some embodiments, the one or more ligation products include intramolecular circular ligation products, intermolecular circular ligation products, intermolecular linear ligation products, or any mixture thereof. In some embodiments, the at least one ligation product comprises an intermolecular circular ligation product. In some embodiments, the at least one ligation product comprises an intermolecular linear ligation product. In some embodiments, the at least one ligation product comprises an intramolecular ligation product.

Also disclosed is a probe-target nucleic acid molecule complex comprising:
(a) a target nucleic acid molecule; and
(b) a probe having a constant region and a unique region, wherein the unique region of the probe is complementary to a portion of the target nucleic acid molecule; and wherein the probe is derived from:
   a linker oligonucleotide comprising a 5' phosphate group and having Formula (IA):

      5' - [A] - [B]ₓ - [C]_{y} - 3' (IA),
   wherein [A] is the "constant region" and includes between about 5 and about 60 nucleotides; [B] is an "identifier region" including an identifier nucleic acid sequence; [C] is the "unique region" and includes between about 5 and about 100 nucleotides and, where x is 0 or 1; and y is 0 or 1; and
   a bridge oligonucleotide comprising a 3' overhang and having Formula (II):

      3' - [D] - 5' (II),
   wherein includes [D] comprises between about 5 and about 50 nucleotides, and wherein at least a portion of the nucleotide sequence of [D] of Formula (II) is complementary to the "constant region" [A] of the linker oligonucleotide of Formula (IA).

A sixth aspect of the present invention is the use of the kit of claim the second aspect in the amplification and/or sequencing of one or more target nucleic acid sequences.

A seventh aspect of the present invention is a method of capturing or enriching at least one target nucleic acid molecule in a library of nucleic acid molecules, the method comprising:
(a) annealing at least one probe to the at least one target nucleic acid molecule in the library of nucleic acid molecules to form at least one probe-target nucleic acid molecule complex, wherein the at least one probe comprises a constant region and a unique region, wherein the unique region includes a nucleotide sequence which hybridizes to a complementary nucleic acid sequence within the at least one target nucleic acid molecule;
(b) extending the at least one probe-target nucleic acid molecule complex to form at least one extended probe-target nucleic acid molecule complex, and performing A-tailing;

generating one or more ligation products from the at least one extended probe-target nucleic acid molecule complex,
wherein the at least one probe includes a bridge oligonucleotide comprising a 3' overhang and having Formula (II):

   3' - [D] - 5' (II),

   wherein [D] comprises between 5 and 50 nucleotides, and where at least a portion of the nucleotide sequence of [D] is complementary to the constant region, and the 3' overhang is a T overhang.

### BRIEF DESCRIPTION OF THE FIGURES

For a general understanding of the features of the disclosure, reference is made to the drawings. In the drawings, like reference numerals have been used throughout to identify identical elements.
FIG. 1 illustrates the formation of a probe from a linker oligonucleotide and a bridge oligonucleotide, wherein the linker oligonucleotide includes a constant region, an identifier region, and a unique region, in accordance with one embodiment of the present disclosure. In some embodiments, the linker oligonucleotide comprises Formula (IA). In other embodiments, the linker oligonucleotide comprises Formula (IB).
FIG. 2 illustrates the formation of an adapter oligonucleotide from a linker oligonucleotide and a bridge oligonucleotide, wherein the linker oligonucleotide includes a constant region in accordance with one embodiment of the present disclosure. In some embodiments, the linker oligonucleotide comprises Formula (IA). In other embodiments, the linker oligonucleotide comprises Formula (IC).
FIG. 3 sets forth a method of preparing one or more ligation products, amplifying the one or more prepared ligation products, and/or performing one or more downstream operations on the amplification products.
FIG. 4 sets forth a workflow for preparing a probe-target nucleic acid molecule complex for ligation.
FIG. 5 illustrates a probe-sample mixture comprising a probe derived from a linker oligonucleotide (the linker oligonucleotide including at least constant region and unique region) and a sample nucleic acid molecule (e.g. a DNA fragment) in accordance with one embodiment of the present disclosure.
FIG. 6 illustrates a probe-sample mixture comprising a denatured probe derived from a linker oligonucleotide (the linker oligonucleotide including at least constant region and unique region) and a denatured sample nucleic acid molecule (e.g. a DNA fragment) in accordance with one embodiment of the present disclosure.
FIG. 7 illustrates a probe-target nucleic acid molecule complex, which is the product of the hybridization of a portion of a probe (such as a probe including at least a constant region and a unique region) with a target nucleic acid molecule in accordance with one embodiment of the present disclosure.
FIG. 8 illustrates an extended probe-target nucleic acid molecule complex, where the extended probe-target nucleic acid molecule complex includes a 3' A-tail in accordance with one embodiment of the present disclosure.
FIG. 9 illustrates the product of a ligation reaction, namely an intramolecular circular ligation product, derived from a single extended probe-target nucleic acid molecule complex.
FIG. 10 illustrates the product of a ligation reaction between two or more extended probe-target nucleic acid molecule complexes. In this embodiment, an intermolecular circular ligation product is formed.
FIG. 11 illustrates the product of a ligation reaction between two or more extended probe-target nucleic acid molecule complexes and an adapter oligonucleotide. In this embodiment, an intermolecular linear ligation product is formed.
FIG. 12 illustrates the various different types of ligation products that may be formed including intramolecular circular ligation products, intermolecular circular ligation products, and intermolecular linear ligation products.
FIG. 13A illustrates the hybridization of forward and reverse inverse PCR primers to a constant region of an intramolecular circular ligation product, where the intramolecular circular ligation product is derived from a single extended probe-target nucleic acid molecule complex (and hence a single probe).
FIG. 13B illustrates the hybridization of forward and reverse inverse PCR primers to constant regions of an intermolecular circular ligation product, where the intermolecular circular ligation product is derived from two extended probe-target nucleic acid molecule complexes (and hence two probes).
FIG. 13C illustrates the hybridization of a forward inverse PCR primer to a constant region of an intermolecular linear ligation product, where the intermolecular linear ligation product is derived from a single extended probe-target nucleic acid molecule complex (and hence a single probe) and an adapter oligonucleotide. FIG. 13C further illustrates that a reverse inverse PCR primer is hybridized to a constant region of the adapter molecule included within the intermolecular linear ligation product.
FIG. 13D illustrates the hybridization of forward and reverse inverse PCR primers to constant regions of an intermolecular linear ligation product, where the intermolecular linear ligation product is derived from two extended probe-target nucleic acid molecule complexes (and hence two probes). FIG. 13D further illustrates that a reverse inverse PCR primer is hybridized to a constant region of the adapter molecule included within the intermolecular linear ligation product.
FIG. 14A illustrates the hybridization of a universal primer to a constant region of an intramolecular circular ligation product, where the intramolecular circular ligation product is derived from a single extended probe-target nucleic acid molecule complex (and hence a single probe).
FIG. 14B illustrates the hybridization of universal primers to constant regions of an intermolecular circular ligation product, where the intermolecular circular ligation product is derived from two extended probe-target nucleic acid molecule complexes (and hence two probes).
FIG. 14C illustrates the hybridization of a universal primer to a constant region of an intermolecular linear ligation product, where the intermolecular linear ligation product is derived from a single extended probe-target nucleic acid molecule complex (and hence a single probe) and an adapter oligonucleotide. FIG. 14C further illustrates that a universal primer is hybridized to a constant region of the adapter molecule included within the intermolecular linear ligation product.
FIG. 14D illustrates the hybridization of universal primers to constant regions of an intermolecular linear ligation product, where the intermolecular linear ligation product is derived from two extended probe-target nucleic acid molecule complexes (and hence two probes). FIG. 14D further illustrates that a universal primer is hybridized to a constant region of the adapter molecule included within the intermolecular linear ligation product.
FIG. 15A illustrates the hybridization of a target-specific RCA primer to a constant region of an intramolecular circular ligation product, where the intramolecular circular ligation product is derived from a single extended probe-target nucleic acid molecule complex (and hence a single probe).
FIG. 15B illustrates the hybridization of target-specific RCA primers to constant regions of an intermolecular circular ligation product, where the intermolecular circular ligation product is derived from two extended probe-target nucleic acid molecule complexes (and hence two probes).
FIG. 15C illustrates the hybridization of a target-specific RCA primer to a constant region of an intermolecular linear ligation product, where the intermolecular linear ligation product is derived from a single extended probe-target nucleic acid molecule complex (and hence a single probe) and an adapter oligonucleotide. FIG. 15C further illustrates that a target-specific RCA primer is hybridized to a constant region of the adapter molecule included within the intermolecular linear ligation product.
FIG. 15D illustrates the hybridization of target-specific RCA primers to constant regions of an intermolecular linear ligation product, where the intermolecular linear ligation product is derived from two extended probe-target nucleic acid molecule complexes (and hence two probes). FIG. 15D further illustrates that a target-specific RCA primer is hybridized to a constant region of the adapter molecule included within the intermolecular linear ligation product.
FIG. 16 illustrates probes generated from linker oligonucleotides having any one of SEQ ID NOS: 1 - 3 and a bridge oligonucleotide having SEQ ID NO: 5.
FIG. 17 illustrates an adapter oligonucleotide generated from a linker oligonucleotide having any one of SEQ ID NO: 4 and a bridge oligonucleotide having SEQ ID NO: 5.
FIG. 18 illustrates the relative locations of the probes 23, 26, and 32 (see FIG. 16) on a Lambda DNA template. Expected product lengths were about 238bp, about 104bp and about 57bp.
FIG. 19 demonstrates feasibility date with phage lambda DNA. All three expected fragment sizes were observed.
FIG. 20 illustrates that a significant amount of off-target captures as well as on-target captures were observed.
FIG. 21 illustrates the artifacts found in the phage lambda study.
FIG. 22 illustrates that capture primer pairs NP_1 and N_2 were able to find NRAS gene variants in a Human Genomic DNA (HD734) with that had an expected Allele frequency of about 1.3%.
FIG. 23 illustrates that capture primer pairs NP_1 and N_2 were able to find NRAS gene variants in a Human Genomic DNA (HD734) with that had an expected Allele frequency of about 1.3%. The correct insert was found in multiple Sequenced Reads.
FIG. 24 depicts that read lengths with inserts ranging from about 1,083bp to as low as about 331bp were observed.
FIG. 25 illustrates that capture primer pairs NP_1 and N_2 were able to find PIK3CA gene variants in a Human Genomic DNA (HD734) with that had an expected Allele frequency of about 30%. The correct insert was found in multiple Sequenced Reads.
FIG. 26 illustrates that capture primer pairs NP_1 and N_2 were able to find PIK3CA gene variants in a Human Genomic DNA (HD734) with that had an expected Allele frequency of about 30%. The correct insert was found in multiple Sequenced Reads with both minus strand and forward strand primer.
FIG. 27 illustrates that large inserts were able to be achieved, some with the correct variant cells.
FIG. 28 depicts that the PIK3CA variant with an expected Allele Frequency of about 30% was observed in Reads.
FIG. 29 provides a table which highlights the results of the study described wherein using Human gDNA (HD734) where variable insert sizes was observed. Variants down to 1.3% were able to be detected. Optimalization of final product cleanup with spri Beads would allow the further identification of other variants.
FIGS. 30 and 31 illustrate that it was possible to locate a chimeric artifact or translocation within the KRAS gene using the methods described herein.

### DETAILED DESCRIPTION

It should also be understood that, unless clearly indicated to the contrary, in any methods claimed herein that include more than one step or act, the order of the steps or acts of the method is not necessarily limited to the order in which the steps or acts of the method are recited.

### Definitions

As used herein, the singular terms "a," "an," and "the" include plural referents unless context clearly indicates otherwise. Similarly, the word "or" is intended to include "and" unless the context clearly indicates otherwise. The term "includes" is defined inclusively, such that "includes A or B" means including A, B, or A and B.

As used herein in the specification and in the claims, "or" should be understood to have the same meaning as "and/or" as defined above. For example, when separating items in a list, "or" or "and/or" shall be interpreted as being inclusive, e.g., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (e.g. "one or the other but not both") when preceded by terms of exclusivity, such as "either," "one of," "only one of" or "exactly one of." "Consisting essentially of," when used in the claims, shall have its ordinary meaning as used in the field of patent law.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b, and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used herein, the terms "amplification" or "amplify" refer to a process in which a copy number increases. Amplification may be a process in which replication occurs repeatedly over time to form multiple copies of a template. Amplification can produce an exponential or linear increase in the number of copies as amplification proceeds. Exemplary amplification strategies include polymerase chain reaction (PCR), loop-mediated isothermal amplification (LAMP), rolling circle replication (RCA), cascade-RCA, nucleic acid based amplification (NASBA), and the like. Also, amplification can utilize a linear or circular template. Amplification can be performed under any suitable temperature conditions, such as with thermal cycling or isothermally. Furthermore, amplification can be performed in an amplification mixture (or reagent mixture), which is any composition capable of amplifying a nucleic acid target, if any, in the mixture. PCR amplification relies on repeated cycles of heating and cooling (i.e., thermal cycling) to achieve successive rounds of replication. PCR can be performed by thermal cycling between two or more temperature setpoints, such as a higher denaturation temperature and a lower annealing/extension temperature, or among three or more temperature setpoints, such as a higher denaturation temperature, a lower annealing temperature, and an intermediate extension temperature, among others. PCR can be performed with a thermostable polymerase, such as Taq DNA polymerase. PCR generally produces an exponential increase in the amount of a product amplicon over successive cycles.

As used herein, the term "complementary" generally refers to the capability for precise pairing between two nucleotides. The term "complementary" refers to the ability to form favorable thermodynamic stability and specific pairing between the bases of two nucleotides at an appropriate temperature and ionic buffer conditions. Complementarity is achieved by distinct interactions between the nucleobases adenine, thymine (uracil in RNA), guanine and cytosine, where adenine pairs with thymine or uracil, and guanine pairs with cytosine. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence.

As used herein, the terms "complementary DNA" or "cDNA" refer to DNA which is copied from RNA. In some embodiments, cDNA is the DNA produced on an RNA template by the action of reverse transcriptase (RNA-dependent DNA-polymerase. cDNA copied from mRNA does not include the various non-coding sequences characteristic of genomic DNA.

As used herein, the term "enrichment" refers to the process of increasing the relative abundance of a population of molecules, e.g. nucleic acid molecules, in a sample relative to the total amount of the molecules initially present in the sample before treatment. Thus, an enrichment step provides a percentage or fractional increase rather than directly increasing for example, the copy number of the nucleic acid sequences of interest as amplification methods, such as a polymerase chain reaction, would.

As used herein, the term "hybridize" refers to the base-pairing between different nucleic acid molecules consistent with their nucleotide sequences.

As used herein, the phrase "next generation sequencing (NGS)" refers to sequencing technologies having high-throughput sequencing as compared to traditional Sanger- and capillary electrophoresis-based approaches, wherein the sequencing process is performed in parallel, for example producing thousands or millions of relatively small sequence reads at a time. Some examples of next generation sequencing techniques include, but are not limited to, sequencing by synthesis, sequencing by ligation, and sequencing by hybridization. These technologies produce shorter reads (anywhere from about 25 to about 500 bp) but many hundreds of thousands or millions of reads in a relatively short time. The term "next-generation sequencing" refers to the so-called parallelized sequencing-by-synthesis or sequencing-by-ligation platforms currently employed by Illumina (e.g. the iSEQ, MiniSEQ, MiSEQ, NextSEQ, NoveSEQ sequencing platforms), and Thermo Fisher Scientific (e.g. the Ion Personal Genome Machine^{™} (PGM^{™}) System). Next-generation sequencing methods may also include nanopore sequencing methods with electronic-detection (Oxford Nanopore (e.g. MinION, GridION, SmidgION, and PromethION devices) and Roche Diagnostics).

As used herein, the terms "nucleic acid" or "polynucleotide" (used interchangeably herein) refer to a polymeric form of nucleotides of any length, either deoxyribonucleotides or ribonucleotides, or analogs thereof. Polynucleotides may have any three-dimensional structure, and may perform any function, known or unknown. Unless specifically limited, the terms encompass nucleic acids or polynucleotides including known analogues of natural nucleotides that have similar binding properties as the reference nucleic acid and are metabolized in a manner similar to naturally occurring nucleotides. Non-limiting examples of polynucleotides include coding or non-coding regions of a gene or gene fragment, loci (locus) defined from linkage analysis, exons, introns, messenger RNA (mRNA), transfer RNA, ribosomal RNA, ribozymes, cDNA, synthetic polynucleotides, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component. Unless otherwise indicated, a particular nucleic acid sequence also implicitly encompasses conservatively modified variants thereof (e.g., degenerate codon substitutions), alleles, orthologues, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

As used herein, the term "oligonucleotide," refers to an oligomer of nucleotide or nucleoside monomer units wherein the oligomer optionally includes non-nucleotide monomer units, and/or other chemical groups attached at internal and/or external positions of the oligomer. The oligomer can be natural or synthetic and can include naturally-occurring oligonucleotides, or oligomers that include nucleosides with non-naturally-occurring (or modified) bases, sugar moieties, phosphodiester-analog linkages, and/or alternative monomer unit chiralities and isomeric structures (e.g., 5'- to 2'-linkage, L-nucleosides, α-anomer nucleosides, β-anomer nucleosides, locked nucleic acids (LNA), peptide nucleic acids (PNA)).

As used herein, a "reaction" between any two different reactive groups (such as any two reactive groups of a reagent and a particle) may mean that a covalent linkage is formed between the two reactive groups (or two reactive functional groups); or may mean that the two reactive groups (or two reactive functional groups) associate with each other, interact with each other, hybridize to each other, hydrogen bond with each other, etc. In some embodiments, the "reaction" includes binding events, e.g. binding events between reactive function groups or binding events between first and second members of a pair of specific binding entities.

As used herein, the term "primer" refers to an oligonucleotide which binds to a specific region of a single-stranded template nucleic acid molecule and initiates nucleic acid synthesis via a polymerase-mediated enzymatic reaction, extending from the 3' end of the primer and complementary to the sequence of the template molecule. PCR amplification primers can be referred to as 'forward' and 'reverse' primers, one of which is complementary to a nucleic acid strand and the other of which is complementary to the complement of that strand. Typically, a primer comprises fewer than about 100 nucleotides and preferably comprises fewer than about 30 nucleotides. Exemplary primers range from about 5 to about 25 nucleotides. Primers can comprise, for example, RNA and/or DNA bases, as well as non-naturally occurring bases. The directionality of the newly forming strand (the daughter strand) is opposite to the direction in which DNA polymerase moves along the template strand. In some cases, a target capture primer specifically hybridizes to a target polynucleotide under hybridization conditions. Such hybridization conditions can include, but are not limited to, hybridization in isothermal amplification buffer (20 mM Tris-HCl, 10 mM (NH4)2SO4), 50 mM KCl, 2 mM MgSO4, 0.1% TWEEN^{®} 20, pH 8.8 at 25° C) at a temperature of about 40°C, 45°C, 50°C, 55°C, 60°C, 65°C, or 70°C.

As used herein, the term "sequence," when used in reference to a nucleic acid molecule, refers to the order of nucleotides (or bases) in the nucleic acid molecules. In cases, where different species of nucleotides are present in the nucleic acid molecule, the sequence includes an identification of the species of nucleotide (or base) at respective positions in the nucleic acid molecule. A sequence is a property of all or part of a nucleic acid molecule. The term can be used similarly to describe the order and positional identity of monomeric units in other polymers such as amino acid monomeric units of protein polymers.

As used herein, the term "sequencing" refers to the determination of the order and position of bases in a nucleic acid molecule. More particularly, the term "sequencing" refers to biochemical methods for determining the order of the nucleotide bases, adenine, guanine, cytosine, and thymine, in a DNA oligonucleotide. Sequencing, as the term is used herein, can include without limitation parallel sequencing or any other sequencing method known of those skilled in the art, for example, chain-termination methods, rapid DNA sequencing methods, wandering-spot analysis, Maxam-Gilbert sequencing, dye- terminator sequencing, or using any other modern automated DNA sequencing instruments.

As used herein, the term "substantially" means the qualitative condition of exhibiting total or near-total extent or degree of a characteristic or property of interest. In some embodiments, "substantially" means within about 5%. In some embodiments, "substantially" means within about 10%. In some embodiments, "substantially" means within about 15%. In some embodiments, "substantially" means within about 20%.

As used herein, the terms "target" or "target sequence" refer to nucleic acid molecule sequences of interest, e.g. those which hybridize to oligonucleotide probes.

As used herein, the term "universal primer" refers to a primer that can hybridize to and support amplification of target polynucleotides having a shared complementary universal primer binding site. Similar, the term "universal primer pair" refers to a forward and reverse primer pair that can hybridize to and support PCR amplification of target polynucleotides having shared complementary forward and reverse universal primer binding sites. Such universal primer(s) and universal primer binding site(s) can allow single or double primer mediated universal amplification (e.g., universal PCR) of target polynucleotide regions of interest. The headings provided herein are for convenience only and do not interpret the scope or meaning of the disclosed embodiments.

### OVERVIEW

The present disclosure is directed to compositions, kits, and methods which facilitate the amplification of a unidirectional primer extension product. In particular, the compositions, kits, and methods described herein facilitate the amplification of a unidirectional primer extension product without the need to incorporate a second polymerase chain reaction primer binding target on a distal end of an initial single-stranded nucleic acid molecule primer extension product. The present disclosure is also directed to methods of amplifying a target enriched sample, such as a target enriched sample prepared using any one of the methods described herein.

In view of the foregoing, in one aspect of the present disclosure is a method of amplifying a plurality of target nucleic acid molecules, e.g. between about 1 and about 10000 target nucleic acid molecules, or between about 1 and about 5000 target nucleic acid molecules, or between about 1 and about 1000 target nucleic acid molecules, using a unidirectional primer extension approach, where the method does not require the incorporation of a second polymerase chain reaction primer binding target on a distal end of an initial single-stranded nucleic acid molecule primer extension product.

Additionally, the present disclosure is also directed to sequencing the plurality of amplified target nucleic acid molecules, such as sequencing the plurality of amplified target nucleic acid molecules using a next-generation sequencing approach. Additionally, the present disclosure is also directed to methods of sequencing using a target enriched sample, such as a target enriched sample prepared using any one of the methods described herein. In some embodiments, the method of the present disclosure generates a library of enriched target nucleic acid molecules to be sequenced. As such, the methods of the present disclosure can be used as a part of a sequencing protocol, including a high throughput single molecule sequencing protocol. In some embodiments, the plurality of amplified target nucleic acid molecules may optionally incorporate one or more identifiers (e.g. unique identifiers, barcodes, etc.) for molecular identification and sample identification, such as described in U.S. Publication No. 2020/0032244, and in U.S. Patent Nos. 7,393,665, 8,168,385, 8,481,292, 8,685,678, and 8,722,368.

### COMPOSITIONS

### Linker Oligonucleotides

One aspect of the present disclosure is a linker oligonucleotide having Formula (IA):

5' - [A] - [B]x - [C]y - 3' (IA),

wherein the linker oligonucleotide comprises a 5' phosphate group, and wherein [A] is a "constant region" which comprises between about 5 and about 60 nucleotides; [B] is an "identifier region" which includes an identifier nucleic acid sequence; [C] is a "unique region" which comprises between about 5 and about 100 nucleotides and is complementary to a portion of a target nucleic acid molecule; x is 0 or 1; and y is 0 or 1. In some embodiments, when y is 0, then x is also 0. In some embodiments, x is 1 and y is 1. In some embodiments, x is 0 and y is 1.

In some embodiments, [A] comprises between 10 and 50 nucleotides. In another embodiments, [A] comprises between about 20 and about 35 nucleotides. In yet other In some embodiments, [A] is complementary to a portion of a nucleotide sequence of a bridge oligonucleotide having Formula (II). In some embodiments, the constant regions are not complementary to any part of the human genome that would allow nonspecific priming products.

In some embodiments, [B] includes one of a barcode, a multiplex identifier, or a unique molecular identifier. A s used herein, the term "barcode" refers to a nucleic acid sequence that can be detected and identified. In some embodiments, the barcodes comprise between about 5 and about 20 nucleotides, such that in a sample, the nucleic acids incorporating the barcodes can be distinguished or grouped according to the barcodes. In some embodiments, the barcodes comprise between about 5 and about 15 nucleotides. In some embodiments, the barcodes comprise between about 5 and about 10 nucleotides. In some embodiments, the barcodes comprise between about 10 and about 15 nucleotides. In some embodiments, the barcodes comprise about 8, about 9, about 10, about 11, about 12, about 13, about 14, or about 15 nucleotides.

In some embodiments, the barcode has the Formula:

(W)(N)(N)(N)(N)(N)(W)(N)(N)(N)(N)(N)(W),

or

(N)(W)(N)(N)(N)(W)(N)(N)(N),

where N includes (in the aggregate) about 25% adenosine; about 25% guanine; about 25% cytosine; and about 25% thymine; and W includes (in the aggregate) about 50% adenosine and about 50% thymine.

As used herein, the term "multiplex identifier" or "MID" refers to a barcode that identifies a source of a target nucleic acid (e.g., a sample from which the nucleic acid is derived). In some embodiments, all or substantially all the target nucleic acids from the same sample will share the same MID. In some embodiments, nucleic acids from different sources or samples can be mixed and sequenced simultaneously. In some embodiments, by using the MIDs the sequence reads (obtained during a sequencing step, such as described herein) can be assigned to individual samples from which the target nucleic acids originated.

As used herein, the term "unique molecular identifier" or "UID" refers to a barcode that identifies a nucleic acid to which it is attached. In some embodiments, all or substantially all the target nucleic acids from the same sample will have different UIDs. In some embodiments, all or substantially all of the progeny (e.g., amplicons) derived from the same original target nucleic acid will share the same UID.

Examples of barcodes, multiplex identifiers, or unique molecular identifiers are described in U.S. Publication No. 2020/0032244, and in U.S. Patent Nos. 7,393,665, 8,168,385, 8,481,292, 8,685,678, and 8,722,368, and in PCT Publication No. WO/2018/138237.

In some embodiments, [B] comprises between about 5 and about 80 nucleotides and includes a barcode, a multiplex identifier, or a unique molecular identifier. In other embodiments, [B] comprises between about 5 and about 60 nucleotides and includes a barcode, a multiplex identifier, or a unique molecular identifier. In yet other embodiments, [B] comprises between about 5 and about 40 nucleotides and includes a barcode, a multiplex identifier, or a unique molecular identifier. In further embodiments, [B] comprises between about 5 and about 25 nucleotides and includes a barcode, a multiplex identifier, or a unique molecular identifier.

In some embodiments, [C] comprises between about 5 and about 75 nucleotides. In other embodiments, [C] comprises between about 5 and about 50 nucleotides. In yet other embodiments, [C] comprises between about 5 and about 30 nucleotides.

In some embodiments, the linker oligonucleotide of Formula (IA) has Formula (IB):

5' - [A] - [B]ₓ - [C] - 3' (IB)

wherein the linker oligonucleotide comprises a 5' phosphate group, and wherein [A] is a "constant region" which comprises between about 5 and about 60 nucleotides; [B] is an "identifier region" and includes an identifier; [C] is a "unique region" which comprises between about 5 and about 100 nucleotides and is complementary to a portion of a target nucleic acid molecule; and x is 0 or 1.

In some embodiments, [A] comprises between about 10 and about 50 nucleotides. In other embodiments, [A] comprises between about 15 and about 40 nucleotides. In yet other embodiments, [A] comprises between about 15 and about 35 nucleotides. In yet other embodiments, [A] comprises between about 15 and about 30 nucleotides. In yet other embodiments, [A] comprises between about 20 and about 35 nucleotides.

In some embodiments, [A] is complementary to a portion of a nucleotide sequence of a bridge oligonucleotide having Formula (II). In some embodiments, [B] includes one of a barcode, a multiplex identifier, or a unique molecular identifier, such as described above with regard to Formula (IA).

In some embodiments, [B] comprises between about 5 and about 80 nucleotides and includes a barcode, a multiplex identifier, or a unique molecular identifier. In other embodiments, [B] comprises between about 5 and about 60 nucleotides and includes a barcode, a multiplex identifier, or a unique molecular identifier. In yet other embodiments, [B] comprises between about 5 and about 40 nucleotides and includes a barcode, a multiplex identifier, or a unique molecular identifier. In further embodiments, [B] comprises between about 5 and about 25 nucleotides and includes a barcode, a multiplex identifier, or a unique molecular identifier. In some embodiments, [C] comprises between about 5 and about 75 nucleotides. In other embodiments, [C] comprises between about 5 and about 50 nucleotides. In yet other embodiments, [C] comprises between about 5 and about 30 nucleotides.

An example of a linker oligonucleotide having Formula (IB) is illustrated in FIG. 1. Other non-limiting examples of linker oligonucleotides having Formula (IB) are set forth below as SEQ ID NOS: 1 - 3 (where each of the examples includes a constant region [A], a region [B], and a region [C]):

AGATCGGAAGAGCACATCCGACGGTAGTGTACAGACAGA
CGCCGAACGATTAGCTCTTCA (SEQ ID NO: 1)

AGATCGGAAGAGCACATCCGACGGTAGTGTACAGACAGA
CAGTTATGGCGGAAAGGTATGC (SEQ ID NO: 2)

AGATCGGAAGAGCACATCCGACGGTAGTGTACAGACAGA
CAAAAGAAGTCAGCTGCGTCG (SEQ ID NO: 3)

In some embodiments, the linker oligonucleotide of Formula (IA) has Formula (IC):

5' - [A] - 3' (IC)

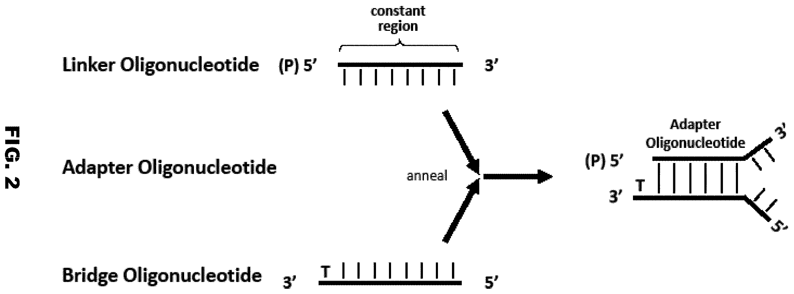

wherein the linker oligonucleotide comprises a 5' phosphate group, and wherein [A] is a "constant region" which comprises between 5 and 60 nucleotides and which is complementary to a portion of a nucleotide sequence of a bridge oligonucleotide having Formula (II); [B] is an "identifier region" which includes an identifier; and [C] is a "unique region" which comprises between 5 100 nucleotides and is complementary to a portion of a target nucleic acid molecule. In some embodiments, [A] comprises between about 10 and about 50 nucleotides. In other embodiments, [A] comprises between about 15 and about 40 nucleotides. In yet other embodiments, [A] comprises between about 15 and about 35 nucleotides. In yet other embodiments, [A] comprises between about 15 and about 30 nucleotides. In yet other embodiments, [A] comprises between about 20 and about 35 nucleotides. An example of a linker oligonucleotide having Formula (IC) is illustrated in FIG. 2. Another non-limiting example of a linker oligonucleotide having Formula (IC) are set forth below as SEQ ID NO: 4:
AGATCGGAAGAGCACATCCGACTACCTATA (SEQ ID NO: 4)

### Bridge Oligonucleotides

Another aspect of the present disclosure is a bridge oligonucleotide having Formula (II):

3' - [D] - 5' (II)

wherein the bridge oligonucleotide includes a 3' overhang; and wherein [D] comprises between 5 and 50 nucleotides and wherein at least a portion of the nucleotide sequence of [D] is complementary to the "constant region" [A] of any one of Formulas (IA), (IB), or (IC).

In some embodiments, [D] comprises between 5 and 40 nucleotides. In other embodiments, [D] comprises between 5 and 30 nucleotides. In yet other embodiments, [D] comprises between 5 and 20 nucleotides. Examples of bridge oligonucleotides having Formula (II) are illustrated in FIGS. 1 and 2. A non-limiting example of a bridge oligonucleotide having Formula (II) is set forth below:
ACTACCGTCGGATGTGCTCTTCCGATCTT (SEQ ID NO: 5)

### Probes

Another aspect of the present disclosure is a Probe comprising (i) a linker oligonucleotide having either Formula (IA) or Formula (IB), and (ii) a bridge oligonucleotide having Formula (II), wherein a portion of the linker oligonucleotide of either Formula (IA) or Formula (IB) is hybridized to at least a portion of the bridge oligonucleotide of Formula (II). In some embodiments, the probe includes a linker oligonucleotide having Formula (IB). An example of a Probe derived from a linker oligonucleotide having Formula (IB) and a bridge oligonucleotide having Formula (II) is illustrated in FIG. 1.

In some embodiments, the linker oligonucleotide of Formula (IB) includes a "constant region" [A] having between about 5 and about 60 nucleotides; and wherein x is 1. In some embodiments, the linker oligonucleotide of Formula (IB) includes a "constant region" [A] having between about 15 and about 50 nucleotides; and wherein x is 1. In some embodiments, the linker oligonucleotide of Formula (IB) includes a "constant region" [A] having between about 15 and about 35 nucleotides; and wherein x is 1. In other embodiments, the linker oligonucleotide of Formula (IB) includes a "constant region" [A] having between about 15 and about 35 nucleotides; wherein x is 1; and the identifier comprises a barcode.

In further embodiments, the linker oligonucleotide of Formula (IB) includes a "constant region" [A] having between about 5 and about 50 nucleotides; wherein x is 1; the identifier comprises barcode; and the "unique region" [C] includes between about 5 and about 100 nucleotides. In further embodiments, the linker oligonucleotide of Formula (IB) includes a "constant region" [A] having between about 5 and about 50 nucleotides; x is 1; the identifier comprises a barcode; and [C] includes a nucleotide sequence which is complementary to at least a portion of a target nucleic acid molecule (such as a target nucleic acid molecule in an obtained biological sample which includes both target nucleic acid molecules and non-target nucleic acid molecules).

In further embodiments, the linker oligonucleotide of Formula (IB) includes a "constant region" [A] having between about 15 and about 35 nucleotides; wherein x is 1; the identifier comprises barcode; and the "unique region" [C] includes between about 5 and about 100 nucleotides. In further embodiments, the linker oligonucleotide of Formula (IB) includes a "constant region" [A] having between about 15 and about 35 nucleotides; x is 1; the identifier comprises a barcode; and [C] includes a nucleotide sequence which is complementary to at least a portion of a target nucleic acid molecule (such as a target nucleic acid molecule in an obtained biological sample which includes both target nucleic acid molecules and non-target nucleic acid molecules).

In further embodiments, the linker oligonucleotide of Formula (IB) includes a "constant region" [A] having between about 25 and about 35 nucleotides; wherein x is 1; the identifier comprises barcode; and the "unique region" [C] includes between about 5 and about 50 nucleotides. In further embodiments, the linker oligonucleotide of Formula (IB) includes a "constant region" [A] having between about 25 and about 35 nucleotides; x is 1; the identifier comprises a barcode; and [C] includes a nucleotide sequence which is complementary to at least a portion of a target nucleic acid molecule. In some embodiments, [A] comprises between about 25 and about 35 nucleotides. In some embodiments, [A] comprises about 25, about 26, about 27, about 28, about 29, about 30 nucleotides, about 31 nucleotides, or about 32 nucleotides.

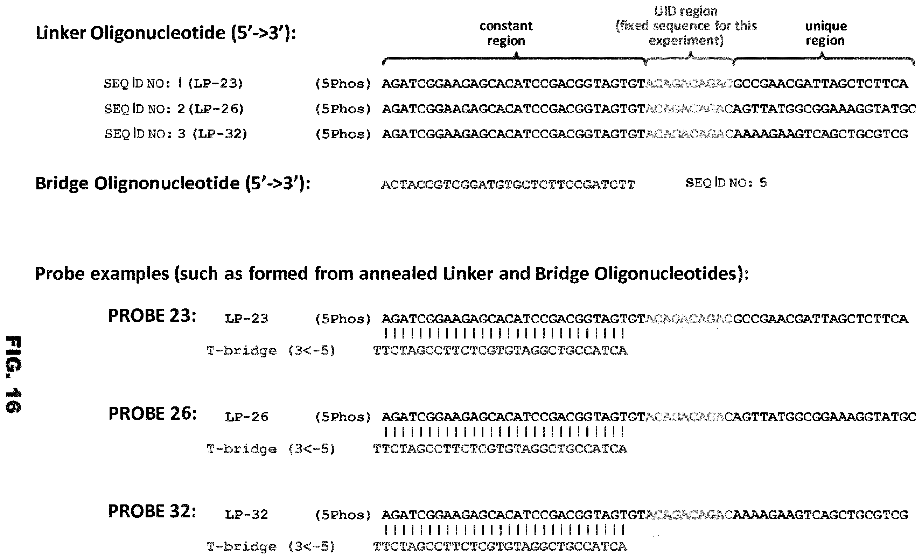

Non-limiting examples of probes formed from linker oligonucleotides and bridge oligonucleotides are set forth in FIG. 16.

### Adapter Oligonucleotides

Another aspect of the present disclosure is an adapter oligonucleotide comprising (i) a linker oligonucleotide having Formula (IC), and (ii) a bridge oligonucleotide having Formula (II), wherein at least a portion of the linker oligonucleotide of Formula (IC) is hybridized to at least a portion of the bridge oligonucleotide of Formula (II). An example of an Adapter Oligonucleotide derived from a linker oligonucleotide having Formula (IC) and a bridge oligonucleotide having Formula (II) is illustrated in FIG. 2.

In some embodiments, the linker oligonucleotide of Formula (IC) includes a "constant region" [A] having between about 5 and about 50 nucleotides, and wherein the bridge oligonucleotide of Formula (II) includes a nucleic acid sequence [D] having between about 5 and about 50 nucleotides, and wherein at least a portion of the nucleotide sequence of [D] is complementary to the "constant region" [A] of Formula (IC). In some embodiments, the linker oligonucleotide of Formula (IC) includes a "constant region" [A] having between about 15 and about 35 nucleotides, and wherein the bridge oligonucleotide of Formula (II) includes a nucleic acid sequence [D] having between about 5 and about 50 nucleotides, and wherein at least a portion of the nucleotide sequence of [D] is complementary to the "constant region" [A] of Formula (IC).

In other embodiments, the linker oligonucleotide of Formula (IC) includes a "constant region" [A] having between about 15 and about 35 nucleotides, and wherein the bridge oligonucleotide of Formula (II) includes a nucleic acid sequence [D] having between about 25 and about 30 nucleotides, and wherein at least a portion of the nucleotide sequence of [D] is complementary to the "constant region" [A] of Formula (IC). In other embodiments, the linker oligonucleotide of Formula (IC) includes a "constant region" [A] having between about 25 and about 35 nucleotides, and wherein the bridge oligonucleotide of Formula (II) includes a nucleic acid sequence [D] having between about 25 and about 30 nucleotides, and wherein at least a portion of the nucleotide sequence of [D] is complementary to the "constant region" [A] of Formula (IC).

### KITS / COMPOSITIONS

Another aspect of the present disclosure is a kit comprising: (i) one or more linker oligonucleotides having Formula (IA); and (ii) one or more bridge oligonucleotides having Formula (II). In some embodiments, the one or more linker oligonucleotides of Formula (IA) include a "constant region" having between about 5 and about 60 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IA) include a "constant region" having between about 5 and about 50 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IA) include a "constant region" having between about 15 and about 35 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IA) include a "constant region" having between about 25 and about 35 nucleotides, and the one or more bridge oligonucleotides of Formula (II) include a nucleic acid sequence [D] which is complementary to at least a portion of the "constant region" [A].

In other embodiments, the one or more linker oligonucleotides of Formula (IA) include a "constant region" having between about 15 and about 35 nucleotides and the one or more bridge oligonucleotides of Formula (II) include a nucleic acid sequence [D] which is complementary to at least a portion of the "constant region" [A]; and where in the linker oligonucleotide further includes a barcode. In yet other embodiments, the one or more linker oligonucleotides of Formula (IA) include a "constant region" having between about 25 and about 35 nucleotides and the one or more bridge oligonucleotides of Formula (II) include a nucleic acid sequence [D] which is complementary to at least a portion of the "constant region" [A]; and where in the linker oligonucleotide further includes a barcode. In some embodiments, [A] comprises about 25, about 26, about 27, about 28, about 29, about 30 nucleotides, about 31 nucleotides, or about 32 nucleotides.

Another aspect of the present disclosure is a kit comprising: (i) one or more linker oligonucleotides having Formula (IB); and (ii) one or more bridge oligonucleotides having Formula (II). In some embodiments, the one or more linker oligonucleotides of Formula (IB) include a "constant region" having between about 5 and about 60 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IB) include a "constant region" having between about 5 and about 50 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IB) include a "constant region" having between about 15 and about 35 nucleotides, and the one or more bridge oligonucleotides of Formula (II) include a nucleic acid sequence [D] which is complementary to at least a portion of the "constant region" [A]. In some embodiments, the one or more linker oligonucleotides of Formula (IB) include a "constant region" having between about 25 and about 35 nucleotides and the one or more bridge oligonucleotides of Formula (II) include a nucleic acid sequence [D] which is complementary to at least a portion of the "constant region" [A].

In other embodiments, the one or more linker oligonucleotides of Formula (IB) include a "constant region" having between about 15 and about 35 nucleotides and the one or more bridge oligonucleotides of Formula (II) include a nucleic acid sequence [D] which is complementary to at least a portion of the "constant region" [A]; and where in the linker oligonucleotide further includes a barcode. In yet other embodiments, the one or more linker oligonucleotides of Formula (IB) include a "constant region" having between about 25 and about 35 nucleotides and the one or more bridge oligonucleotides of Formula (II) include a nucleic acid sequence [D] which is complementary to at least a portion of the "constant region" [A]; and where in the linker oligonucleotide further includes a barcode.

Another aspect of the present disclosure is a kit comprising: (i) one or more linker oligonucleotides having Formula (IC); and (ii) one or more bridge oligonucleotides having Formula (II). In some embodiments, the one or more linker oligonucleotides of Formula (IC) include a "constant region" having between about 5 and about 50 nucleotides and the one or more bridge oligonucleotides of Formula (II) include a nucleic acid sequence [D] which is complementary to at least a portion of the "constant region" [A]. In some embodiments, the one or more linker oligonucleotides of Formula (IC) include a "constant region" having between about 15 and about 35 nucleotides and the one or more bridge oligonucleotides of Formula (II) include a nucleic acid sequence [D] which is complementary to at least a portion of the "constant region" [A]. In some embodiments, the one or more linker oligonucleotides of Formula (IC) include a "constant region" having between about 25 and about 35 nucleotides and the one or more bridge oligonucleotides of Formula (II) include a nucleic acid sequence [D] which is complementary to at least a portion of the "constant region" [A].

In some embodiments, the one or more linker oligonucleotides of Formula (IC) include a "constant region" having between about 15 and about 35 nucleotides and the one or more bridge oligonucleotides of Formula (II) include a nucleic acid sequence [D] which is complementary to at least a portion of the "constant region" [A]. In some embodiments, the one or more linker oligonucleotides of Formula (IC) include a "constant region" having between about 25 and about 35 nucleotides and the one or more bridge oligonucleotides of Formula (II) include a nucleic acid sequence [D] which is complementary to at least a portion of the "constant region" [A].

Another aspect of the present disclosure is a kit comprising: (i) a linker oligonucleotide having Formula (IB); (ii) a linker oligonucleotide having Formula (IIC); and (iii) two bridge oligonucleotides having Formula (II); wherein the linker oligonucleotide having Formula (IB) is capable of hybridizing with a first of the two bridge oligonucleotides having Formula (II), and wherein the linker oligonucleotide having Formula (IC) is capable of hybridizing with a second of the two bridge oligonucleotide having Formula (II). In some embodiments, the kit includes one or more different linker oligonucleotides having Formula (IB) and wherein the one or more different linker oligonucleotides having Formula (IB) each include at least a different identifier. In some embodiments, each different identifier is a barcode. In some embodiments, each of the linker oligonucleotides of Formulas (IB) and (IC) include a constant region and where the constant region of Formulas (IB) and (IC) are the same.

In some embodiments, any of the kits of the present disclosure may further include one or more primers. In some embodiments, any of the kits of the present disclosure may further include one or more forward and/or reverse inverse PCR primers. In some embodiments, the one or more forward and/or reverse inverse PCR primers are capable of binding to portions of the "constant region" [A] of a linker oligonucleotide having Formulas (IA) to (IC). In other embodiments, any of the kits of the present disclosure may further include a universal primer for rolling circle amplification. In some embodiments, the universal primer for rolling circle amplification is capable of binding to a portion of a "constant region" [A] of a linker oligonucleotide having Formulas (IA) to (IC). In yet other embodiments, any of the kits of the present disclosure may further include a target-specific primer for rolling circle amplification.

In some embodiments, any of the kits of the present disclosure may further include one or reagents for conducting a polymerase chain reaction (PCR). In some embodiments, the PCR reagents include deoxynucleoside triphosphates (dNTPs), in particular all of the four naturally-occurring deoxynucleoside triphosphates (dNTPs). In some embodiments, the PCR reagents include deoxyribonucleoside triphosphate molecules, including all of dATP, dCTP, dGTP, dTTP. In some embodiments, the PCR reagents also include compounds useful in assisting the activity of the nucleic acid polymerase. For example, in some embodiments, the PCR reagent include a divalent cation, e.g., magnesium ions. In some embodiments, the magnesium ions are provided in the form of magnesium chloride, magnesium acetate, or magnesium sulfate.

In some embodiments, the PCR reagents further include a buffer or buffer solution. Non-liming examples of buffers include citric acid, potassium dihydrogen phosphate, boric acid, diethyl barbituric acid, piperazine-N,N'-bis(2-ethanesulfonic acid), dimethylarsinic acid, 2-(N-morpholino)ethanesulfonic acid, tris(hydroxymethyl)methylamine (TRIS), 2-(N-morpholino)ethanesulfonic acid (TAPS), N,N-bis(2-hydroxyethyl)glycine(Bicine), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), and combinations thereof. In other embodiments, the buffer may be comprised of tris(hydroxymethyl)kit (TRIS), 2-(N-morpholino)ethanesulfonic acid (TAPS), N,N-bis(2-hydroxyethyl)glycine(Bicine), N -tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), or a combination thereof.

In some embodiments, any of the kits of the present disclosure may further include at least one polymerase, modified polymerase, or thermostable polymerase. As used herein, the term "polymerase" refers to an enzyme that performs template-directed synthesis of polynucleotides. A DNA polymerase can add free nucleotides only to the 3' end of the newly forming strand. This results in elongation of the newly forming strand in a 5'-3' direction. No known DNA polymerase is able to begin a new chain (de novo). DNA polymerase can add a nucleotide only on to a pre-existing 3'-OH group, and, therefore, needs a primer at which it can add the first nucleotide. Non-limiting examples of polymerases include prokaryotic DNA polymerases (e.g. Pol I, Pol II, Pol III, Pol IV and Pol V), eukaryotic DNA polymerase, archaeal DNA polymerase, telomerase, reverse transcriptase and RNA polymerase. Reverse transcriptase is an RNA-dependent DNA polymerase which synthesizes DNA from an RNA template. The reverse transcriptase family contain both DNA polymerase functionality and RNase H functionality, which degrades RNA base-paired to DNA. RNA polymerase, is an enzyme that synthesizes RNA using DNA as a template during the process of gene transcription. RNA polymerase polymerizes ribonucleotides at the 3' end of an RNA transcript.

In some embodiments, suitable polymerases may be derived from: archaea (e.g., Thermococcus litoralis (Vent, GenBank: AAA72101), Pyrococcus furiosus (Pfu, GenBank: D12983, BAA02362), Pyrococcus woesii, Pyrococcus GB-D (Deep Vent, GenBank: AAA67131), Thermococcus kodakaraensis KODI (KOD, GenBank: BD175553, BAA06142; Thermococcus sp. strain KOD (Pfx, GenBank: AAE68738)), Thermococcus gorgonarius (Tgo, Pdb: 4699806), Sulfolobus solataricus (GenBank: NC002754, P26811), Aeropyrum pernix (GenBank: BAA81109), Archaeglobus fulgidus (GenBank: 029753), Pyrobaculum aerophilum (GenBank: AAL63952), Pyrodictium occultum (GenBank: BAA07579, BAA07580), Thermococcus 9 degree Nm (GenBank: AAA88769, Q56366), Thermococcus fumicolans (GenBank: CAA93738, P74918), Thermococcus hydrothermalis (GenBank: CAC18555), Thermococcus sp. GE8 (GenBank: CAC12850), Thermococcus sp. JDF-3 (GenBank: AX135456; WO0132887), Thermococcus sp. TY (GenBank: CAA73475), Pyrococcus abyssi (GenBank: P77916), Pyrococcus glycovorans (GenBank: CAC12849), Pyrococcus horikoshii (GenBank: NP 143776), Pyrococcus sp. GE23 (GenBank: CAA90887), Pyrococcus sp. ST700 (GenBank: CAC 12847), Thermococcus pacificus (GenBank: AX411312.1), Thermococcus zilligii (GenBank: DQ3366890), Thermococcus aggregans, Thermococcus barossii, Thermococcus celer (GenBank: DD259850.1), Thermococcus profundus (GenBank: E14137), Thermococcus siculi (GenBank: DD259857.1), Thermococcus thioreducens, Thermococcus onnurineus NA1, Sulfolobus acidocaldarium, Sulfolobus tokodaii, Pyrobaculum calidifontis, Pyrobaculum islandicum (GenBank: AAF27815), Methanococcus jannaschii (GenBank: Q58295), Desulforococcus species TOK, Desulforococcus, Pyrolobus, Pyrodictium, Staphylothermus, Vulcanisaetta, Methanococcus (GenBank: P52025) and other archaeal B polymerases, such as GenBank AAC62712, P956901, BAAA07579)), thermophilic bacteria Thermus species (e.g., flavus, ruber, thermophilus, lacteus, rubens, aquaticus), Bacillus stearothermophilus, Thermotoga maritima, Methanothermus fervidus, KOD polymerase, TNA1 polymerase, Thermococcus sp. 9 degrees N-7, T4, T7, phi29, Pyrococcus furiosus, P. abyssi, T. gorgonarius, T. litoralis, T. zilligii, T. sp. GT, P. sp. GB-D, KOD, Pfu, T. gorgonarius, T. zilligii, T. litoralis and Thermococcus sp. 9N-7 polymerases.

As used herein, the term "modified DNA polymerase" refers to a DNA polymerase originated from another (i.e., parental) DNA polymerase and contains one or more amino acid alterations (e.g., amino acid substitution, deletion, or insertion) compared to the parental DNA polymerase. In some embodiments, a modified DNA polymerases of the disclosure is originated or modified from a naturally-occurring or wild-type DNA polymerase. In some embodiments, a modified DNA polymerase of the disclosure is originated or modified from a recombinant or engineered DNA polymerase including, but not limited to, chimeric DNA polymerase, fusion DNA polymerase or another modified DNA polymerase. Typically, a modified DNA polymerase has at least one changed phenotype compared to the parental polymerase. Examples of modified polymerases are described in United States Patent Application Publication No. 2016/0222363.

As used herein, the term "thermostable polymerase" refers to an enzyme that is stable to heat, is heat resistant, and retains sufficient activity to effect subsequent polynucleotide extension reactions and does not become irreversibly denatured (inactivated) when subjected to the elevated temperatures for the time necessary to effect denaturation of double-stranded nucleic acids. The heating conditions necessary for nucleic acid denaturation are well known in the art and are exemplified in, e.g., U.S. Pat. Nos. 4,683,202, 4,683,195, and 4,965,188. As used herein, a thermostable polymerase is suitable for use in a temperature cycling reaction such as the polymerase chain reaction ("PCR"), a primer extension reaction, or an end-modification (e.g., terminal transferase, degradation, or polishing) reaction. Irreversible denaturation for purposes herein refers to permanent and complete loss of enzymatic activity. For a thermostable polymerase, enzymatic activity refers to the catalysis of the combination of the nucleotides in the proper manner to form polynucleotide extension products that are complementary to a template nucleic acid strand. Thermostable DNA polymerases from thermophilic bacteria include, e.g., DNA polymerases from Thermotoga maritima, Thermus aquaticus, Thermus thermophilus, Thermus flavus, Thermus filiformis, Thermus species sps17, Thermus species Z05, Thermus caldophilus, Bacillus caldotenax, Thermotoga neopolitana, Thermosipho africanus, and other thermostable DNA polymerases disclosed above.

In some embodiments, a polymerase may be a modified naturally occurring Type A polymerase. A further embodiment of the invention generally relates to a method wherein a modified Type A polymerase, e.g., in a primer extension, end-modification (e.g., terminal transferase, degradation, or polishing), or amplification reaction, may be selected from any species of the genus Meiothermus, Thermotoga, or Thermomicrobium. Another embodiment of the invention generally pertains to a method wherein the polymerase, e.g., in a primer extension, end-modification (e.g., terminal transferase, degradation or polishing), or amplification reaction, may be isolated from any of Thermus aquaticus (Taq), Thermus thermophilus, Thermus caldophilus, or Thermus filiformis. A further embodiment of the invention generally encompasses a method wherein the modified Type A polymerase, e.g., in a primer extension, end-modification (e.g., terminal transferase, degradation, or polishing), or amplification reaction, may be isolated from Bacillus stearothermophilus, Sphaerobacter thermophilus, Dictoglomus thermophilum, or Escherichia coli. In another embodiment, the invention generally relates to a method wherein the modified Type A polymerase, e.g., in a primer extension, end-modification (e.g., terminal transferase, degradation, or polishing), or amplification reaction, may be a mutant Taq-E507K polymerase. Another embodiment of the invention generally pertains to a method wherein a thermostable polymerase may be used to effect amplification of the target nucleic acid.

In some embodiments, any of the kits of the present disclosure may further include a ligase. In some embodiments, the ligase is a DNA ligase. In some embodiments, the ligase is a thermostable single stranded RNA or DNA ligase such as the Thermophage Ligase or its derivatives such as Circligase^{™} and Circligase^{™} II (Epicentre Tech., Madison, Wise.). In other embodiments, the ligase is a T4 ligase.

In some embodiments, any one of the kits of the present disclosure may include a sequencing device, such as a sequencing device for "next generation sequencing." The term "next generation sequencing" refers to sequencing technologies having high-throughput sequencing as compared to traditional Sanger- and capillary electrophoresis-based approaches, wherein the sequencing process is performed in parallel, for example producing thousands or millions of relatively small sequence reads at a time. Some examples of next generation sequencing techniques include, but are not limited to, sequencing by synthesis, sequencing by ligation, and sequencing by hybridization. These technologies produce shorter reads (anywhere from about 25 - about 500 bp) but many hundreds of thousands or millions of reads in a relatively short time.

Examples of such sequencing devices available from Illumina (San Diego, CA) include, but are not limited to iSEQ, MiniSEQ, MiSEQ, NextSEQ, NoveSEQ. It is believed that the Illumina next-generation sequencing technology uses clonal amplification and sequencing by synthesis (SBS) chemistry to enable rapid sequencing. The process simultaneously identifies DNA bases while incorporating them into a nucleic acid chain. Each base emits a unique fluorescent signal as it is added to the growing strand, which is used to determine the order of the DNA sequence.

A non-limiting example of a sequencing device available from ThermoFisher Scientific (Waltham, MA) includes the Ion Personal Genome Machine^{™} (PGM^{™}) System. It is believed that Ion Torrent sequencing measures the direct release of H+ (protons) from the incorporation of individual bases by DNA polymerase. A non-limiting example of a sequencing device available from Pacific Biosciences (Menlo Park, CA) includes the PacBio Sequel Systems. A non-limiting example of a sequencing device available from Roche (Pleasanton, CA) is the Roche 454.

Next-generation sequencing methods may also include nanopore sequencing methods. In general, three nanopore sequencing approaches have been pursued: strand sequencing in which the bases of DNA are identified as they pass sequentially through a nanopore, exonuclease-based nanopore sequencing in which nucleotides are enzymatically cleaved one-by-one from a DNA molecule and monitored as they are captured by and pass through the nanopore, and a nanopore sequencing by synthesis (SBS) approach in which identifiable polymer tags are attached to nucleotides and registered in nanopores during enzyme-catalyzed DNA synthesis. Common to all these methods is the need for precise control of the reaction rates so that each base is determined in order.

Strand sequencing requires a method for slowing down the passage of the DNA through the nanopore and decoding a plurality of bases within the channel; ratcheting approaches, taking advantage of molecular motors, have been developed for this purpose. Exonuclease-based sequencing requires the release of each nucleotide close enough to the pore to guarantee its capture and its transit through the pore at a rate slow enough to obtain a valid ionic current signal. In addition, both of these methods rely on distinctions among the four natural bases, two relatively similar purines and two similar pyrimidines. The nanopore SBS approach utilizes synthetic polymer tags attached to the nucleotides that are designed specifically to produce unique and readily distinguishable ionic current blockade signatures for sequence determination. In some embodiments, sequencing of nucleic acids comprises via nanopore sequencing comprises: preparing nanopore sequencing complexes and determining polynucleotide sequences. Methods of preparing nanopores and nanopore sequencing are described in U.S. Patent Application Publication No. 2017/0268052, and PCT Publication Nos. WO2014/074727, WO2006/028508, WO2012/083249, and WO/2014/074727. In some embodiments, tagged nucleotides may be used in the determination of the polynucleotide sequences (see, e.g., PCT Publication No. WO/2020/131759, WO/2013/191793, and WO/2015/148402).

In some embodiments, any one of the kits of the present disclosure may include software for analyzing obtained sequencing data. Analysis of the data generated by sequencing is generally performed using software and/or statistical algorithms that perform various data conversions, e.g., conversion of signal emissions into base calls, conversion of base calls into consensus sequences for a nucleic acid template, etc. Such software, statistical algorithms, and the use of such are described in detail, in U.S. Patent Application Publication Nos. 2009/0024331 2017/0044606 and in PCT Publication No. WO/2018/034745.

### METHODS

The present disclosure also provides methods of amplifying a target nucleic acid molecule, such as a target nucleic acid molecule within an obtained sample. In some embodiments, the methods described herein facilitate the amplification of a unidirectional primer extension product without the need to incorporate a second polymerase chain reaction primer binding target into an initial single-stranded nucleic acid molecule primer extension product. The methods described herein rely on probes (such as any of the probes described herein which are comprised of a linker oligonucleotide and a bridge oligonucleotide) which include a constant region to which one or more primers may hybridize.

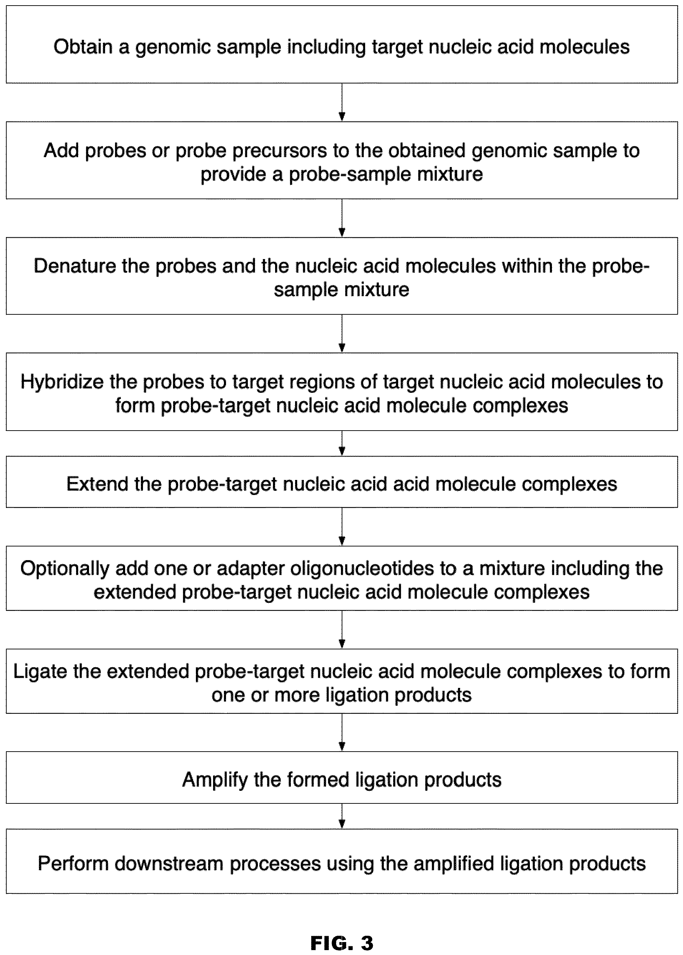

FIG. 3 illustrates a workflow for preparing an obtained sample for amplification, amplifying target nucleic acids within the obtained sample; and performing one or more downstream processes using the amplification products. Each of the steps illustrated in FIG. 3 are described in detail herein.

### Sample Preparation

In some embodiments, the methods described herein comprise obtaining a genomic sample (step 310). In some embodiments, the obtained genomic sample is derived from a biological sample, such as a biological sample derived from a mammalian subject. In some embodiments, the obtained genomic sample is derived from a tissue sample, such as a tissue sample derived from a mammalian subject. In some embodiments, the obtained genomic sample is derived from a tissue sample of a human subject. In some embodiments, the obtained genomic sample is derived from a fragment of a solid tissue or a solid tumor derived from a mammalian subject or a human patient, e.g., by biopsy.

In other embodiments, the obtained nucleic acid library is derived from a cytology sample, such as a cytology sample derived from a mammalian subject. In other embodiments, the obtained nucleic acid library is derived from a cytology sample, such as a cytology sample derived from a human subject.

In other embodiments, the obtained genomic sample may also comprise body fluids (e.g., urine, sputum, serum, plasma or lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, and/or fecal samples). In other embodiments, the obtained genomic sample may comprise whole blood or blood fractions where tumor cells may be present.

In some embodiments, the obtained genomic sample includes DNA molecules, RNA molecules, cDNA molecules, mRNA molecules, rRNA molecules, mtDNA, siRNA molecules, or any combination thereof. In other embodiments, the obtained genomic sample may comprise cell-free material such as cell-free DNA or RNA including cell-free tumor DNA or tumor RNA. In some embodiments, the sample is a cell-free sample, e.g., cell-free blood-derived sample where cell-free tumor DNA or tumor RNA are present. In other embodiments, the sample is a cultured sample, e.g., a culture or culture supernatant containing or suspected to contain an infectious agent or nucleic acids derived from the infectious agent. In some embodiments, the infectious agent is a bacterium, a protozoan, a virus, or a mycoplasma.

In some embodiments, the obtained genomic sample includes a plurality of target nucleic acid molecules and/or a plurality of non-target nucleic acid molecules. In some embodiments, the obtained genomic sample comprises a plurality of single stranded polynucleotides. In some embodiments, the single stranded polynucleotides include one or more translocations, inversions, or deletions. In some embodiments, the target nucleic acid is a gene or a gene fragment. In other embodiments, the target nucleic acid includes a genetic variant, e.g., a polymorphism, including a single nucleotide polymorphism or variant (SNP or SNV), or a genetic rearrangement resulting e.g., in a gene fusion. In some embodiments, the target nucleic acid comprises a biomarker. In other embodiments, the target nucleic acid is characteristic of a particular organism, e.g., aids in identification of the pathogenic organism or a characteristic of the pathogenic organism, e.g., drug sensitivity or drug resistance. In yet other embodiments, the target nucleic acid is characteristic of a human subject, e.g., the HLA or KIR sequence defining the subject's unique HLA or KIR genotype. In yet other embodiments, all the sequences in the obtained genomic are target nucleic acids e.g., in shotgun genomic sequencing.

Methods for isolating nucleic acids from biological samples and/or purifying the samples are known, e.g., as described in Sambrook, and several kits are commercially available (e.g., High Pure RNA Isolation Kit, High Pure Viral Nucleic Acid Kit, and MagNA Pure LC Total Nucleic Acid Isolation Kit, DNA Isolation Kit for Cells and Tissues, DNA Isolation Kit for Mammalian Blood, High Pure FFPET DNA Isolation Kit, available from Roche). In the context of the presently disclosed methods, genomic DNA can be collected, purified, and/or isolated.

It will be appreciated that nucleic acid molecules may be isolated from biological samples using any of a variety of procedures known in the art, for example, MagMAX^{™} DNA Multi-Sample Ultra Kit (Applied Biosystems, Thermo Fisher Scientific), the MagMAX^{™} Express-96 Magnetic Particle Processor and the KingFisher^{™} Flex Magnetic Particle Processor (Thermo Fisher Scientific), a RecoverAll^{™} Total Nucleic Acid Isolation Kit for FFPE and PureLink^{™} FFPE RNA Isolation Kit (Ambion^{™}, Thermo Fisher Scientific), the ABI Prism^{™} 6100 Nucleic Acid PrepStation and the ABI Prism^{™} 6700 Automated Nucleic Acid Workstation (Applied Biosystems, Thermo Fisher Scientific), and the like.

In some embodiments, obtained genomic sample is further processed prior to any further downstream operations. In some embodiments, the nucleic acids within the obtained genomic sample may be prepared for downstream processing by fragmenting, cutting, or shearing the nucleic acids. In some embodiments, the fragmenting, cutting, and/or shearing may be accomplishing using such procedures as mechanical force, sonication, restriction endonuclease cleavage, or any method known in the art. In other embodiments, no fragmentation is necessary (some genomic samples may already consist of appropriately sized fragments and will not require additional fragmentation).

In some embodiments, the nucleic acid molecules within the obtained genomic sample are sheared into fragments to provide a population of nucleic acid fragments. In some embodiments, shearing of the obtained genomic sample is effectuated using mechanical (e.g. nebulization or sonication) and/or enzymatic fragmentation (e.g. restriction endonucleases). In some embodiments, the generated nucleic acid fragments are randomly sized. In some embodiments, the generated nucleic acid fragments have a length which are less than about 1000 base pairs. In other embodiments, the generated nucleic acid fragments comprises sequence fragments having a sequence size ranging from between about 100 to about 1000 base pairs in length. In yet other embodiments, the generated nucleic acid fragments comprises sequence fragments having a sequence size ranging from between about 500 to about 750 base pairs in length. In some embodiments, adapters, such as those including a specific barcode sequence, are then added via a ligation reaction to the population of nucleic acids.

### Formation of Probe-Target Nucleic Acid Molecule Complexes

After the genomic sample is obtained (step 310) and optionally further prepared (step 320), a probe-sample mixture is formed (see, for example, FIG. 3, step 320) by adding one or more probes or one or more probe pre-cursors to the obtained genomic sample. In some embodiments, one or more probes (such as any of the probe oligonucleotides described herein, including any of the probes prepared by hybridizing a linker oligonucleotide having Formula (IA) or Formula (IB) with a bridge oligonucleotide of Formula (II)) are added to the prepared genomic sample to form a probe-sample mixture.

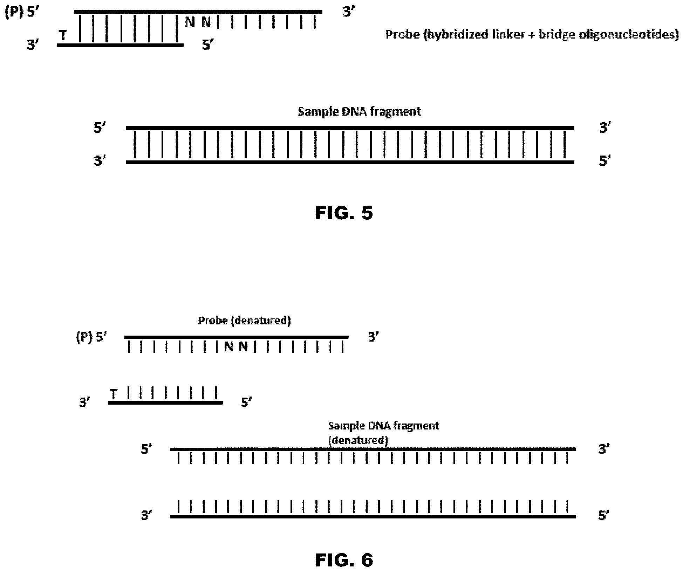

FIG. 5 illustrates a probe-sample mixture including (i) a probe derived from linker and bridge oligonucleotides; and (ii) a target nucleic acid molecule, e.g. a target nucleic acid molecule that has been fragmented. In some embodiments, each probe of the one or more probes added to the genomic sample includes a nucleotide sequence (a "unique region") which is complementary to a portion of a nucleotide sequence of a target nucleic acid sequence within the prepared genomic sample. In addition, each of the probes includes a constant region to which primers, added in a downstream processing step, may hybridize. In some embodiments, each probe of the one or more probes added to the genomic sample includes one or more of a barcode, a multiplex identifier, or a unique molecular identifier.

In other embodiments, probe precursors are added to the prepared genomic sample and the probe is formed in situ from the probe precursor components. In some embodiments, the probe precursor components include (i) one or more linker oligonucleotides having Formula (IA) or Formula (IB); and (ii) one or more bridge oligonucleotides having Formula (II). In some embodiments, each of the one or more linker oligonucleotides includes a nucleotide sequence (a "unique region") which is complementary to a portion of a nucleotide sequence of a target nucleic acid sequence within the prepared genomic sample; and a constant region to which primers, added in a downstream processing step, may hybridize. In some embodiments, each of the one or more linker oligonucleotides includes one or more of a barcode, a multiplex identifier, or a unique molecular identifier.

After the probe-sample mixture is formed (step 320), the probes and/or the obtained genomic material are denatured (step 330). In some embodiments, denaturing comprises heating the probe-sample mixture to a temperature ranging from between about 80°C to about 110°C. In some embodiments, denaturation of the probes and/or the genomic material comprises heating the probe-sample mixture to a temperature of about 95°C. FIG. 6 illustrates a probe-sample mixture including (i) a probe, derived from linker and bridge oligonucleotides, which has been denatured; and (ii) a double stranded target nucleic acid molecule which has been denatured.

In some embodiments, the denatured probe is then annealed to the denatured target nucleic acid molecule to form a probe-target nucleic acid molecule complex. Since the formed probe-target nucleic acid molecule complex is formed from a probe including a constant region, the formed probe-target nucleic acid molecule complex includes a constant region (to which primers, added in a downstream processing step, may hybridize).

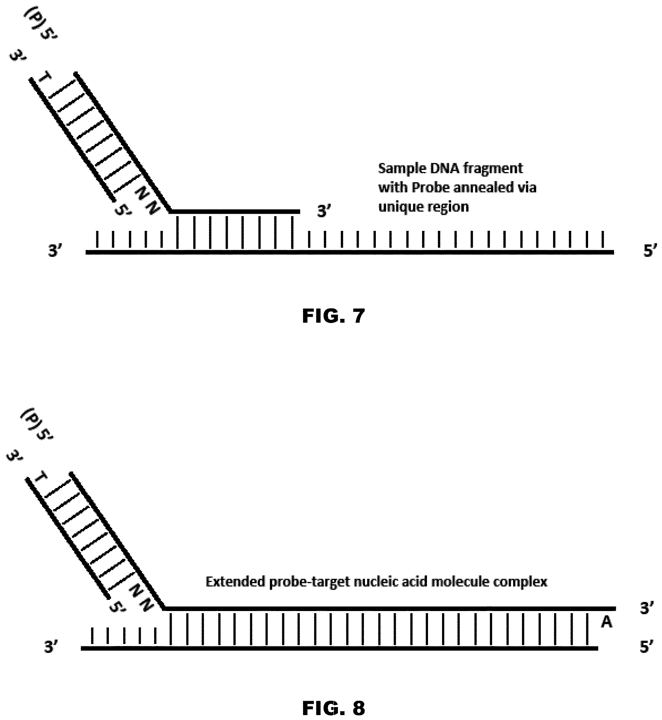

To form the probe-target nucleic acid complex, in some embodiments the temperature of a probe-sample mixture is cooled to a temperature ranging from between about 35°C to about 60°C to facilitate annealing, i.e. the lowering of the temperature of the probe-sample mixture allows the probes to assemble and then anneal to complementary regions of target nucleic acid sequences. In this manner, the probes become hybridized to target nucleic acid molecules to form one or more probe-target nucleic acid molecule complexes (step 340). FIG. 7 illustrates a probe annealed to a target nucleic acid molecule, i.e. a probe-target nucleic acid molecule complex. While not shown in FIG. 7, a unique region of the probe hybridizes to a complementary portion of the target nucleic acid.

### Preparation of Extended Probe-Target Nucleic Acid Molecule Complexes for Ligation

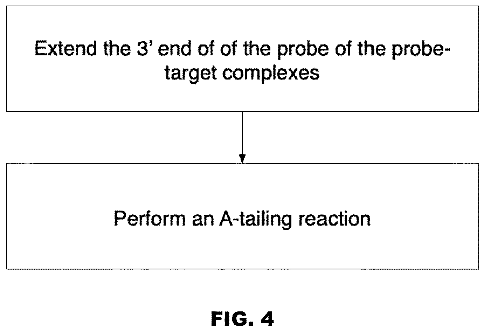

Subsequent to the formation of the probe-target nucleic acid molecule complexes (step 340), the probe-target nucleic acid molecule complexes are prepared for ligation (step 350). With reference to FIG. 4, in some embodiments, the probe-target nucleic acid molecule complexes are prepared for ligation by (i) extending the 3' end of the probe of the probe-target nucleic acid molecule complex (step 351); and (ii) performing a "tailing" reaction (step 352). The probe-target nucleic acid molecule complexes prepared for ligation are referred to herein as "extended probe-target nucleic acid molecule complexes." FIG. 8 illustrates an extended probe-target nucleic acid molecule complex, such as one including an A-tail.

In some embodiments, the 3' end of the probe of the probe-target nucleic acid molecule complex is extended (step 351) through the use of a first polymerase (including any of the polymerases described herein). Depending on the type of nucleic acid molecule being analyzed, the polymerase may be a DNA-dependent DNA polymerase ("DNA polymerase") or an RNA-dependent DNA polymerase ("reverse transcriptase"). Suitable polymerases are selected from a Taq or Taq-derived polymerase (e.g., KAPA 2G polymerase from KAPA BIOSYSTEMS); or a B-family DNA polymerase (e.g., KAPA HIFI polymerase from KAPA BIOSYSTEMS). In some embodiments, extension of the 3' end of the probe-target nucleic acid molecule is accomplished with nucleotides in the presence of a DNA polymerase and at a temperature ranging from about 60°C to about 75°C. Further methods of extending a nucleotide sequence of a probe-target nucleic acid molecule complex are described in PCT Publication No. WO/2019/121842.

In some embodiments, a "tailing" reaction is performed using a polymerase (step 352). In some embodiments, A-tailing is done as a separate step. In other embodiments, A-tailing is performed during the extension reaction. Tailing is an enzymatic method for adding a non-templated nucleotide to the 3' end of a blunt, double-stranded DNA molecule. In some embodiments, the polymerase utilized for the "tailing" reaction is the same as the polymerase used for the extension reaction. In other embodiments, the polymerase used for the "tailing" reaction is different than the polymerase used for the extension reaction. In some embodiments, a Taq polymerase is utilized for A-tailing.

Since the formed extended probe-target nucleic acid molecule complex is derived from a probe-target nucleic acid molecule complex which comprises a probe including a constant region, the extended probe-target nucleic acid molecule complex includes a constant region (to which primers, added in a downstream processing step, may hybridize).

### Ligation / Ligation Product Generation

Following the preparation of the extended probe-target nucleic acid molecule complexes (step 350), the extended probe-target nucleic acid molecule complexes are ligated to form one or more ligation products (step 370) (e.g. intramolecular circular ligation products, intermolecular circular ligation products, intermolecular linear ligation products, or any mixture thereof). The term "ligation" refers to a condensation reaction joining two nucleic acid strands wherein a 5'-phosphate group of one molecule reacts with the 3'-hydroxyl group of another molecule. Ligation is an enzymatic reaction catalyzed by a ligase or a topoisomerase. In some embodiments, the ligation step utilizes a ligase capable of catalyzing a reaction between a 5'-phosphate and a 3'-OH group of a nucleic acid. In some embodiments, the ligase is a DNA or RNA ligase capable of template-independent ligation, such as a viral ligase described (see, for example, PCT Publication No. WO2010094040). In some embodiments, the ligase is a thermostable single stranded RNA or DNA ligase such as the Thermophage Ligase or its derivatives such as Circligase^{™} and Circligase^{™} II (Epicentre Tech., Madison, Wise.). In some embodiments, the ligase is a T4 ligase.

In some embodiments, the formed extended probe-target nucleic acid molecule complexes (see step 350) are ligated (step 370) by contacting the mixture including the probe-target nucleic acid molecule complexes with a ligase (e.g. T4 ligase or any other ligase described herein), including any of the ligase molecules described herein. In other embodiments, the extended probe-target nucleic acid molecule complexes are ligated by contacting the mixture including the probe-target nucleic acid molecule complexes with a ligase (e.g. T4 ligase) and with one or more adapter oligonucleotides (see steps 360 and 370), including any of the adapter oligonucleotides described herein. In some embodiments, the ligation reactions are carried out at a temperature ranging from between about 10°C to about 30°C for a time period ranging from between about 10 minutes to about 12 hours. In some embodiments, the ligation reactions are carried out at a temperature ranging from between about 10°C to about 30°C for a time period ranging from between about 10 minutes to about 6 hours. In some embodiments, the ligation reactions are carried out at a temperature ranging from between about 10°C to about 30°C for a time period ranging from between about 10 minutes to about 4 hours. In some embodiments, the ligation reactions are carried out at a temperature ranging from between about 10°C to about 30°C for a time period ranging from between about 10 minutes to about 2 hours. In some embodiments, ligation is performed at about 16°C for between about 1 hour to about 12 hours. In other embodiments, ligation is performed at about 20 °C for about 2 hours. In yet other embodiments, ligation is performed at about 25 °C for about 15 minutes.

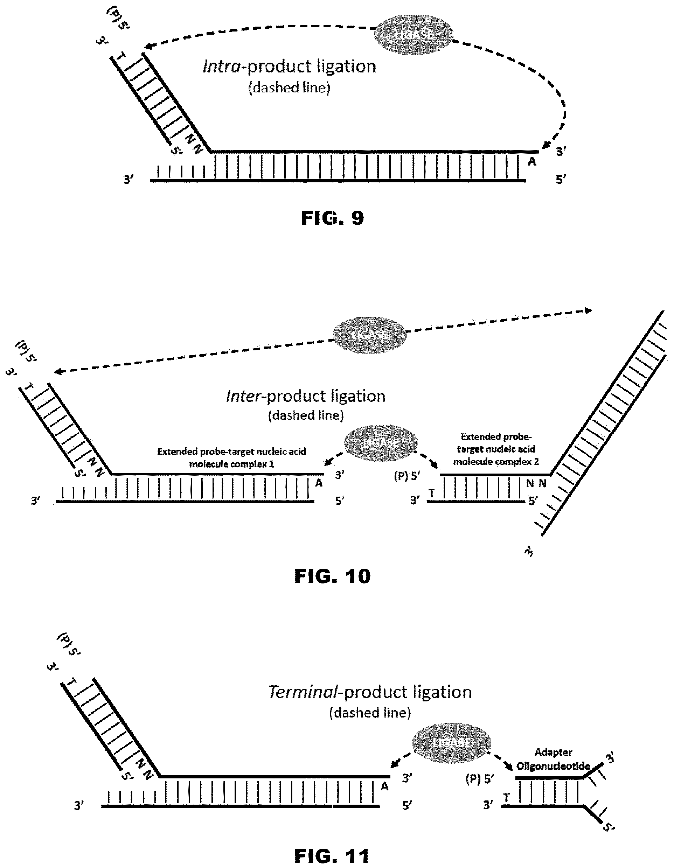

In some embodiments, an extended probe-target nucleic acid molecule complex is intramolecularly ligated such that an intramolecular circular ligation product is formed (see, for example, FIG. 9). In these embodiments, the intramolecular circular ligation product comprises a single extended probe-target nucleic acid molecule complex. In some embodiments, the intramolecular circular ligation product includes a nick within the probe portion of the extended probe-target nucleic acid molecule complexes, such as a nick in a location proximal to a unique identifier, if present. In some embodiments, the intramolecular circular ligation product includes a single constant region, i.e. a constant region included within the probe (or probe portion) of the extended probe-target nucleic acid molecule complex from which the intramolecular circular ligation product is derived.

In some embodiments, the extended probe-target nucleic acid molecule complexes are intermolecularly ligated. In some embodiments, the intermolecular ligation between extended probe-target nucleic acid molecule complexes creates concatemers of various numbers of units, e.g. two or more concatemeric units, three or more concatemeric units, four or more concatemeric units, five or more concatemeric units, etc. Specifically, the ligation reaction may join both strands of a double-stranded extended probe-target nucleic acid molecule complex to both strands of another double-stranded extended probe-target nucleic acid molecule complex thus joining the two double-stranded extended probe-target nucleic acid molecule complexes. In some embodiments, the intermolecular ligation forms intermolecular circulation products (see, for example, FIG. 10) and/or intermolecular linear ligation products (see, for example, FIG. 11), depending on whether one or more adapter oligonucleotides are present during the ligation reaction. In some embodiments, the intermolecular circular ligation products include one or more constant regions, i.e. constant regions included within the probes (or probe portions) of the extended probe-target nucleic acid molecule complexes from which the intermolecular circular ligation product is derived. Likewise, in some embodiments, the intermolecular linear ligation products include one or more constant regions, i.e. constant regions included within the probes (or probe portions) of the extended probe-target nucleic acid molecule complexes and/or the adapter oligonucleotide ligated to a terminal extended probe-target nucleic acid molecule complex.

In some embodiments, intermolecular ligation may terminate through a circularizing ligation event between two terminal extended probe-target nucleic acid molecule complexes in the chain of concatemers. In some embodiments, an intermolecular circular ligation product is formed from two or more extended probe-target nucleic acid molecule complexes. In other embodiments, an intermolecular circular ligation product is formed from two extended probe-target nucleic acid molecule complexes. In other embodiments, an intermolecular circular ligation product is formed from three extended probe-target nucleic acid molecule complexes. In other embodiments, an intermolecular circular ligation product is formed from four extended probe-target nucleic acid molecule complexes. In other embodiments, an intermolecular circular ligation product is formed from five extended probe-target nucleic acid molecule complexes.

In some embodiments, one or more extended probe-target nucleic acid molecule complexes are intermolecularly ligated in the presence of one or more adapter molecules such that an intermolecular linear ligation product is formed. In some embodiments, in order to generate one or more intermolecular linear ligation products one or more adapter oligonucleotides (including any of those adapter oligonucleotides described herein) are added to the mixture including the extended probe-target nucleic acid molecule complexes and the ligase. Given that both the extended probe-target nucleic acid molecule complexes and the adapter oligonucleotides are double stranded, the ligation between an adapter oligonucleotide and a terminal extended probe-target nucleic acid molecule complex terminates concatamerization and prevents circularization.

In some embodiments, an intermolecular linear ligation product includes two or more extended probe-target nucleic acid molecule complexes and an adapter oligonucleotide. In other embodiments, an intermolecular linear ligation product includes two extended probe-target nucleic acid molecule complexes and an adapter oligonucleotide. In yet other embodiments, an intermolecular linear ligation product includes three extended probe-target nucleic acid molecule complexes and an adapter oligonucleotide. In further embodiments, an intermolecular linear ligation product includes four extended probe-target nucleic acid molecule complexes and an adapter oligonucleotide. In even further embodiments, an intermolecular linear ligation product includes five extended probe-target nucleic acid molecule complexes and an adapter oligonucleotide. In other embodiments, the intermolecular linear ligation product includes only a single extended probe-target nucleic acid molecule complex "capped" at a terminal end with an adapter oligonucleotide.

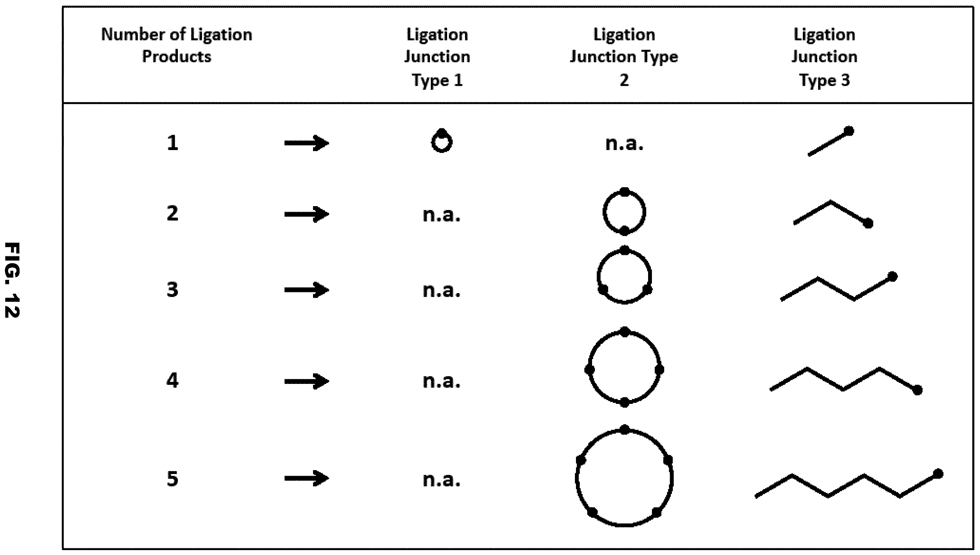

The different ligation products that may be formed are summarized in FIG. 12. As noted above, while different ligation products are formed during the step of ligation, each ligation product includes at least one constant region, depending on the number of extended probe-target nucleic acid molecule complexes within the ligation product and/or the presence of an adapter oligonucleotide. In some embodiments, the one or more constant regions present in the ligation products are targeted with one or more primers during amplification, as described herein (again, the constant regions are provided by the probe portions included within the ligation products).

### Amplification

Following the generation of the ligation products (step 370), the generated ligation products are amplified (step 380). One method of amplifying a target sequence is with a polymerase mediated technique called polymerase chain reaction (PCR). In general, PCR is a method for increasing the concentration of a segment of a target sequence in a mixture of genomic DNA without cloning or purification. Polymerase chain reaction ("PCR") is described, for example, in U.S. Pat. No. 4,683,202; U.S. Pat. No. 4,683,195; U.S. Pat. No. 4,000,159; U.S. Pat. No. 4,965,188; U.S. Pat. No. 5,176,995). Examples of PCR techniques that can be used include, but are not limited to, quantitative PCR, quantitative fluorescent PCR (QF-PCR), multiplex fluorescent PCR (MF-PCR), real time PCR (RT-PCR), single cell PCR, restriction fragment length polymorphism PCR (PCR-RFLP), PCR-RFLP/RT-PCR-RFLP, hot start PCR, nested PCR, in situ polony PCR, in situ rolling circle amplification (RCA), bridge PCR, picotiter PCR, digital PCR, droplet digital PCR, and emulsion PCR. Droplet and digital droplet PCR systems are further described in U.S. Patent No, 9,822,393 and 10,676,778. In some embodiments, the generated ligation products are amplified using one or both of inverse PCR and rolling circle amplification.

### Inverse PCR

As noted above, the generated ligation products (e.g. intramolecular circular -ligation products, intermolecular circular ligation products, intermolecular linear ligation products, or any mixture thereof) may be amplified using inverse PCR. Traditional PCR amplifications utilize oligonucleotide primers that hybridize to opposite strands. The primers are oriented such that extension proceeds inwards across the region between the two primers. Since the product of DNA synthesis of one primer serves as the template for the other primer, the PCR procedure of repeated cycles of DNA denaturation, annealing of primers, and extension by DNA polymerase results in an exponential increase in the number of copies of the region bounded by the primer. While traditional polymerase chain reaction is used to amplify a segment of a nucleic acid molecules that lies between two inward-pointing primers, inverse PCR is used to amplify DNA sequences that flank one end of a known DNA sequence and for which no primers are available. As such, inverse PCR is a variant of the polymerase chain reaction that is used to amplify DNA with only one known sequence.

In some embodiments, forward and reverse inverse PCR primers are introduced to an amplification mixture including the ligation products, e.g. intramolecular circular ligation products, intermolecular circular ligation products, and/or intermolecular linear ligation products. In some embodiments, the amplification reaction mixture comprises the forward and reverse inverse PCR primers and a master mix. In some embodiments, the amplification reaction mixture comprises the forward and reverse inverse PCR primers, a polymerase, and a mixture of four different deoxyribonucleotide triphosphate (dNTPs). In some embodiments, the amplification mixture further comprises magnesium chloride (MgCl₂) and a reaction buffer.

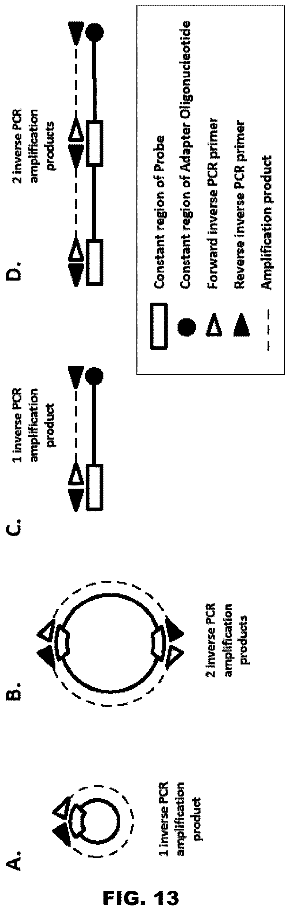

In some embodiments, the forward and reverse inverse PCR primers each have a nucleotide sequence that is at least partially complementary to a portion of a constant region included within the ligation products formed in step 370 and included within the amplification mixture. Said another way, the forward and reverse inverse PCR primers each have a nucleotide sequence that is at least partially complementary to a portion of (i) a constant region of a probe (or probe portion) of each extended probe-target nucleic acid molecule complex included within each ligation product; and/or (ii) a constant region of an adapter oligonucleotide ligated to a terminal extended probe-target nucleic acid molecule complex. In some embodiments, a forward inverse PCR primer may hybridize to a first portion of a constant region of the probe portion of each extended probe-target nucleic acid molecule complex included within each ligation product (see, e.g., FIGS. 13A -13D). In some embodiments, a reverse inverse PCR primer may hybridize to a second portion of a constant region of the probe portion of each extended probe-target nucleic acid molecule complex included within each ligation product (see, e.g., FIGS. 13A - 13D). In other embodiments, a reverse inverse PCR primer may hybridize to a portion of a constant region of an adapter oligonucleotide ligated to an extended probe-target nucleic acid molecule complex within an intermolecular linear ligation product (see, e.g., FIGS. 13C - 13D).

For example, a constant region may have a nucleic acid sequence having SEQ ID NO: 6, where SEQ ID NO: 6 comprises a first portion and a second portion. In some embodiments, a forward primer hybridizes to the first portion of the constant region; and a reverse primer having hybridizes to the second portion of the constant region.
AGATCGGAAGAGCACATCCGACGGTAGTGT (SEQ ID NO: 6)

Examples of suitable primers are set forth below:

CAAGCAGAAGACGGCATACGAGATTCGCCTTAGTGACTG
GAGTTCAGACGTGTGCTCTTCCGATCT (SEQ ID NO: 7)

AATGATACGGCGACCACCGAGATCTACACTAGATCGCTCT
TTCCCTACACGATCCGACGGTAGTGT (SEQ ID NO: 8)

In some embodiments, each constant region included within each ligation product is the same. In these embodiments, each of the forward and reverse inverse PCR primers introduced to the amplification mixture are the same.

In those embodiments where a ligation product is derived from only a single extended probe-target nucleic acid molecule complex, inverse PCR will produce only one amplification product. For instance, FIG. 13A illustrates the binding of forward and reverse inverse PCR primers to an intramolecular circular ligation product. Here, a forward primer and a reverse primer are shown at least partially hybridized to complementary portions within the intramolecular circular ligation product. Said another way, a forward primer and a reverse primer are shown in FIG. 13A as at least partially hybridized to complementary portions of a constant region of a probe of an extended probe-target nucleic acid molecule complex included within the intramolecular circular ligation product. In this particular example, inverse PCR will produce one amplification product.

By way of another example, FIG. 13C illustrates the hybridization of forward and reverse inverse PCR primers to an intermolecular linear ligation product including only a single probe-target nucleic acid molecule complex. Specifically, FIG. 13C illustrates forward and reverse inverse PCR primers hybridized to complementary portions of one or more constant regions included within a ligation product. For instance, a forward inverse PCR primer is shown at least partially hybridized to a portion of a constant region of a probe of an extended probe-target nucleic acid molecule complex; and a reverse inverse PCR primer is shown at least partially hybridized to a portion of a constant region of an adapter oligonucleotide ligated to the extended probe-target nucleic acid molecule complex. For those intermolecular linear ligation products derived from only a single extended probe-target nucleic acid molecule complex, inverse PCR will produce only one amplification product.

In some embodiments, and depending on the number of extended probe-target nucleic acid molecule complexes that constitute each intermolecular ligation product (e.g. intermolecular circular ligation products, intermolecular linear ligation products), multiple sets of forward and reverse inverse PCR primers may become hybridized to each ligation product following their introduction to the amplification mixture including the one or more ligation products (see FIGS. 13B and 13D). In some embodiments, two or more sets of forward and reverse inverse PCR primers at least partially hybridize to portions of constant regions of each intermolecular ligation product. In some embodiments, three or more sets of forward and reverse inverse PCR primers at least partially hybridize to portions of constant regions of each intermolecular ligation product. In some embodiments, four or more sets of forward and reverse inverse PCR primers at least partially hybridize to portions of constant regions of each intermolecular ligation product. In some embodiments, five or more sets of forward and reverse inverse PCR primers at least partially hybridize to portions of constant regions of each intermolecular ligation product. In some embodiments, six or more sets of forward and reverse inverse PCR primers at least partially hybridize to portions of constant regions of each intermolecular ligation product. In some embodiments, seven or more sets of forward and reverse inverse PCR primers at least partially hybridize to portions of constant regions of each intermolecular ligation product.

With reference to FIG. 13B, two sets of forward and reverse inverse PCR primers are shown at least partially hybridized to complementary portions of constant regions included within an intermolecular circular ligation product. In this particular example, the intermolecular circular ligation product is derived from two extended probe-target nucleic acid molecule complexes, and thus is derived from two probes each including a constant region. As such, forward and reverse inverse PCR primers may at least partially hybridize to complementary portions of constant regions of each probe of each extended probe-target nucleic acid molecule complex that constitutes the intermolecular circular ligation product. In this particular example, inverse PCR will produce two amplification products.

While not depicted, three sets of forward and reverse inverse PCR primers may at least partially hybridize to complementary portions of constant regions of an intermolecular circular ligation product derived from three extended probe-target nucleic acid molecule complexes. Likewise, four sets of forward and reverse inverse PCR primer may at least partially hybridize to complementary portions of constant regions of an intermolecular circular ligation product derived from four extended probe-target nucleic acid molecule complexes. Similarly, five sets of forward and reverse inverse PCR primer may at least partially hybridize to complementary portions of constant regions of an intermolecular circular ligation product derived from five extended probe-target nucleic acid molecule complexes.

With reference to FIG. 13D, two sets of forward and reverse inverse PCR primers are shown at least partially hybridized to complementary portions of constant regions included within an intermolecular linear ligation product. In this particular example, the intermolecular linear ligation product is derived from two extended probe-target nucleic acid molecule complexes and an adapter oligonucleotide. In this example, forward and reverse inverse PCR primers may at least partially hybridize to complementary portions of constant regions of each probe of each extended probe-target nucleic acid molecule complex that constitutes the intermolecular linear ligation product and/or at least partially hybridize to a complementary portion of a constant region of an adapter oligonucleotide ligated to a terminal extended probe-target nucleic acid molecule complex. In this particular example, inverse PCR will produce two amplification products. The skilled artisan will appreciate that one amplification product will be produced by inverse PCR for each extended probe-target nucleic acid molecule complex included within the intermolecular linear ligation product. Thus, the skilled artisan will appreciate that inverse PCR will produce three or more amplification products for intermolecular linear ligation products that include three or more extended probe-target nucleic acid molecule complexes.

In some embodiments, the PCR reaction mixture (ligation products, polymerase, etc.) is temperature cycled, typically between about 20 to about 40 times. In some embodiments, denaturation of the nucleic acid template sequence is achieved at about 95°C. In some embodiments, the temperature is cooled to about 37°C to about 60°C to anneal the forward and reverse inverse PCR primers to the constant regions of the ligation products. In some embodiments, extension of the forward and reverse inverse PCR primers with nucleotides by the DNA polymerase is achieved at temperatures ranging from about 60°C to about 72°C. In some embodiments, conventional cycling conditions are about 95°C for about 5 minutes initially to denature all template nucleic acid, followed by about 2 to about 40 repeats of about 95°C for about 30 seconds, about 60°C for about 30 seconds, and about 72°C for about 1 minute.

In some embodiments, the time spent at each temperature can be optimized for specific assays. For instance, the amplification of very short target sequences may require much shorter incubations at each temperature than that of very large target sequences. In some embodiments, each round of temperature cycling results in two times more target sequence than the prior round. In some embodiments, this leads to the exponential amplification of the original template, often resulting in millions or billions of copies of the original nucleic acid target.

In some embodiments, the steps of denaturation, primer annealing and polymerase extension, i.e., denaturation, annealing and extension constitute one "cycle;" there can be numerous "cycles") can be repeated a predetermined number of times (to obtain a high concentration of an amplified segment (the amplicon) of the desired target sequence. For instance, the steps of denaturation, primer annealing and polymerase extension may each be repeated 2 or more times, 4 or more times, 8 or more times, 16 or more times, 32 or more times, 64 or more times, 128 or more times, 256 or more times, etc.

### Rolling Circle Amplification

Rolling circle replication (RCA) is a process of unidirectional nucleic acid replication that can rapidly synthesize multiple copies of circular molecules of DNA or RNA. RCA is an isothermal enzymatic process that uses DNA or RNA polymerases (e.g., Φ29 DNA polymerase) to produce single stranded DNA (ssDNA) or RNA molecules which are a connection in series of complementary units of a template. An RCA reaction contains four parts: (1) a DNA polymerase and homologous buffer; (2) a relatively short DNA or RNA primer; (3) a circle template; and (4) deoxynucleotide triphosphates (dNTPs). In RCA, nucleotides (nt) are added continuously to a primer annealed to a circular template by polymerase, which produces a long ssDNA with hundreds to thousands of repeat units.

In some embodiments, a universal primer for RCA is introduced to an amplification mixture including the ligation products (e.g. intramolecular circular ligation products, intermolecular circular ligation products, intermolecular linear ligation products, or any mixture thereof). In some embodiments, a one or more target specific RCA primers are introduced to an amplification mixture including the ligation products. In some embodiments, the amplification reaction mixture comprises the universal primer and/or target specific RCA primers and a master mix. In some embodiments, the amplification reaction mixture comprises the universal primer and/or target specific RCA primers, a polymerase, and a mixture of four different deoxyribonucleotide triphosphate (dNTPs). In some embodiments, the amplification mixture further comprises magnesium chloride (MgCl₂) and a reaction buffer.

In some embodiments, the universal primer has a nucleotide sequence that is at least partially complementary to a portion of a constant region included within the formed ligation products. In some embodiments, the universal primer has a nucleotide sequence that is at least partially complementary to a portion of (i) a constant region of a probe of each extended probe-target nucleic acid molecule complex included within each ligation product; and/or (ii) a constant region of an adapter oligonucleotide ligated to an extended probe-target nucleic acid molecule complex within intermolecular linear ligation products.

For example, a constant region may have a nucleic acid sequence having SEQ ID NO: 6. In some embodiments, universal primer has a nucleic acid sequence having SEQ ID NO: 9 (AGATCGCTCTTTCCCTACA) which is at least partially complementary to a portion of the constant region.

In some embodiments, each constant region included within each ligation product is the same. In these embodiments, each of the forward and reverse inverse PCR primers introduced to the amplification mixture are the same.

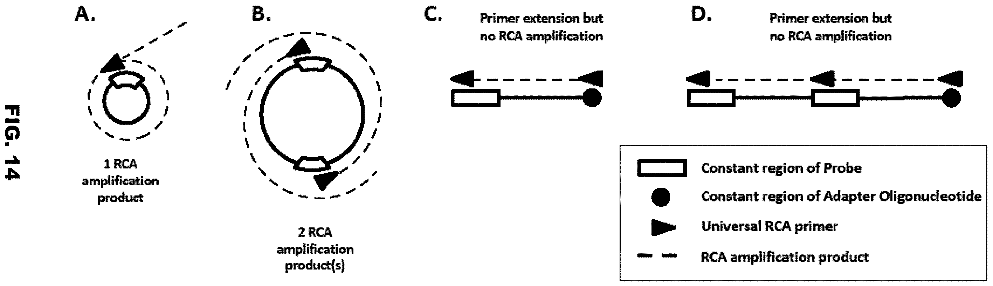

FIG. 14A illustrates the binding of a universal primer for RCA to an intramolecular circular ligation product. Here, the universal primer is shown at least partially hybridized to a complementary portion of a constant region of a probe of an extended probe-target nucleic acid molecule complex included within the intramolecular circular ligation product. In this particular example, RCA will produce one amplification product.

FIG. 14B illustrates the hybridization of a universal primer for PRCA to an intermolecular circular ligation product including two extended probe-target nucleic acid molecule complexes. Specifically, FIG. 14B illustrates two universal primers at least partially hybridized to complementary portions of two constant regions included within an intermolecular circulation ligation product. For instance, each universal primer is shown at least partially hybridized to a portion of a constant region of a probe of an extended probe-target nucleic acid molecule complex. In this particular example, RCA will produce two amplification products. The skilled artisan will appreciate that one amplification product will be produced by RCA for each extended probe-target nucleic acid molecule complex included within the intermolecular circular ligation product. Thus, the skilled artisan will appreciate that RCA will produce three or more amplification products for intermolecular circular ligation products that include three or more extended probe-target nucleic acid molecule complexes.

FIGS. 14C and 14D illustrate the hybridization of one or more universal primers for PCA to intermolecular linear ligation products. Each of these figures illustrate that RCA will permit primer extension, but produce no RCA amplification products.

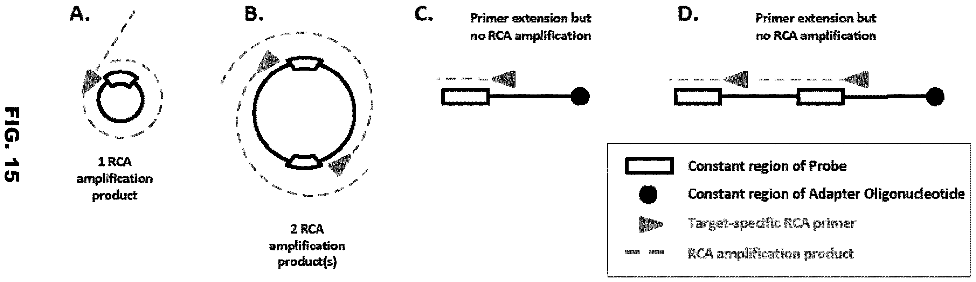

FIGS. 15A - 15D illustrate RCA using target specific RCA primers as opposed to universal primers. In these embodiments, the target specific RCA primers are illustrated as hybridized to portions of the target nucleic acid of the extended probe-target nucleic acid molecule complex of each ligation product. Just as noted herein, the number of amplification products produced by RCA using target specific RCA primers is dependent upon the number of extended probe-target nucleic acid molecule complexes included within each ligation product.

In some embodiments, the RCA reaction mixture (ligation products, polymerase, etc.) is temperature cycled, typically between about 20 to about 40 times. In some embodiments, denaturation of the nucleic acid template sequence is achieved at about 95°C. In some embodiments, the temperature is cooled to about 37°C to about 60°C to anneal the universal primers and/or the target specific RCA primers to the constant regions and/or target sequence. In some embodiments, conventional cycling conditions are about 95°C for about 5 minutes initially to denature all template nucleic acid, followed by about 2 to about 40 repeats of about 95°C for about 30 seconds, about 60°C for about 30 seconds, and about 72°C for about 1 minute.

In some embodiments, the time spent at each temperature can be optimized for specific assays. For instance, the amplification of very short target sequences may require much shorter incubations at each temperature than that of very large target sequences. In some embodiments, each round of temperature cycling results in two times more target sequence than the prior round. In some embodiments, this leads to the exponential amplification of the original template, often resulting in millions or billions of copies of the original nucleic acid target.

In some embodiments, the steps of denaturation, primer annealing and polymerase extension, i.e., denaturation, annealing and extension constitute one "cycle;" there can be numerous "cycles") can be repeated a predetermined number of times (to obtain a high concentration of an amplified segment (the amplicon) of the desired target sequence. For instance, the steps of denaturation, primer annealing and polymerase extension may each be repeated 2 or more times, 4 or more times, 8 or more times, 16 or more times, 32 or more times, 64 or more times, 128 or more times, 256 or more times, etc.

### Sequencing

Following amplification (step 380), the amplified products are sequenced (step 390) and further analyzed. Exemplary sequencing methods that may be utilized include, without limitation thereto, Sanger sequencing and dye-terminator sequencing, as well as next-generation sequencing (NGS) technologies such as pyrosequencing, nanopore sequencing, micropore-based sequencing, nanoball sequencing, MPSS, SOLID, Solexa, Ion Torrent, Starlite, SMRT, tSMS, sequencing by synthesis, sequencing by ligation, mass spectrometry sequencing, polymerase sequencing, RNA polymerase (RNAP) sequencing, microscopy-based sequencing, microfluidic Sanger sequencing, microscopy-based sequencing, RNAP sequencing, tunneling currents DNA sequencing, and in vitro virus sequencing. See WO2014144478, WO2015058093, WO2014106076 and WO2013068528.

DNA sequencing technologies have advanced exponentially. Most recently, high-throughput sequencing (or next-generation sequencing) technologies parallelize the sequencing process, producing thousands or millions of sequences at once. In ultra-high- throughput sequencing as many as 500,000 sequencing-by-synthesis operations may be run in parallel. Next- generation sequencing lowers the costs and greatly increases the speed over the industry standard dye-terminator methods.

Pyrosequencing amplifies DNA inside water droplets in an oil solution (emulsion PCR), with each droplet containing a single DNA template attached to a single primer-coated bead that then forms a clonal colony. The sequencing machine contains many pico liter- volume wells each containing a single bead and sequencing enzymes. Pyrosequencing uses luciferase to generate light for detection of the individual nucleotides added to the nascent DNA, and the combined data are used to generate sequence read-outs. See Margulies, M et al. 2005, Nature, 437, 376-380. Pyrosequencing sequencing is a sequencing-by-synthesis technology that utilizes also utilizes pyrosequencing. Pyrosequencing sequencing of DNA involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil- water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed. In another embodiment, pyrosequencing is used to measure gene expression. Pyrosequecing of RNA applies similar to pyrosequencing of DNA, and is accomplished by attaching applications of partial rRNA gene sequencings to microscopic beads and then placing the attachments into individual wells. The attached partial rRNA sequence is then amplified in order to determine the gene expression profile. Sharon Marsh, Pyrosequencing® Protocols in Methods in Molecular Biology, Vol. 373, 15-23 (2007).

Another example of a sequencing technique that can be used in the methods of the present disclosure is nanopore sequencing (Soni G V and Meller, AClin Chem 53: 1996-2001, 2007). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence. See Bayley, Clin Chem. 2015 Jan; 61(1):25-31.

Another example of a DNA and RNA detection technique that may be used in the methods of the present disclosure is SOLiD^{™} technology (Applied Biosystems). SOLiD^{™} technology systems is a ligation based sequencing technology that may utilized to run massively parallel next generation sequencing of both DNA and RNA. In DNA SOLiD^{™} sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in micro-reactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In other embodiments, SOLiD^{™} Serial Analysis of Gene Expression (SAGE) is used to measure gene expression. Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484 487 (1995); and Velculescu et al., Cell 88:243 51 (1997).

Another sequencing technique that can be used in the methods of the present disclosure includes, for example, Helicos True Single Molecule Sequencing (tSMS) (Harris T. D. et al. (2008) Science 320: 106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm. The flow cell is then loaded into an instrument, e.g., HeliScope sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step. Further description of tSMS is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Another example of a sequencing technology that may be used in the methods of the present disclosure includes the single molecule, real-time (SMRT) technology of Pacific Biosciences to sequence both DNA and RNA. In SMRT, each of the four DNA bases is attached to one of four different fluorescent dyes. These dyes are phospho-linked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated. In order to sequence RNA, the DNA polymerase is replaced with a reverse transcriptase in the ZMW, and the process is followed accordingly.

Another example of a sequencing technique that can be used in the methods of the present disclosure involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used in the methods of the present disclosure involves using an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

DNA nanoball sequencing is a type of high throughput sequencing technology used to determine the entire genomic sequence of an organism. The method uses rolling circle replication to amplify small fragments of genomic DNA into DNA nanoballs. Unchained sequencing by ligation is then used to determine the nucleotide sequence. This method of DNA sequencing allows large numbers of DNA nanoballs to be sequenced per run. See WO2014122548 and Drmanac et al., Science. 2010 Jan 1;327(5961):78-81; Porreca, Nat Biotechnol. 2010 Jan;28(1):43-4.

Massively Parallel Signature Sequencing (MPSS) was one of the earlier next- generation sequencing technologies. MPSS uses a complex approach of adapter ligation followed by adapter decoding, reading the sequence in increments of four nucleotides.

Polony sequencing combines an in vitro paired-tag library with emulsion PCR, an automated microscope, and ligation-based sequencing chemistry to sequence an E. coli genome. The technology was also incorporated into the Applied Biosystems SOLiD platform.

In Solexa sequencing, DNA molecules and primers are first attached on a slide and amplified with polymerase so that local clonal colonies, initially coined "DNA colonies", are formed. To determine the sequence, four types of reversible terminator bases (RT -bases) are added and non-incorporated nucleotides are washed away. Unlike pyrosequencing, the DNA chains are extended one nucleotide at a time and image acquisition can be performed at a delayed moment, allowing for large arrays of DNA colonies to be captured by sequential images taken from a single camera. SOLiD technology employs sequencing by ligation. Here, a pool of all possible oligonucleotides of a fixed length are labeled according to the sequenced position.

Oligonucleotides are annealed and ligated; the preferential ligation by DNA ligase for matching sequences results in a signal informative of the nucleotide at that position. Before sequencing, the DNA is amplified by emulsion PCR. The resulting beads, each containing single copies of the same DNA molecule, are deposited on a glass slide. The result is sequences of quantities and lengths comparable to Solexa sequencing.

In Ion Torrent^{™} sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and is attached at a resolution such that the fragments are individually resolvable. Addition of one or more nucleotides releases a proton (H+), which signal detected and recorded in a sequencing instrument. The signal strength is proportional to the number of nucleotides incorporated. Ion Torrent data may also be output as a FASTQ file. See U.S. publication numbers 2009/0026082, 2009/0127589, 2010/0035252, 2010/0137143, 2010/0188073, 2010/0197507, 2010/0282617, 2010/0300559, 2010/0300895, 2010/0301398, and 2010/0304982.

### EXAMPLES

### Example 1 - Phage Lambda Experiment

The Objective of this experiment was to attempt to perform a single primer extension a-tailing, ligation and RCA reaction using Lambda DNA as our model system. In this model system we targeted 3 closely aligned sites with expected product sizes 236bp, 104bp and 57bp.

Probes were designed to target the 2,322 bp HindIII fragment of enterobacteria phage lambda (NCBI GenBank ACCESSION = NC_001416; VERSION = NC_001416.1, GI:9626243). The template for the experiment was commercial Lambda x HindIII DNA molecular weight marker (1ng). Three probes (such as any of the probes described herein) were designed, with different expected product sizes. All probes were ordered from Integrated DNA Technologies (IDT). Amplification primers, used to amplify the product and introduce sample indexes and Illumina sequencing adapters, were identical to those used in the Roche HEAT-Seq product.

Phage Lambda x HindIII DNA was used as the target (see SEQ ID NO: 10).

AGCTTTTCTAATTTAACCTTTGTCAGGTTACCAACTACTAA
GGTTGTAGGCTCAAGAGGGTGTGTCCTGTCGTAGGTAAATAACTGACCT

GTCGAGCTTAATATTCTATATTGTTGTTCTTTCTGCAAAAAAGTGGGGA
AGTGAGTAATGAAATTATTTCTAACATTTATCTGCATCATACCTTCCGA
GCATTTATTAAGCATTTCGCTATAAGTTCTCGCTGGAAGAGGTAGTTTTT
TCATTGTACTTTACCTTCATCTCTGTTCATTATCATCGCTTTTAAAACGG
TTCGACCTTCTAATCCTATCTGACCATTATAATTTTTTAGAATGGTTTCA
TAAGAAAGCTCTGAATCAACGGACTGCGATAATAAGTGGTGGTATCCA
GAATTTGTCACTTCAAGTAAAAACACCTCACGAGTTAAAACACCTAAGT
TCTCACCGAATGTCTCAATATCCGGACGGATAATATTTATTGCTTCTCTT
GACCGTAGGACTTTCCACATGCAGGATTTTGGAACCTCTTGCAGTACTA
CTGGGGAATGAGTTGCAATTATTGCTACACCATTGCGTGCATCGAGTAA
GTCGCTTAATGTTCGTAAAAAAGCAGAGAGCAAAGGTGGATGCAGATG
AACCTCTGGTTCATCGAATAAAACTAATGACTTTTCGCCAACGACATCT
ACTAATCTTGTGATAGTAAATAAAACAATTGCATGTCCAGAGCTCATTC
GAAGCAGATATTTCTGGATATTGTCATAAAACAATTTAGTGAATTTATC
ATCGTCCACTTGAATCTGTGGTTCATTACGTCTTAACTCTTCATATTTAG
AAATGAGGCTGATGAGTTCCATATTTGAAAAGTTTTCATCACTACTTAG
TTTTTTGATAGCTTCAAGCCAGAGTTGTCTTTTTCTATCTACTCTCATAC
AACCAATAAATGCTGAAATGAATTCTAAGCGGAGATCGCCTAGTGATTT
TAAACTATTGCTGGCAGCATTCTTGAGTCCAATATAAAAGTATTGTGTA
CCTTTTGCTGGGTCAGGTTGTTCTTTAGGAGGAGTAAAAGGATCAAATG
CACTAAACGAAACTGAAACAAGCGATCGAAAATATCCCTTTGGGATTC
TTGACTCGATAAGTCTATTATTTTCAGAGAAAAAATATTCATTGTTTTCT
GGGTTGGTGATTGCACCAATCATTCCATTCAAAATTGTTGTTTTACCAC
ACCCATTCCGCCCGATAAAAGCATGAATGTTCGTGCTGGGCATAGAATT
AACCGTCACCTCAAAAGGTATAGTTAAATCACTGAATCCGGGAGCACTT
TTTCTATTAAATGAAAAGTGGAAATCTGACAATTCTGGCAAACCATTTA
ACACACGTGCGAACTGTCCATGAATTTCTGAAAGAGTTACCCCTCTAAG
TAATGAGGTGTTAAGGACGCTTTCATTTTCAATGTCGGCTAATCGATTT
GGCCATACTACTAAATCCTGAATAGCTTTAAGAAGGTTATGTTTAAAAC
CATCGCTTAATTTGCTGAGATTAACATAGTAGTCAATGCTTTCACCTAA
GGAAAAAAACATTTCAGGGAGTTGACTGAATTTTTTATCTATTAATGAA
TAAGTGCTTACTTCTTCTTTTTGACCTACAAAACCAATTTTAACATTTCC
GATATCGCATTTTTCACCATGCTCATCAAAGACAGTAAGATAAAACATT

GTAACAAAGGAATAGTCATTCCAACCATCTGCTCGTAGGAATGCCTTAT
TTTTTTCTACTGCAGGAATATACCCGCCTCTTTCAATAACACTAAACTCC
AACATATAGTAACCCTTAATTTTATTAAAATAACCGCAATTTATTTGGC
GGCAACACAGGATCTCTCTTTTAAGTTACTCTCTATTACATACGTTTTCC
ATCTAAAAATTAGTAGTATTGAACTTAACGGGGCATCGTATTGTAGTTT
TCCATATTTAGCTTTCTGCTTCCTTTTGGATAACCCACTGTTATTCATGTT
GCATGGTGCACTGTTTATACCAACGATATAGTCTATTAATGCATATATA
GTATCGCCGAACGATTAGCTCTTCAGGCTTCTGAAGAAGCGTTTCAAGT
ACTAATAAGCCGATAGATAGCCACGGACTTCGTAGCCATTTTTCATAAG
TGTTAACTTCCGCTCCTCGCTCATAACAGACATTCACTACAGTTATGGC
GGAAAGGTATGCATGCTGGGTGTGGGGAAGTCGTGAAAGAAAAGAAGT
CAGCTGCGTCGTTTGACATCACTGCTATCTTCTTACTGGTTATGCAGGTC
GTAGTGGGTGGCACACAA (SEQ ID NO: 10)

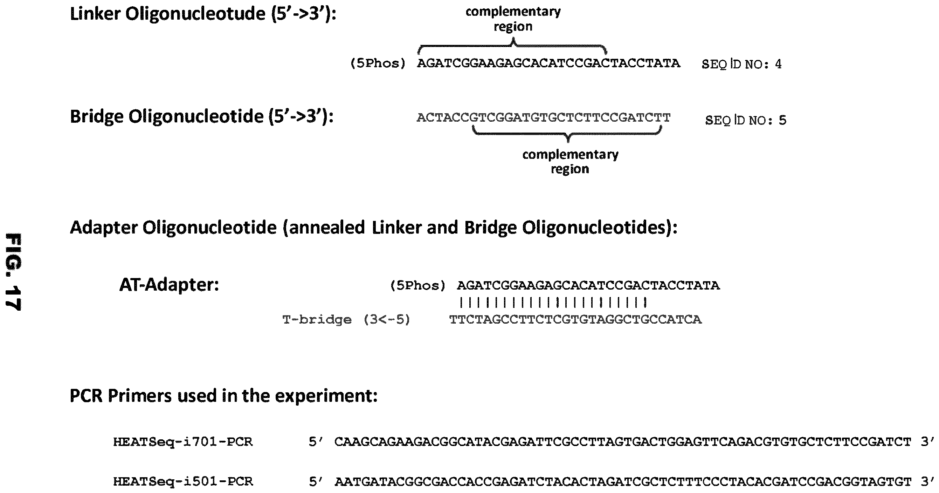

Probes and oligonucleotides are as described in FIGS. 16 and 17.

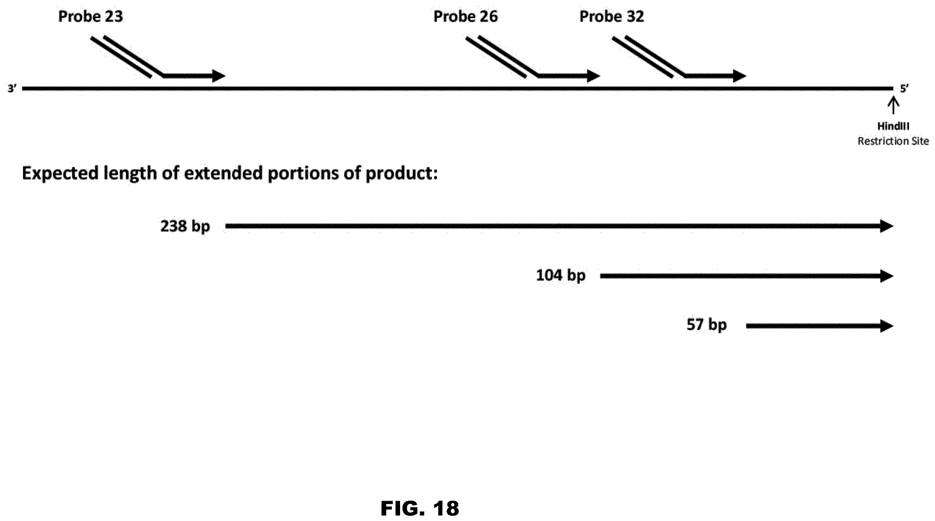

The relative locations of probes 23, 26, and 32 (see FIG. 16) on Lambda DNA are illustrated in FIG. 18.

### Protocol

Lambda Phage x HindIII as template (1 ng)
Mix template with extension primer(s), buffer, and polymerase.
Denature, anneal, extend, A-tail.
Add T-bridge adapter; denature and anneal 60° with slow ramp to 4°
Ligate with or without including the AT-adapter (both worked).
Purify and amplify using the target Specific RCA primers
Add Sequencing Adapters using HEAT-Seq PCR primers.
Sequence (Illumina MiSeq 2x101 PE sequencing).
Analyze (map reads, BWA).
Visualize mapped reads (IGV).

In this study Lambda DNA diluted to 1ng/µl was fragmented to1 kb using a covaris LE220 sonciator. Ten microliter of sonicated DNA was aliquoted into strip tubes. Extension/A-tailing master mix containing 1.5µl extension primers at 12 µM (IDT), 12.5 µl 2X 2G Taq Multiplex enzyme master mix was prepared on ice 13.5 ul was added to the sonicated DNA. The sample was placed in Proflex PCR system thermal cycler incubated at 95°C for 5min followed by 9 cycles of 95°C for 30 seconds, 80°C for 0.01seconds ramp down to 48°C at a rate of 0.1°/second, 48°C for 45 seconds, 72°C for 90 seconds, and a final extension of 72°C for 5 minutes. After this extension and A-tailing 1 µl of T-bridge Adapter 12µM was added to the extended product. Reaction containing the T-bridge adapter were incubated at 95°C for 5 mins, slow ramp down to 60°C at a rate of 0.1°C /second incubated at 60°C for 10 min, slow ramp down to 4°C at a rate of 0.1°C /second hold at 4°C overnight.

The ligation Master Mix was setup on ice using New England Bio labs T4 DNA Ligase and buffer was added at volume 25 µl to the T-bridged adapted product to provide a total volume of 50 µl. Ligation reactions were incubated at overnight at 16°C. After Ligation reaction, 1.25 µl of Exonuclease I and Exonuclease III was added to each reaction and incubated at 37°C for 30 minutes, followed by a 95°C heat kill for 5mins.

This 25 µl purified product was added to the following Rolling Circle Amplification Master mix containing 2.5 µl 10X Phi29 buffer, 2 µl of 12 µM target specific Rolling Circle Amplification (RCA), and primers, and 5.38 of PCR Grade water. Denature reaction containing target specific RCA primers by incubating at 98°C for 3 minutes, immediately quick chill sample in ice water bath for 2 minutes. Prepare Second Master mix containing 2.5 µl 10X Phi29 buffer, 1 µl Phi29 enzyme (10,000units/ml), 0.5 µl 100X BSA, 3.125 µl 20% DMSO, 10mM DNTP 3.125 µl, 4.87 µl of PCR Grade water. Added 15.12 µl of Second Master Mix to Denatured reaction incubate at 60°C for 30 minutes, heat kill at 95°C for 10 minutes RCA reaction was purified with 0.75X Ampure XP beads and eluted in 22.5 µl.

Indexing of Amplified Product was done using a 5X phusion buffer master mix, added 2 µl of 10mM deoxynucleotide triphosphate, 4 µl of 5uM Nextera Index A, 4 µl of 5uM Nextera Index B and 47.5 µl of Nuclease Free water. Combined 22.5 µl Of Amplified product to the 77.5 µl of the Phusion master mix. Place in Proflex PCR system thermal cycler programmed to 98°C 30 second, followed by 30 cycles of 98°C for 10 seconds, 60°C for 30 seconds, 72°C for 20 seconds, final extensions at 72°C for 120 seconds, hold at 4°C.

Amplified Libraries were diluted to 2nM, pooled and sequenced on the illumine Miseq using the Miseq Reagent v3 Kit (600 cycles) sequenced pair reads 2x101. Custom RD1 Sequencing primer was added to the sequencing run. Added 3.4 ul of 100uM Custom Read 1 sequencing primer 5'-TCTTTCCCTACACGATCCGACGGTAGTGT-3' (SEQ ID NO: 11) to Position of 12 of sequencing cartridge.

### Results

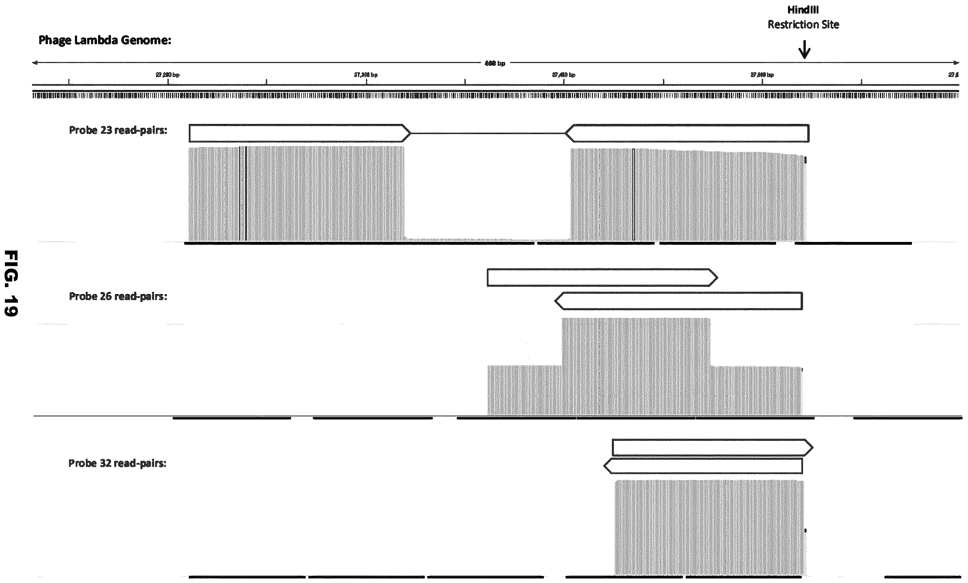

The results are illustrated in FIGS. 19, 20, and 21.

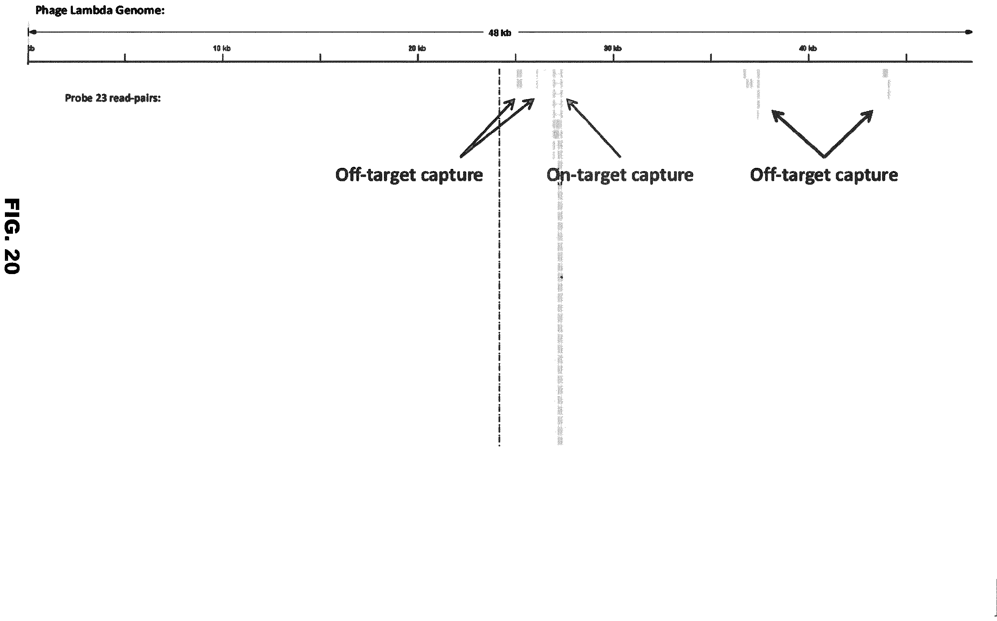

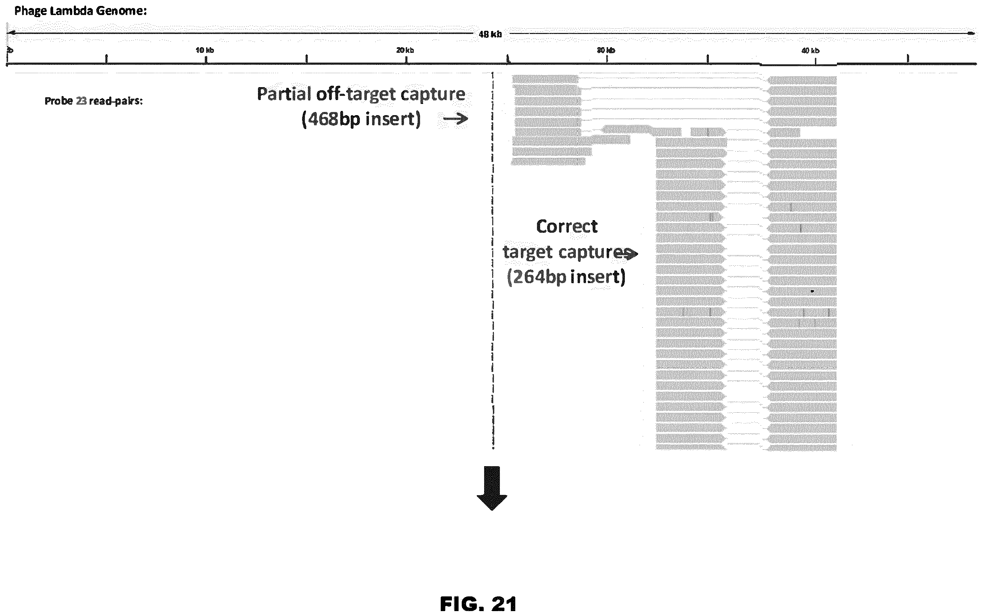

Sequenced Reads aligned to the expected target regions of the Lambda genome and visualized to using IGV. All of the expected product sizes were observed in the sequenced reads FIG 19. The reads extended to the HINDIII restriction sited in the Lambda genome FIG 19. We observed some off-target aligned reads from our probe sets, but the majority of our probes selected covered our target region FIG 20. The sequenced reads aligned to the Lambda genome extended past the expected target of 236bp up to 486bp. We were still able to capture our target of interest FIG 21.

### Example 2 - Human gDNA experiment

The objective of this experiment our single primer extension a-tailing, ligation and RCA method on human genomic DNA. Determine our ability to detect gene variants using HD734 cell line with variable allele frequencies down to 1%.

Probes (including any of the probes described herein) were designed using existing HEAT-Seq probe extension primer sequences (hg19 coordinates) to target a series of engineered cancer mutations that are present at variable allele frequencies in cell line DNA mixtures (Horizon Discovery). All probes were ordered from Integrated DNA Technologies (IDT). 10ng of gDNA (HD734; Tru-Q 7 Reference Standard; 1.3% Tier) was used as template for capture. Fragmented ~1kb (Covaris). RCA was used for initial amplification, followed by inverse PCR to incorporate sequencing adapters and sample indexes.

### Equipment, Materials and Reagents

### Equipment:

PCR Thermocycler
Mini centrifuge
Eppendorf centrifuge
Vortex
Agilent Bioanalyzier or Tapestation instrument
Illumina Sequencing Platforms
Qubit
Nanodrop

### Reagents and chemicals:

Tru-Q NGS DNA 7 Multiplex gDNA Multiplex, 1 µg (horizon, Cat# HD734)
KAPA 2x 2G Multiplex Enzyme Master mix
12uM of the Primer Set of the present disclosure (1-19) custom
12uM T-bridge Primer set (custom)
Deionized & nuclease-free water (Fisher Scientific, Cat#4387936)
Neb T4 Ligase and Ligase buffer (NEB, Cat #M0202)
Neb Exonuclease I (Neb, Cat#M0293)
Neb Exonulcease III (Neb, Cat#M0206L)
Ampure Beads
NEB Phi 29 enzyme and buffer (Neb, Cat# M0269L)
100X BSA (Neb, Cat# B9200)
DMSO (sigma, cat# D9170-1VL)
DNTP
Thermofisher 5X Phusion PCR Mix (Neb, Cat# M0530)
Illumina Index primers

### Experimental Protocol

Setup the following components on ice:

| **Component** | **1X reaction (25 µl)** |
|---|---|
| 1kb covarise sheared HD734 or Lambda DNA (1ng/ul) | 10 µl |
| 2X 2G Mulitplex enzyme Mastermix | 12.5 µl |
| 12uM NP-primers 1-19 | 1.5ul µl |
| | 25ul |

Place combined components into 0.2ml strip tubes place in PCR Thermocycler programed with the following settings

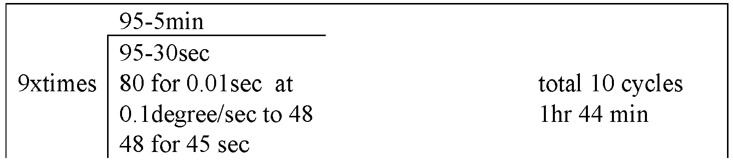

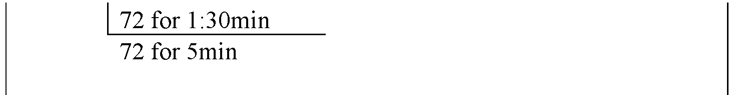

After the program has completed. Add into the amplified reaction about 1ul of 12uM T-bridge primer.

Place amplified samples back into the thermocycler and programed with the following conditions:

| |
|---|
| 95 for 5min |
| 60 for 0.1degree/sec |
| 60 for 10min |
| 0.01 degree/sec to 4 |
| 4 degree forever |

Setup following Ligation Master Mix reaction components:

| **Component** | **1X reaction (25 µl)** |
|---|---|
| NEB 10X T4 Ligase buffer | 5 µl |
| NEB T4 Ligase enzyme | 2 µl |
| Water | 18µl |
| | 25 µl |

Add about 25ul of Ligation Master Mix to the T-Bridged DNA product
Incubate for about 2 hour or overnight at about 16°C
Prepare an Exonuclease I/III master mix

| **Component** | **1X reaction (25 µl)** |
|---|---|
| NEB Exonuclease I | 1.25 µl |
| NEB Exonuclease III | 1.25 µl |
| | 2.5ul |

Add about 2.5ul of Exonuclease Master Mix to each reaction tube.
Incubate at about 37°C for about 30 minutes
Heat kill reaction at about 95°C for about 5 minutes
Perform a .75x bead based cleanup by combine the following components

| **Component** | **1X reaction (25 µl)** |
|---|---|
| DNA product | 53.5µl |
| Ampure Beads | 75 µl |
| Nuclease Free water | 46.5 µl |

Thoroughly resuspend the beads by pipetting up and down or vortexing
Incubate the tubes at room temperature for 5 min to bind DNA to beads.
Place tubes on a magnet to capture the beads. Incubate until the liquid is clear.
Carefully remove and discard 95 µl of supernatant
Keeping the tubes on the magnet add about 200ul of about 70% ethanol
Incubate the tubes on the magnet at room temperature for ≥30 sec.
Carefully remove and discard the ethanol.
Keeping the tubes on the magnet add about 200ul of about 70% ethanol
Incubate the tubes on the magnet at room temperature for ≥30 sec.
Carefully remove and discard the ethanol. Try to remove all residual ethanol without disturbing the beads.
Dry the beads at room temperature for about 3 to about 5min, or until all of the ethanol has evaporated.
Resuspend beads by adding about 27 µl of water; thoroughly resuspend the beads by vortexing or pipetting up and down.
Incubate the tubes at room temperature for about 3 min to elute the DNA off the beads.
Place the tubes on a magnet to capture the beads. Incubate until the liquid is clear.
Carefully transfer about 25 µl of supernatant into a new tube.

### Prepare RCA Amplification with template specific Primers

### Prepare Two Separate RCA Master Mixes

| **Component Master Mix 1** | **1X reaction (25 µl)** |
|---|---|
| 10X Phi29 Buffer | 2.5 µl |
| 12uM RCA Primers | 2 µl |
| water | 5.38 µl |
| total | 9.88ul |

| **Component Master Mix 2** | **1X reaction (25 µl)** |
|---|---|
| 10x Phi29 buffer | 2.5 µl |
| 100x BSA | 0.5 µl |
| 20% DMSO | 3.125ul µl |
| 10uM DNTP | 3.125 µl |
| Phi29 enzyme | 1 |
| Water | 4.87 |

Add 9.88 µl of Master Mix 1 to the 25 µl of DNA
Incubate for 3 minutes at 95°C
Quick Chill in ice water bath for 2 minutes
Add 15.12 µl of Master Mix 2 for the denatured
Incubate for 60 mins at 30°C
Heat kill at 65°C for 10mins
Perform a .75x bead based cleanup by combine the following components

| **Component** | **1X reaction (25 µl)** |
|---|---|
| RCA Reactions | 50µl |
| Ampure Beads | 75 µl |
| Nuclease Free water | 50 µl |

Thoroughly resuspend the beads by pipetting up and down or vortexing
Incubate the tubes at room temperature for 5 min to bind DNA to beads.
Place tubes on a magnet to capture the beads. Incubate until the liquid is clear.
Carefully remove and discard 95 µl of supernatant
Keeping the tubes on the magnet add 200ul of 70% ethanol
Incubate the tubes on the magnet at room temperature for ≥30 sec.
Carefully remove and discard the ethanol.
Keeping the tubes on the magnet add 200ul of 70% ethanol
Incubate the tubes on the magnet at room temperature for ≥30 sec.
Carefully remove and discard the ethanol. Try to remove all residual ethanol without disturbing the beads.
Dry the beads at room temperature for about 3 to about 5 minutes, or until all of the ethanol has evaporated.
Resuspend beads by adding 25 µl of water, thoroughly suspend the beads by vortexing or pipetting up and down.
Incubate the tubes at room temperature for 3 min to elute the DNA off the beads.
Place the tubes on a magnet to capture the beads. Incubate until the liquid is clear.
Carefully transfer 22.5 µl of supernatant into a new tube.

### Amplification for Indexing

Setup the Amplification Master Mix on Ice

| **Component** | **1X reaction (25 µl)** |
|---|---|
| 5X Phusion Buffer | 20 µl |
| 10mM DNTP | 2 µl |
| Index primer A 5uM | 4 µl |
| Index primer B 5uM | 4 µl |
| Water | 47.5 µl |
| Total | 77.5 µl |

Add 77.5 µl of Amplification Master Mix to the 22.5ul RCA material place in thermocycler and run the following program

| | |
|---|---|
| | 98°C 30sec |
| 30xtimes | 98°C 10sec |
| | 60°C 30sec |
| | 72°C 20sec |
| | 72°C 2mins |
| | Hold at 4 |

24 cycles for 20ng or 30 cycles for 10ng
Perform a .75x bead based cleanup by combine the following components

| **Component** | **1X reaction (25 µl)** |
|---|---|
| PCR Reactions | 100µl |
| Ampure Beads | 75 µl |
| | |

Thoroughly resuspend the beads by pipetting up and down or vortexing
Incubate the tubes at room temperature for 5 min to bind DNA to beads.
Place tubes on a magnet to capture the beads. Incubate until the liquid is clear.
Carefully remove and discard 95 µl of supernatant
Keeping the tubes on the magnet add 200ul of 70% ethanol
Incubate the tubes on the magnet at room temperature for ≥30 sec.
Carefully remove and discard the ethanol.
Keeping the tubes on the magnet add 200ul of 70% ethanol
Incubate the tubes on the magnet at room temperature for ≥30 sec.
Carefully remove and discard the ethanol. Try to remove all residual ethanol without disturbing the beads.
Dry the beads at room temperature for 3-5min, or until all of the ethanol has evaporated.
Resuspend beads by adding 27 µl of water; thoroughly suspend the beads by vortexing or pipetting up and down.
Incubate the tubes at room temperature for 3 min to elute the DNA off the beads.
Place the tubes on a magnet to capture the beads. Incubate until the liquid is clear.
Carefully transfer 25 µl of supernatant into a new tube.
Store product at -20 until sequencing
Quantitate by Nanodrop or qubit assess product sizes by Bioanalyzier or Tapestation
Pool and Sequence on Miseq or Nextseq

### Description, Results, and Discussion

In this study Horizon Genomic DNA HD734 diluted to 1ng/µl was fragmented to 1 kb using a covaris LE220 sonciator. Ten microliter of sonicated DNA was aliquoted into strip tubes. Extension/A-tailing master mix containing 1.5µl extension primers at 12 µM (IDT), 12.5 µl 2X 2G Taq Multiplex enzyme master mix was prepared on ice 13.5 ul was added to the sonicated DNA. Sample was placed in Proflex PCR system thermal cycler incubated at 95°C for 5min followed by 9 cycles of 95°C for 30 seconds, 80°C for 0.01seconds ramp down to 48°C at a rate of 0.1°/second, 48°C for 45 seconds, 72°C for 90 seconds, and a final extension of 72°C for 5 minutes. After this extension and A-tailing 1 µl of T-bridge Adapter 12µM was added to the extended product. Reaction containing the T-bridge adapter were incubated at 95°C for 5 mins, slow ramp down to 60°C at a rate of 0.1°C /second incubated at 60°C for 10 min, slow ramp down to 4°C at a rate of 0.1°C /second hold at 4°C overnight.

The ligation Master Mix was setup on ice using New England Bio labs T4 DNA Ligase and buffer was added at volume 25 µl to the T-bridged adapted product total volume is 50 µl. Ligation reactions were incubated at overnight at 16°C. After Ligation reaction 1.25 µl of Exonuclease I and Exonuclease III was added to each reaction and incubated at 37°C for 30 minutes, followed by a 95°C heat kill for 5mins. Exonucleases reaction was purified with a 0.75X Ampure XP beads and eluted in 25 µl water.

This 25 µl purified product was added to the following Rolling Circle Amplification Master mix containing 2.5 µl 10X Phi29 buffer, 2 µl of 12 µM target specific Rolling Circle Amplification (RCA), and primers, and 5.38 of PCR Grade water. Denature reaction containing target specific RCA primers by incubating at 98°C for 3 minutes, immediately quick chill sample in ice water bath for 2 minutes. Prepare Second Master mix containing 2.5 µl 10X Phi29 buffer, 1 µl Phi29 enzyme (10,000units/ml), 0.5 µl 100X BSA, 3.125 µl 20% DMSO, 10mM DNTP 3.125 µl, 4.87 µl of PCR Grade water. Added 15.12 µl of Second Master Mix to Denatured reaction incubate at 60°C for 30 mins, heat kill at 95°C for 10 mins. RCA reaction was purified with 0.75X Ampure XP beads and eluted in 22.5 µl. Tested bead cleanup down to .65X Ampure Beads at this step.

Indexing of Amplified Product was done using a 5X phusion buffer master mix, added 2 µl of 10mM deoxynucleotide triphosphate, 4 µl of 5uM Index A, 4 µl of 5uM Index B and 47.5 µl of Nuclease Free water. Combined 22.5 µl Of Amplified product to the 77.5 µl of the Phusion master mix. Place in Proflex PCR system thermal cycler programmed to 98°C 30 second, followed by 30 cycles of 98°C for 10 seconds, 60°C for 30 seconds, 72°C for 20 seconds, final extensions at 72°C for 120 seconds, hold at 4°C. PCR Reaction cleaned up with a 0.75X Ampure XP beads. Tested Bead ratio 0.65X, 0.70X Ampure Beads at this step.

Amplified Libraries were diluted to 2nM, pooled and sequenced on the illumine Miseq using the Miseq Reagent v3 Kit (600 cycles) sequenced pair reads 2x101. Custom RD1 Sequencing primer was added to the sequencing run. Added 3.4 ul of 100uM Custom Read 1 sequencing primer 5'-TCTTTCCCTACACGATCCGACGGTAGTGT -3 (SEQ ID NO: 11) to Position of 12 of sequencing cartridge.

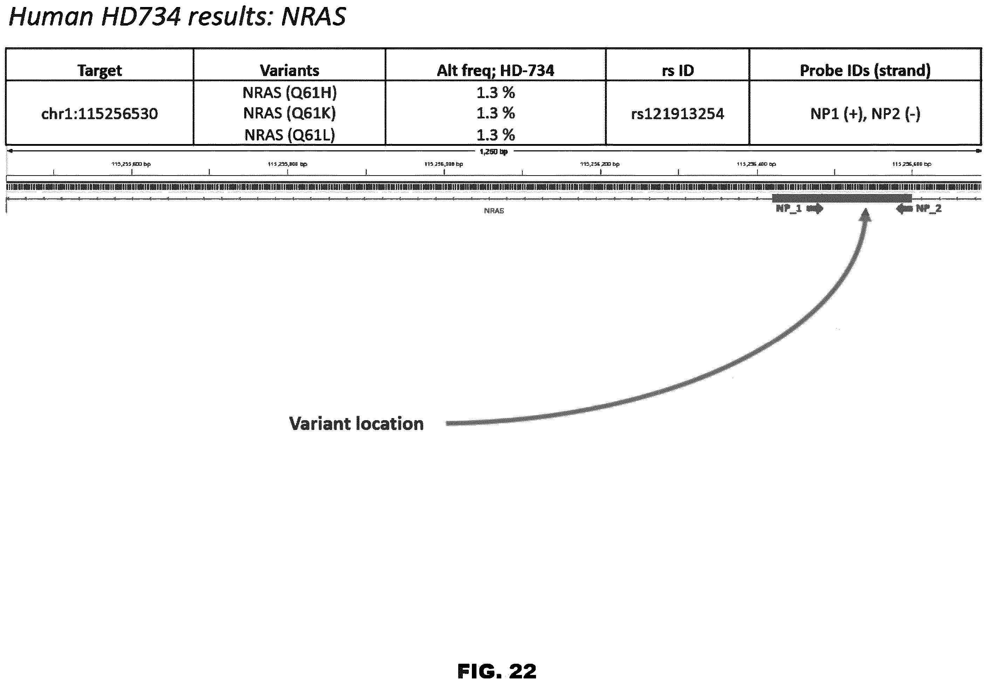

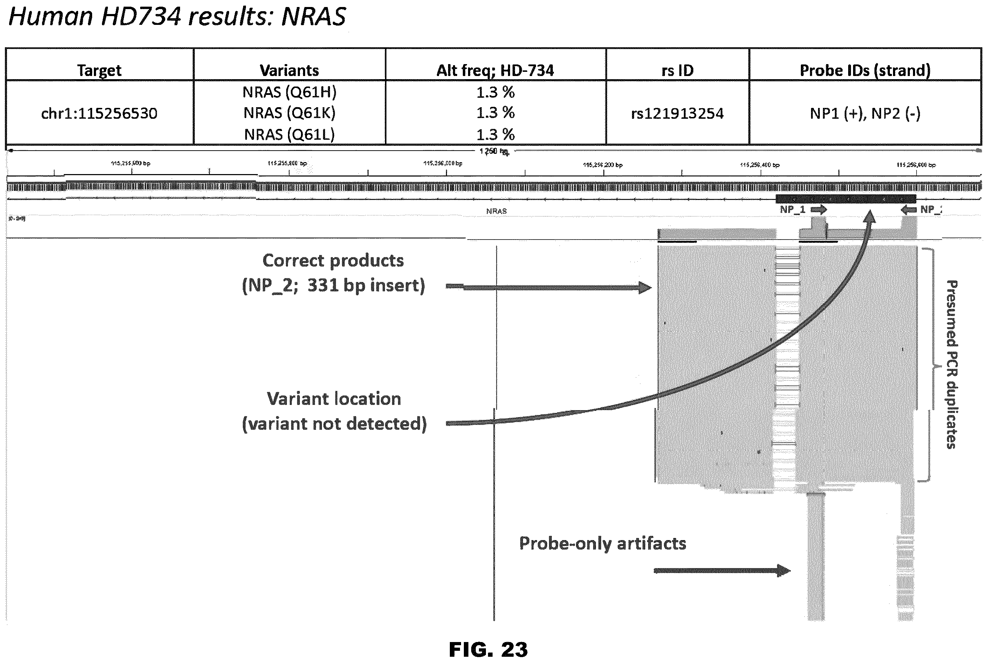

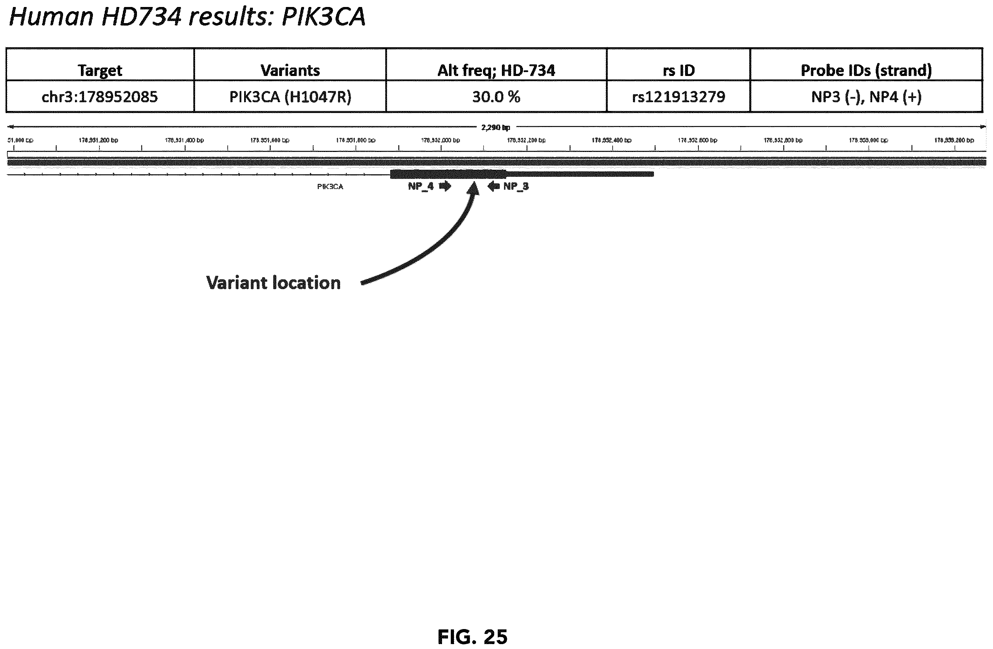

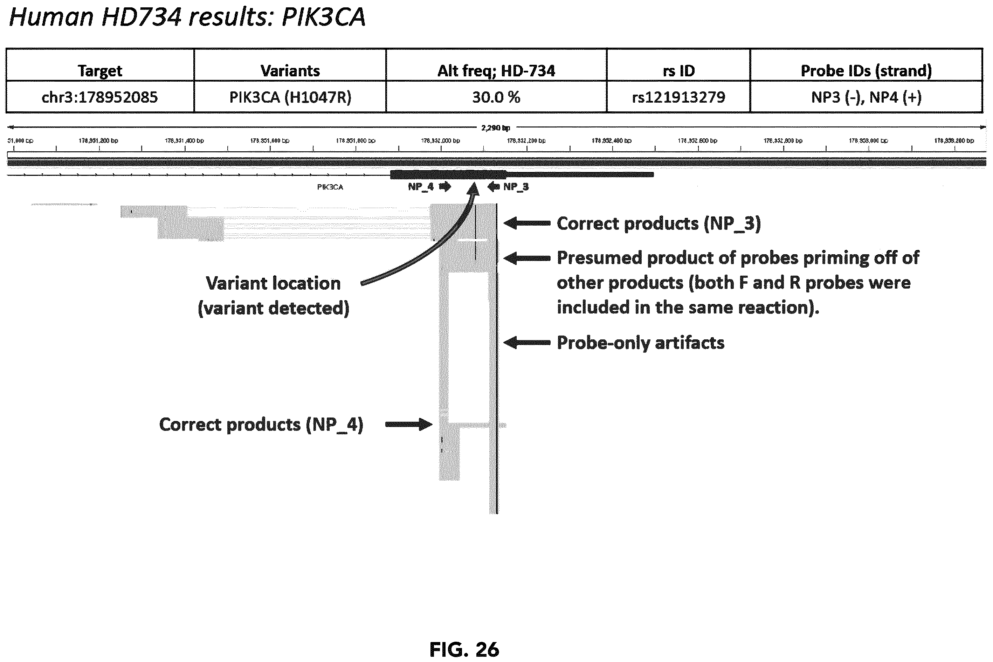

Results are illustrated in FIGS. 22 - 31

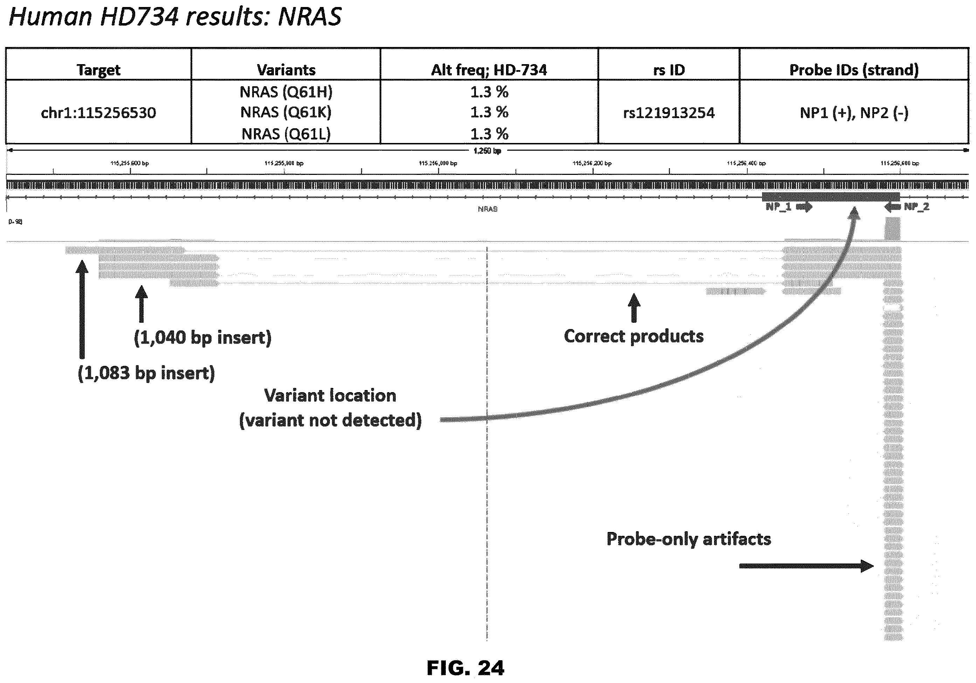

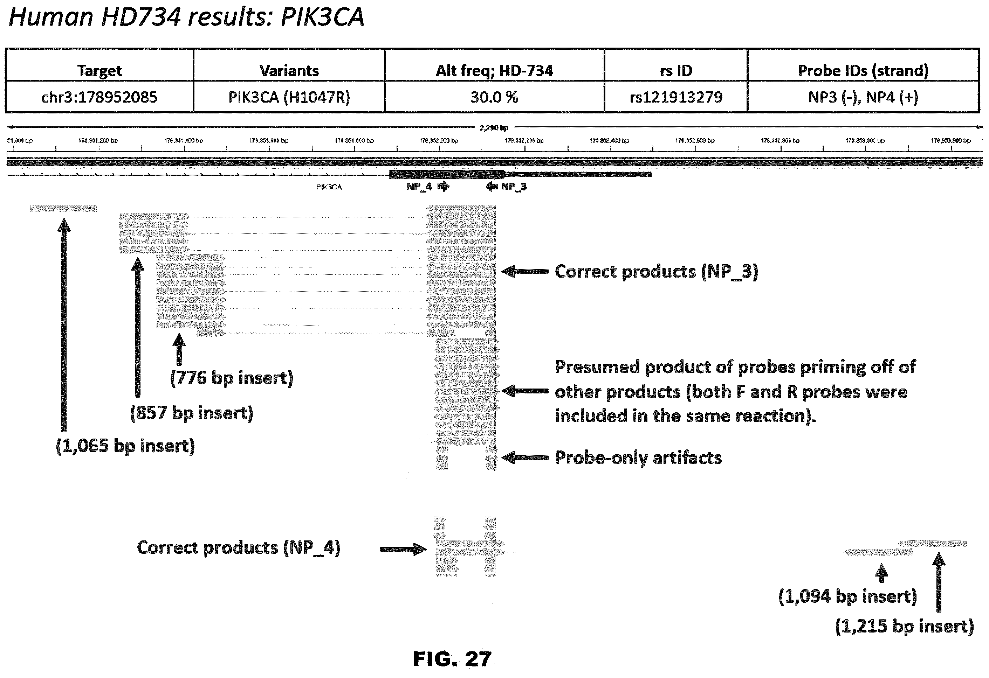

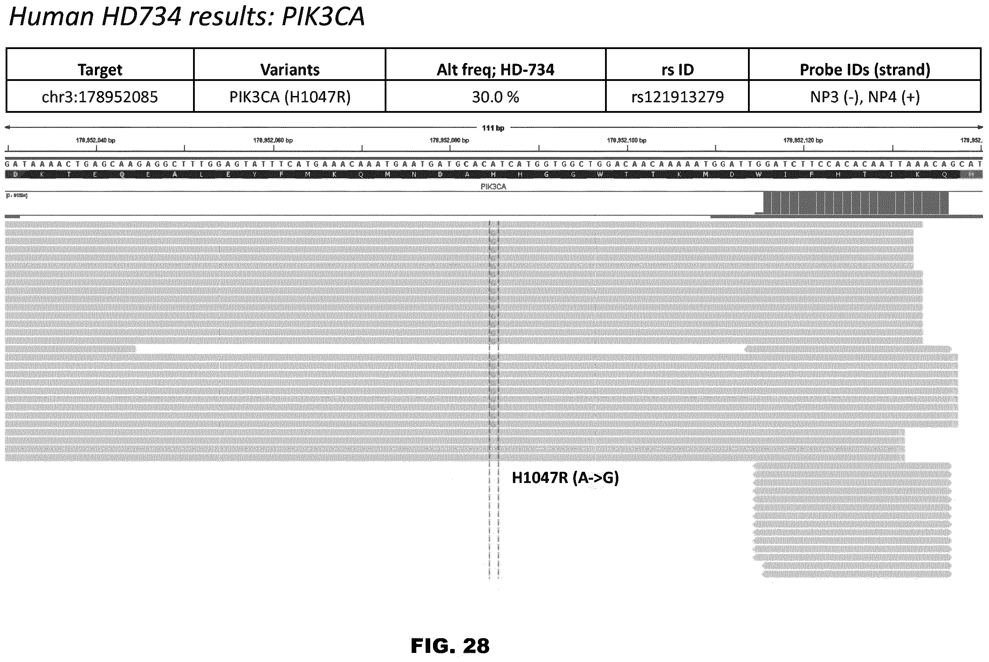

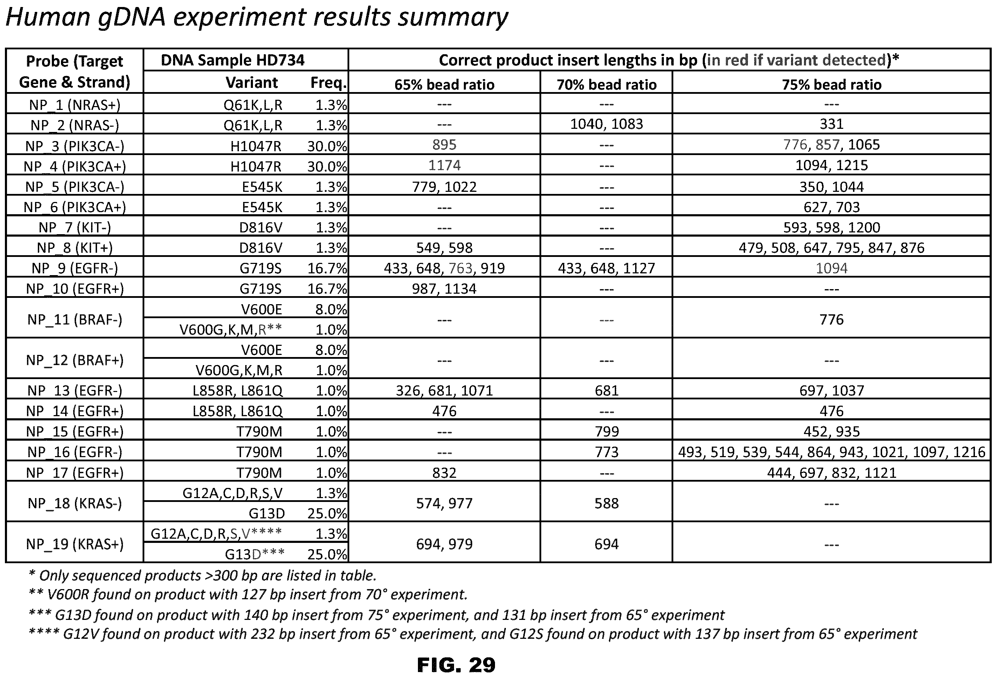

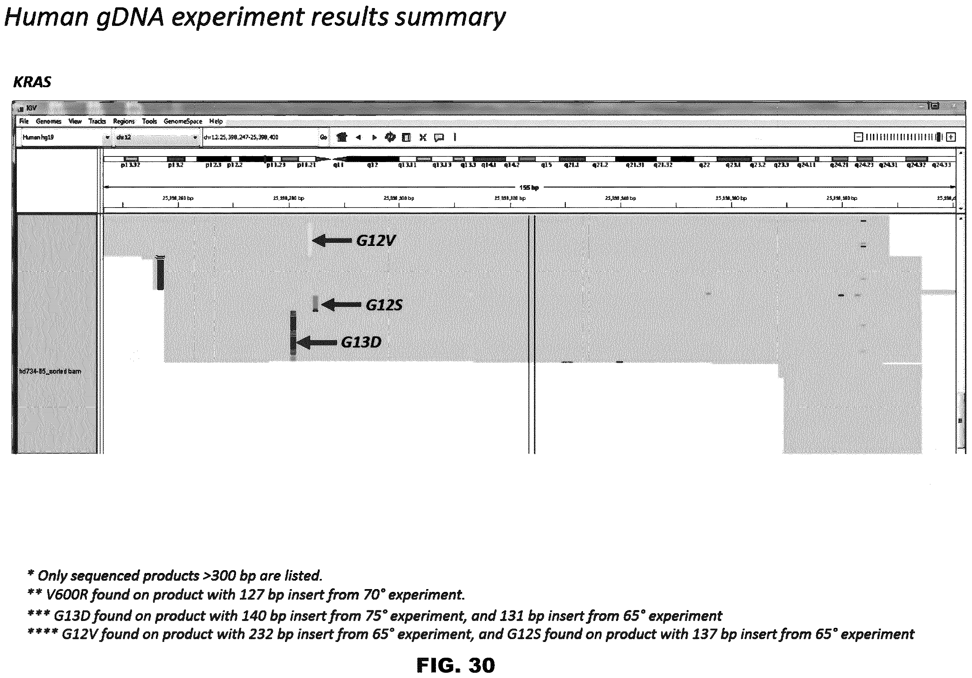

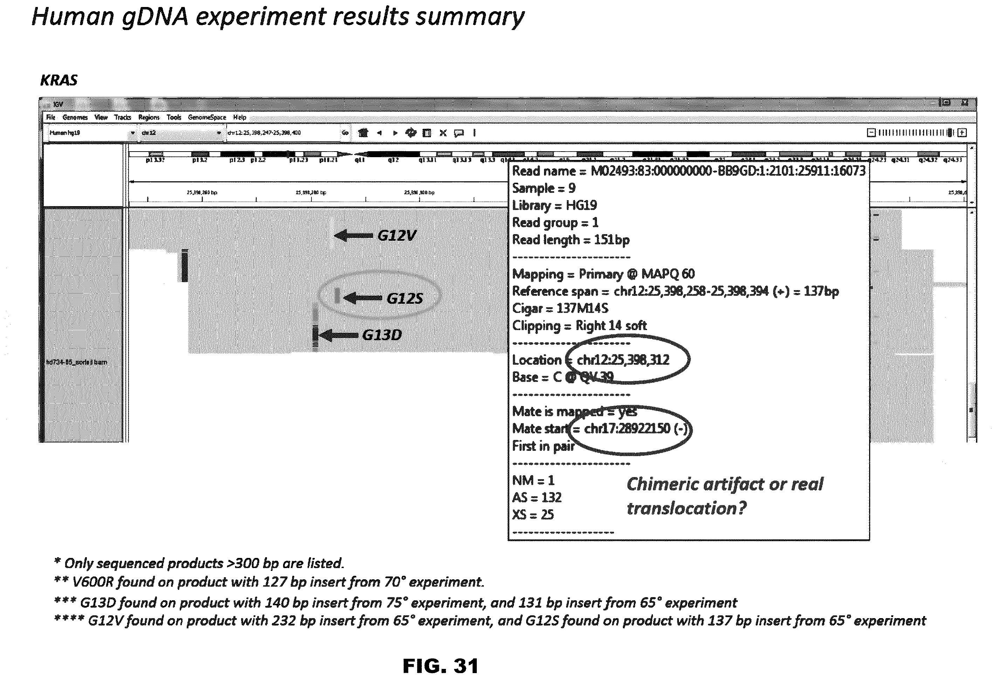

Very little optimization has been done with this method or this probe design therefore we observed a number of probe artifacts this in indicated in most of the IVG images. The probes in this designed targeted 19 regions of the human genome. The selected probes targeted specific cancer genes in the Horizon HD734 cell line. In as little as 10 ng of DNA we were able to align to 16 of the 19 genes after testing different Ampure XP bead cleanup ratios of the RCA product Fig 29. We were able to identify sequence variants present at sub-Mendelian allele frequencies Fig 29. Probes targeting the PICK3CA gene were able to correctly identify in H1047R (A->G) Fig 28. We observed capture products of varying lengths and was able to generate capture products of at least 1.2kb FIG 27&24. A possible chimeric mutation or translocation in KRAS gene detected using this method FIGS. 30 and 31.

## Claims

1. A method of enriching one or more target nucleic acids in a sample, comprising:
(a) annealing one or more probes to the one or more target nucleic acid molecules in the sample to form one or more probe-target nucleic acid molecule complexes, wherein the one or more probes each comprise a constant region and a unique region, wherein the unique region of each probe includes a nucleotide sequence which hybridizes to a complementary nucleic acid sequence within the one or more target nucleic acid molecules;
(b) extending the one or more probe-target nucleic acid molecule complexes to form one or more extended probe-target nucleic acid molecule complexes, and performing A-tailing;
(c) generating one or more ligation products from the one or more extended probe-target nucleic acid molecule complexes;
(d) introducing one or more primers to the generated one or more ligation products, wherein the one or more primers hybridize to one or more complementary portions of the constant region within each of the one or more ligation products; and
(e) amplifying the one or more ligation products in the presence of a polymerase to provide one or more amplification products,
wherein the one or more probes each include a bridge oligonucleotide comprising a 3' overhang and having Formula (II):
3' - [D] - 5' (II),
wherein [D] comprises between 5 and 50 nucleotides, and where at least a portion of the nucleotide sequence of [D] is complementary to the constant region, and the 3' overhang is a T overhang.

2. The method of claim 1, further comprising sequencing the one or more amplification products.

3. The method of claim 1, wherein the one or more ligation products comprise intramolecular ligation products derived from a single extended probe-target nucleic acid molecule complex.

4. The method of claim 1, wherein the one or more ligation products comprise intermolecular ligation products formed from two or more extended probe-target nucleic acid molecule complexes.

5. The method of claim 1, further comprising introducing one or more adapter oligonucleotides to a mixture including the one or more extended probe- target nucleic acid molecule complexes.

6. The method of claim 1, wherein the one or more primers are forward and reverse inverse PCR primers.

7. The method of claim 1, wherein the amplification comprises performing an inverse polymerase chain reaction.

8. The method of claim 1, wherein each of the one or more probes are derived from:
(i) a linker oligonucleotide comprising a 5' phosphate group and having Formula (IA):
5'-[A]-[B]ₓ-[C]_{y}-3' (IA),
wherein [A] is the "constant region" and includes between about 5 and about 60 nucleotides; [B] is an "identifier region" including an identifier nucleic acid sequence; [C] is the "unique region" and includes between about 5 and about 100 nucleotides; x is 0 or 1; and y is 1; and
(ii) a bridge oligonucleotide comprising a 3' overhang and having Formula (II):
3' - [D] - 5' (II),
wherein [D] comprises between about 5 and about 50 nucleotides, and wherein at least a portion of the nucleotide sequence of [D] is complementary to the "constant region" [A], and the 3' overhang is a T overhang.

9. A kit comprising:
(i) a first linker oligonucleotide comprising a 5' phosphate group and having Formula (IA):
5' - [A] - [B]ₓ - [C]_{y} - 3' (IA),
wherein [A] is a "constant region" including between about 5 and about 60 nucleotides; [B] is an "identifier region" including an identifier nucleic acid sequence; [C] is a target specific "unique region" including between about 5 and about 100 nucleotides; x is 0 or 1; and y is 1; and
(ii) a bridge oligonucleotide comprising a 3' overhang and having Formula (II):
3' - [D] - 5' (II),
wherein [D] comprises between 5 and 50 nucleotides, and where at least a portion of the nucleotide sequence of [D] is complementary to the "constant region" [A], and the 3' overhang is a T overhang.

10. The kit of claim 9, further comprising one or more primers which are each at least partially complementary to a portion of the "constant region" [A] of the first linker oligonucleotide having Formula (IA).

11. The kit of claim 9, further comprising a second linker oligonucleotide comprising a 5' phosphate group and having Formula (IC):
5' - [A] - 3' (IC).

12. A master mix comprising:
(a) one or more ligation products, wherein each ligation product of the one or more ligation products includes a probe derived from:
(i) a linker oligonucleotide comprising a 5' phosphate group and having Formula (IA):
5' - [A] - [B]ₓ - [C]_{y} - 3' (IA),
wherein [A] is a "constant region" comprising between about 5 and about 60 nucleotides; [B] is an "identifier region" including an identifier nucleic acid sequence; [C] is a target specific "unique region" including between about 5 and about 100 nucleotides; x is 0 or 1; and y is 1; and
(ii) a bridge oligonucleotide having Formula (II):
3' - [D] - 5' (II),
wherein the bridge oligonucleotide includes a 3' overhang; and wherein [D] comprises between 5 and 50 nucleotides, and wherein at least a portion of the nucleotide sequence of [D] is complementary to the "constant region" [A] of Formula (IA), and the 3' overhang is a T overhang; and
(b) one or more primers each having a nucleotide sequence which is at least partially complementary to a portion of the "constant region" [A] included within the one or more ligation products.

13. A master mix comprising:
(a) one or more ligation products, wherein each ligation product of the one or more ligation products includes a probe derived from:
(i) a linker oligonucleotide comprising a 5' phosphate group and having Formula (IA):
5' - [A] - [B]ₓ - [C] - 3' (IB),
wherein [A] is a "constant region" comprising between 5 and 60 nucleotides; [B] is an "identifier region" including an identifier; [C] is a target specific "unique region" comprising between 5 and 100 nucleotides; and x is 0 or 1;
(ii) a bridge oligonucleotide comprising a 3' overhang and having Formula (II):
3' - [D] - 5' (II),
wherein [D] comprises between 5 and 50 nucleotides, and wherein at least a portion of the nucleotide sequence of [D] is complementary to the "constant region" [A] of Formula (IB), and the 3' overhang is a T overhang;
(b) one or more primers having a nucleotide sequence which is at least partially complementary to a portion of the "constant region" [A] included within the one or more ligation products.

14. A composition comprising: (a) a probe; and (b) an adapter oligonucleotide, where the probe is derived from:
(i) a first linker oligonucleotide comprising a 5' phosphate group and having Formula (IB):
5' - [A] - [B]ₓ - [C] - 3' (IB),
wherein [A] is a "constant region" comprising between about 5 and about 60 nucleotides; [B] is an "identifier region" including an identifier; [C] is a target specific "unique region" comprising between 5 and 100 nucleotides; and x is 0 or 1;
(iii) a first bridge oligonucleotide comprising a 3' overhang and having Formula (II):
3' - [D] - 5' (II),
wherein [D] comprises between about 5 and about 50 nucleotides, and wherein at least a portion of the nucleotide sequence of [D] of the first bridge oligonucleotide is complementary to the "constant region" [A] of the first linker oligonucleotide, and the 3' overhang is a T overhang; and
where the adapter oligonucleotide is derived from:
(i) a second linker oligonucleotide having Formula (IC):
5' - [A] - 3' (IC);
and
(ii) a second bridge oligonucleotide comprising a 3' overhang and having Formula (II):
3' - [D] - 5' (II),
wherein at least a portion of the nucleotide sequence of [D] of the second bridge oligonucleotide is complementary to the "constant region" [A] of the second linker oligonucleotide, and the 3' overhang is a T overhang.

15. Use of the kit of claim 9 in the amplification and/or sequencing of one or more target nucleic acid sequences.

16. A method of capturing or enriching at least one target nucleic acid molecule in a library of nucleic acid molecules, the method comprising:
(a) annealing at least one probe to the at least one target nucleic acid molecule in the library of nucleic acid molecules to form at least one probe-target nucleic acid molecule complex, wherein the at least one probe comprises a constant region and a unique region, wherein the unique region includes a nucleotide sequence which hybridizes to a complementary nucleic acid sequence within the at least one target nucleic acid molecule;
(b) extending the at least one probe-target nucleic acid molecule complex to form at least one extended probe-target nucleic acid molecule complex, and performing A-tailing;
generating one or more ligation products from the at least one extended probe-target nucleic acid molecule complex,
wherein the at least one probe includes a bridge oligonucleotide comprising a 3' overhang and having Formula (II):
3' - [D] - 5' (II),
wherein [D] comprises between 5 and 50 nucleotides, and where at least a portion of the nucleotide sequence of [D] is complementary to the constant region, and the 3' overhang is a T overhang.

## Patentansprüche

1. Verfahren zum Anreichern einer oder mehrerer Zielnukleinsäure(n) in einer Probe, umfassend:
(a) Annealen einer oder mehrerer Sonde(n) an das eine oder die mehreren Zielnukleinsäuremolekül(e) in der Probe zur Bildung eines oder mehrerer Sonde-Zielnukleinsäuremolekül-Komplexe(s), wobei die eine oder mehreren Sonde(n) jeweils eine konstante Region und eine einzigartige Region umfasst/umfassen, wobei die einzigartige Region jeder Sonde eine Nukleotidsequenz einschließt, die an eine komplementäre Nukleinsäuresequenz innerhalb des einen Zielnukleinsäuremoleküls oder der mehreren Zielnukleinsäuremoleküle hybridisiert;
(b) Verlängern des einen oder der mehreren Sonde-Zielnukleinsäuremolekül-Komplexe(s) zur Bildung eines verlängerten oder mehrerer verlängerter Sonde-Zielnukleinsäuremolekül-Komplexe(s) und Durchführen von A-Tailing;
(c) Erzeugen eines oder mehrerer Ligationsprodukte(s) aus dem einen oder den mehreren verlängerten Sonde-Zielnukleinsäuremolekül-Komplex(en);
(d) Einbringen eines oder mehrerer Primer(s) in das/die erzeugte(n) eine oder mehreren Ligationsprodukt(e), wobei der eine oder die mehreren Primer an einen oder mehrere komplementäre(n) Abschnitt(e) der konstanten Region innerhalb jedes von dem einen oder den mehreren Ligationsprodukt(en) hybridisiert/hybridisieren; und
(e) Amplifizieren des einen oder der mehreren Ligationsprodukte(s) in der Gegenwart einer Polymerase, um ein oder mehrere Amplifikationsprodukt(e) bereitzustellen,
wobei die eine oder mehreren Sonde(n) jeweils ein Brückenoligonukleotid einschließt/einschließen, das einen 3'-Überhang umfasst und Formel (II) aufweist:
3' - [D] - 5' (II),
wobei [D] zwischen 5 und 50 Nukleotide umfasst und wobei mindestens ein Abschnitt der Nukleotidsequenz von [D] komplementär zu der konstanten Region ist und der 3'-Überhang ein T-Überhang ist.

2. Verfahren nach Anspruch 1, ferner umfassend das Sequenzieren des einen oder der mehreren Amplifikationsprodukte(s).

3. Verfahren nach Anspruch 1, wobei das eine oder die mehreren Ligationsprodukt(e) intramolekulare Ligationsprodukte umfasst/umfassen, die von einem einzelnen verlängerten Sonde-Zielnukleinsäuremolekül-Komplex abgeleitet sind.

4. Verfahren nach Anspruch 1, wobei das eine oder die mehreren Ligationsprodukt(e) intramolekulare Ligationsprodukte umfasst/umfassen, die aus zwei oder mehr verlängerten Sonde-Zielnukleinsäuremolekül-Komplexen gebildet sind.

5. Verfahren nach Anspruch 1, ferner umfassend das Einbringen eines oder mehrerer Adapter-Oligonukleotide(s) in ein Gemisch, das den einen oder die mehreren verlängerten Sonde-Zielnukleinsäuremolekül-Komplex(e) einschließt.

6. Verfahren nach Anspruch 1, wobei der eine oder die mehreren Primer Vorwärts- und Rückwärts-Primer für inverse PCR ist/sind.

7. Verfahren nach Anspruch 1, wobei die Amplifikation das Durchführen einer inversen Polymerasekettenreaktion umfasst.

8. Verfahren nach Anspruch 1, wobei jede der einen oder mehreren Sonde(n) von Folgendem abgeleitet ist:
(i) einem Linker-Oligonukleotid, das eine 5'-Phosphatgruppe umfasst und Formel (IA) aufweist:
5' - [A] - [B]ₓ - [C]_{y} - 3' (IA),
wobei [A] die "konstante Region" ist und zwischen etwa 5 und etwa 60 Nukleotide einschließt; [B] eine "Identifikationsregion" ist, die eine Identifikationsnukleinsäuresequenz einschließt; [C] die "einzigartige Region" ist und zwischen etwa 5 und etwa 100 Nukleotide einschließt; x 0 oder 1 ist; und y 1 ist; und
(ii) einem Brückenoligonukleotid, das einen 3'-Überhang umfasst und Formel (II) aufweist:
3' - [D] - 5' (II),
wobei [D] zwischen etwa 5 und etwa 50 Nukleotide umfasst und wobei mindestens ein Abschnitt der Nukleotidsequenz von [D] komplementär zu der "konstanten Region" [A] ist und der 3'-Überhang ein T-Überhang ist.

9. Kit, umfassend:
(i) ein erstes Linker-Oligonukleotid, das eine 5'-Phosphatgruppe umfasst und Formel (IA) aufweist:
5' - [A] - [B]ₓ - [C]_{y} - 3' (IA),
wobei [A] eine "konstante Region" ist, die zwischen etwa 5 und etwa 60 Nukleotide einschließt; [B] eine "Identifikationsregion" ist, die eine Identifikationsnukleinsäuresequenz einschließt; [C] eine zielspezifische "einzigartige Region" ist, die zwischen etwa 5 und etwa 100 Nukleotide einschließt; x 0 oder 1 ist; und y 1 ist; und
(ii) ein Brückenoligonukleotid, das einen 3'-Überhang umfasst und Formel (II) aufweist:
3' - [D] - 5' (II),
wobei [D] zwischen 5 und 50 Nukleotide umfasst und wobei mindestens ein Abschnitt der Nukleotidsequenz von [D] komplementär zu der "konstanten Region" [A] ist und der 3'-Überhang ein T-Überhang ist.

10. Kit nach Anspruch 9, ferner umfassend einen oder mehrere Primer, der/die jeweils mindestens teilweise komplementär zu einem Abschnitt der "konstanten Region" [A] des ersten Linker-Oligonukleotids mit Formel (IA) ist/sind.

11. Kit nach Anspruch 9, ferner umfassend ein zweites Linker-Oligonukleotid, das eine 5'-Phosphatgruppe umfasst und Formel (IC) aufweist:
5' - [A] - 3' (IC).

12. Mastermix, umfassend:
(a) ein oder mehrere Ligationsprodukt(e), wobei jedes Ligationsprodukt von dem einen oder den mehreren Ligationsprodukt(en) eine Sonde einschließt, die von Folgendem abgeleitet ist:
(i) einem Linker-Oligonukleotid, das eine 5'-Phosphatgruppe umfasst und Formel (IA) aufweist:
5' - [A] - [B]ₓ - [C]_{y} - 3' (IA),
wobei [A] eine "konstante Region" ist, die zwischen etwa 5 und etwa 60 Nukleotide umfasst; [B] eine "Identifikationsregion" ist, die eine Identifikationsnukleinsäuresequenz einschließt; [C] eine zielspezifische "einzigartige Region" ist, die zwischen etwa 5 und etwa 100 Nukleotide einschließt; x 0 oder 1 ist; und y 1 ist; und
(ii) einem Brückenoligonukleotid, das Formel (II) aufweist:
3' - [D] - 5' (II),
wobei das Brückenoligonukleotid einen 3'-Überhang einschließt und wobei [D] zwischen 5 und 50 Nukleotide umfasst und wobei mindestens ein Abschnitt der Nukleotidsequenz von [D] komplementär zu der "konstanten Region" [A] von Formel (IA) ist und der 3'-Überhang ein T-Überhang ist; und
(b) einen oder mehrere Primer, der/die jeweils eine Nukleotidsequenz aufweist/aufweisen, die mindestens teilweise komplementär zu einem Abschnitt der "konstanten Region" [A] ist, der innerhalb des einen oder der mehreren Ligationsprodukte(s) eingeschlossen ist.

13. Mastermix, umfassend:
(a) ein oder mehrere Ligationsprodukt(e), wobei jedes Ligationsprodukt von dem einen oder den mehreren Ligationsprodukt(en) eine Sonde einschließt, die von Folgendem abgeleitet ist:
(i) einem Linker-Oligonukleotid, das eine 5'-Phosphatgruppe umfasst und Formel (IA) aufweist:
5' - [A] - [B]ₓ - [C] - 3' (IB),
wobei [A] eine "konstante Region" ist, die zwischen 5 und 60 Nukleotide umfasst; [B] eine "Identifikationsregion" ist, die eine Identifikation einschließt; [C] eine zielspezifische "einzigartige Region" ist, die zwischen 5 und 100 Nukleotide umfasst; und x 0 oder 1 ist;
(ii) einem Brückenoligonukleotid, das einen 3'-Überhang umfasst und Formel (II) aufweist:
3' - [D] - 5' (II),
wobei [D] zwischen 5 und 50 Nukleotide umfasst und wobei mindestens ein Abschnitt der Nukleotidsequenz von [D] komplementär zu der "konstanten Region" [A] von Formel (IB) ist und der 3'-Überhang ein T-Überhang ist;
(b) einen oder mehrere Primer, der/die jeweils eine Nukleotidsequenz aufweist/aufweisen, die mindestens teilweise komplementär zu einem Abschnitt der "konstanten Region" [A] ist, der innerhalb des einen oder der mehreren Ligationsprodukte(s) eingeschlossen ist.

14. Zusammensetzung, umfassend: (a) eine Sonde und (b) ein Adapter-Oligonukleotid, wobei die Sonde von Folgendem abgeleitet ist:
(i) einem ersten Linker-Oligonukleotid, das eine 5'-Phosphatgruppe umfasst und Formel (IB) aufweist:
5' - [A] - [B]ₓ - [C] - 3' (IB),
wobei [A] eine "konstante Region" ist, die zwischen etwa 5 und etwa 60 Nukleotide umfasst; [B] eine "Identifikationsregion" ist, die eine Identifikation einschließt; [C] eine zielspezifische "einzigartige Region" ist, die zwischen 5 und 100 Nukleotide umfasst; und x 0 oder 1 ist;
(ii) einem ersten Brückenoligonukleotid, das einen 3'-Überhang umfasst und Formel (II) aufweist:
3' - [D] - 5' (II),
wobei [D] zwischen etwa 5 und etwa 50 Nukleotide umfasst und wobei mindestens ein Abschnitt der Nukleotidsequenz von [D] des ersten Brückenoligonukleotids komplementär zu der "konstanten Region" [A] des ersten Linker-Oligonukleotids ist und der 3'-Überhang ein T-Überhang ist; und
wobei das Adapter-Oligonukleotid von Folgendem abgeleitet ist:
(i) einem zweiten Linker-Oligonukleotid, das Formel (IC) aufweist:
5' - [A] - 3' (IC);
und
(ii) einem zweiten Brückenoligonukleotid, das einen 3'-Überhang umfasst und Formel (II) aufweist:
3' - [D] - 5' (II),
wobei mindestens ein Abschnitt der Nukleotidsequenz von [D] des zweiten Brückenoligonukleotids komplementär zu der "konstanten Region" [A] des zweiten Linker-Oligonukleotids ist und der 3'-Überhang ein T-Überhang ist.

15. Verwendung des Kits nach Anspruch 9 bei der Amplifikation und/oder dem Sequenzieren einer oder mehrerer Zielnukleinsäuresequenz(en).

16. Verfahren zum Fangen oder Anreichern mindestens eines Zielnukleinsäuremoleküls in einer Bibliothek von Nukleinsäuremolekülen, wobei das Verfahren Folgendes umfasst:
(a) Annealen mindestens einer Sonde an mindestens ein Zielnukleinsäuremolekül in der Bibliothek von Nukleinsäuremolekülen zur Bildung mindestens eines Sonde-Zielnukleinsäuremolekül-Komplexes, wobei die mindestens eine Sonde eine konstante Region und eine einzigartige Region umfasst, wobei die einzigartige Region eine Nukleotidsequenz einschließt, die an eine komplementäre Nukleinsäuresequenz innerhalb des mindestens einen Zielnukleinsäuremoleküls hybridisiert;
(b) Verlängern des mindestens einen Sonde-Zielnukleinsäuremolekül-Komplexes zur Bildung mindestens eines verlängerten Sonde-Zielnukleinsäuremolekül-Komplexes und Durchführen von A-Tailing;
Erzeugen eines oder mehrerer Ligationsprodukte(s) aus dem mindestens einen verlängerten Sonde-Zielnukleinsäuremolekül-Komplex,
wobei die mindestens eine Sonde ein Brückenoligonukleotid einschließt, das einen 3'-Überhang umfasst und Formel (II) aufweist:
3' - [D] - 5' (II),
wobei [D] zwischen 5 und 50 Nukleotide umfasst und wobei mindestens ein Abschnitt der Nukleotidsequenz von [D] komplementär zu der konstanten Region ist und der 3'-Überhang ein T-Überhang ist.

## Revendications

1. Procédé d'enrichissement d'un ou de plusieurs acides nucléiques cibles dans un échantillon, comprenant :
(a) la renaturation d'une ou de plusieurs sondes à la ou aux molécules d'acide nucléique cibles dans l'échantillon pour former un ou plusieurs complexes sonde-molécule d'acide nucléique cible, la ou les sondes comprenant chacune une région constante et une région unique, la région unique de chaque sonde comprenant une séquence nucléotidique qui s'hybride à une séquence d'acide nucléique complémentaire au sein de la ou des molécules d'acide nucléique cibles ;
(b) l'extension du ou des complexes sonde-molécule d'acide nucléique cible pour former un ou plusieurs complexes sonde-molécule d'acide nucléique cible étendus, et la réalisation d'un ajout de queue d'adénine ;
(c) la génération d'un ou de plusieurs produits de ligature à partir du ou des complexes sonde-molécule d'acide nucléique cible étendus ;
(d) l'introduction d'une ou de plusieurs amorces dans le ou les produits de ligature générés, la ou les amorces s'hybridant à une ou plusieurs parties complémentaires de la région constante au sein de chacun du ou des produits de ligature ; et
(e) l'amplification du ou des produits de ligature en présence d'une polymérase pour fournir un ou plusieurs produits d'amplification,
dans lequel la ou les sondes comprennent chacune un oligonucléotide pont comprenant une extrémité 3' débordante et ayant la formule (II) :
3' - [D] - 5' (II),
dans lequel [D] comprend entre 5 et 50 nucléotides, et où au moins une partie de la séquence nucléotidique de [D] est complémentaire de la région constante, et l'extrémité 3' débordante est une extrémité T débordante.

2. Procédé selon la revendication 1, comprenant en outre le séquençage du ou des produits d'amplification.

3. Procédé selon la revendication 1, dans lequel le ou les produits de ligature comprennent des produits de ligature intramoléculaire dérivés d'un complexe sonde-molécule d'acide nucléique cible étendu unique.

4. Procédé selon la revendication 1, dans lequel le ou les produits de ligature comprennent des produits de ligature intermoléculaire dérivés de deux complexes sonde-molécule d'acide nucléique cible étendus ou plus.

5. Procédé selon la revendication 1, comprenant en outre l'introduction d'un ou de plusieurs oligonucléotides adaptateurs dans un mélange comprenant le ou les complexes sonde-molécule d'acide nucléique cible étendus.

6. Procédé selon la revendication 1, dans lequel la ou les amorces sont des amorces de PCR inverse sens et antisens.

7. Procédé selon la revendication 1, dans lequel l'amplification comprend la réalisation d'une réaction en chaîne par polymérase inverse.

8. Procédé selon la revendication 1, dans lequel chacune de la ou des sondes est dérivée de :
(i) un oligonucléotide lieur comprenant un groupe phosphate en 5' et ayant la formule (IA) :
5' - [A] - [B]ₓ - [C]_{y} - 3' (IA),
dans lequel [A] représente la « région constante » et comprend entre environ 5 et environ 60 nucléotides ; [B] représente une « région identifiante » comprenant une séquence d'acide nucléique identifiante ; [C] représente la « région unique » et comprend entre environ 5 et environ 100 nucléotides ; x représente 0 ou 1 ; et y représente 1 ; et
(ii) un oligonucléotide pont comprenant une extrémité 3' débordante et ayant la formule (II) :
3' - [D] - 5' (II),
dans lequel [D] comprend entre environ 5 et environ 50 nucléotides, et dans lequel au moins une partie de la séquence nucléotidique de [D] est complémentaire de la « région constante » [A], et l'extrémité 3' débordante est une extrémité T débordante.

9. Kit comprenant :
(i) un premier oligonucléotide lieur comprenant un groupe phosphate en 5' et ayant la formule (IA) :
5' - [A] - [B]ₓ - [C]_{y} - 3' (IA),
dans lequel [A] représente une « région constante » comprenant entre environ 5 et environ 60 nucléotides ; [B] représente une « région identifiante » comprenant une séquence d'acide nucléique identifiante ; [C] représente une « région unique » spécifique de la cible comprenant entre environ 5 et environ 100 nucléotides ; x représente 0 ou 1 ; et y représente 1 ; et
(ii) un oligonucléotide pont comprenant une extrémité 3' débordante et ayant la formule (II) :
3' - [D] - 5' (II),
dans lequel [D] comprend entre 5 et 50 nucléotides, et où au moins une partie de la séquence nucléotidique de [D] est complémentaire de la « région constante » [A], et l'extrémité 3' débordante est une extrémité T débordante.

10. Kit selon la revendication 9, comprenant en outre une ou plusieurs amorces qui sont chacune au moins partiellement complémentaires d'une partie de la « région constante » [A] du premier oligonucléotide lieur ayant la formule (IA).

11. Kit selon la revendication 9, comprenant en outre un second oligonucléotide lieur comprenant un groupe phosphate en 5' et ayant la formule (IC) :
5' - [A] - 3' (IC).

12. Mélange maître comprenant :
(a) un ou plusieurs produits de ligature, dans lequel chaque produit de ligature du ou des produits de ligature comprend une sonde dérivée de :
(i) un oligonucléotide lieur comprenant un groupe phosphate en 5' et ayant la formule (IA) :
5' - [A] - [B]ₓ - [C]_{y} - 3' (IA),
dans lequel [A] représente une « région constante » comprenant entre environ 5 et environ 60 nucléotides ; [B] représente une « région identifiante » comprenant une séquence d'acide nucléique identifiante ; [C] représente une « région unique » spécifique de la cible comprenant entre environ 5 et environ 100 nucléotides ; x représente 0 ou 1 ; et y représente 1 ; et
(ii) un oligonucléotide pont ayant la formule (II) :
3' - [D] - 5' (II),
dans lequel l'oligonucléotide pont comprend une extrémité 3' débordante ; et dans lequel [D] comprend entre 5 et 50 nucléotides, et dans lequel au moins une partie de la séquence nucléotidique de [D] est complémentaire de la « région constante » [A] de formule (IA), et l'extrémité 3' débordante est une extrémité T débordante ; et
(b) une ou plusieurs amorces ayant chacune une séquence nucléotidique qui est au moins partiellement complémentaire d'une partie de la « région constante » [A] comprise au sein du ou des produits de ligature.

13. Mélange maître comprenant :
(a) un ou plusieurs produits de ligature, dans lequel chaque produit de ligature du ou des produits de ligature comprend une sonde dérivée de :
(i) un oligonucléotide lieur comprenant un groupe phosphate en 5' et ayant la formule (IA) :
5' - [A] - [B]ₓ - [C] - 3' (IB),
dans lequel [A] représente une « région constante » comprenant entre 5 et 60 nucléotides ; [B] représente une « région identifiante » comprenant un identifiant ; [C] représente une « région unique » spécifique de la cible comprenant entre 5 et 100 nucléotides ; et x représente 0 ou 1 ;
(ii) un oligonucléotide pont comprenant une extrémité 3' débordante et ayant la formule (II) :
3' - [D] - 5' (II),
dans lequel [D] comprend entre 5 et 50 nucléotides, et dans lequel au moins une partie de la séquence nucléotidique de [D] est complémentaire de la « région constante » [A] de formule (IB), et l'extrémité 3' débordante est une extrémité T débordante ;
(b) une ou de plusieurs amorces ayant une séquence nucléotidique qui est au moins partiellement complémentaire d'une partie de la « région constante » [A] comprise au sein du ou des produits de ligature.

14. Composition comprenant : (a) une sonde ; et (b) un oligonucléotide adaptateur, où la sonde est dérivée de :
(i) un premier oligonucléotide lieur comprenant un groupe phosphate en 5' et ayant la formule (IB) :
5' - [A] - [B]ₓ - [C] - 3' (IB),
dans lequel [A] représente une « région constante » comprenant entre environ 5 et environ 60 nucléotides ; [B] représente une « région identifiante » comprenant un identifiant ; [C] représente une « région unique » spécifique de la cible comprenant entre 5 et 100 nucléotides ; et x représente 0 ou 1 ;
(ii) un premier oligonucléotide pont comprenant une extrémité 3' débordante et ayant la formule (II) :
3' - [D] - 5' (II),
dans lequel [D] comprend entre environ 5 et environ 50 nucléotides, et dans lequel au moins une partie de la séquence nucléotidique de [D] du premier oligonucléotide de pont est complémentaire de la « région constante » [A] du premier oligonucléotide lieur, et l'extrémité 3' débordante est une extrémité T débordante ; et
où l'oligonucléotide adaptateur est dérivé de :
(i) un second oligonucléotide lieur ayant la formule (IC) :
5' - [A] - 3' (IC) ;
et
(ii) un second oligonucléotide pont comprenant une extrémité 3' débordante et ayant la formule (II) :
3' - [D] - 5' (II),
dans lequel au moins une partie de la séquence nucléotidique de [D] du second oligonucléotide pont est complémentaire de la « région constante » [A] du second oligonucléotide lieur, et l'extrémité 3' débordante est une extrémité T débordante.

15. Utilisation du kit selon la revendication 9 dans l'amplification et/ou le séquençage d'une ou de plusieurs séquences d'acide nucléique cibles.

16. Procédé de capture ou d'enrichissement d'au moins une molécule d'acide nucléique cible dans une banque de molécules d'acide nucléique, le procédé comprenant :
(a) la renaturation d'au moins une sonde à l'au moins une molécule d'acide nucléique cible dans la banque de molécules d'acide nucléique pour former au moins un complexe sonde-molécule d'acide nucléique cible, l'au moins une sonde comprenant une région constante et une région unique, la région unique comprenant une séquence nucléotidique qui s'hybride à une séquence d'acide nucléique complémentaire au sein de l'au moins une molécule d'acide nucléique cible ;
(b) l'extension de l'au moins un complexe sonde-molécule d'acide nucléique cible pour former au moins un complexe sonde-molécule d'acide nucléique cible étendu, et la réalisation d'un ajout de queue d'adénine ;
la génération d'un ou de plusieurs produits de ligature à partir de l'au moins un complexe sonde-molécule d'acide nucléique cible étendu,
dans lequel l'au moins une sonde comprend un oligonucléotide pont comprenant un surplomb 3' et ayant la formule (II) :
3' - [D] - 5' (II),
dans lequel [D] comprend entre 5 et 50 nucléotides, et où au moins une partie de la séquence nucléotidique de [D] est complémentaire de la région constante, et l'extrémité 3' débordante est une extrémité T débordante.
